# EUROPEAN PATENT SPECIFICATION

(11) **EP 3 630 212 B1**
(45) Date of publication and mention of the grant of the patent: **11.02.2026**
(21) Application number: 18810828.6
(22) Date of filing: 30.05.2018
(51) Int. Cl.: A61L 27/26, A61F 2/06, A61F 2/07, A61L 27/16, A61L 27/18

(54) **VASCULAR GRAFTS HAVING A MODIFIED SURFACE**
GEFÄSSPROTHESEN MIT EINER MODIFIZIERTEN OBERFLÄCHE
GREFFONS VASCULAIRES AYANT UNE SURFACE MODIFIÉE

(30) Priority: 30.05.2017 US 201762512230 P
(43) Date of publication of application: 08.04.2020
(73) Proprietor: Evonik Canada Inc., L7N3J5 Burlington, ON (CA)
(72) Inventor: HO, Jeannette, Toronto, Ontario M4K 1Z1 (CA); SANTERRE, J. Paul, Toronto, Ontario M4Y 1G3 (CA); STEEDMAN, Mark A., Toronto, Ontario M4N 1P5 (CA); SWENOR, Jamie Robert, Toronto, Ontario M5R 2R3 (CA)
(74) Representative: Evonik Patent Association
(86) International application number: PCT/CA2018/050628
(87) International publication number: WO 2018/218347

(56) References cited:
- EP-A2- 0 692 264
- EP-A2- 1 669 044
- WO-A2-02/098477
- WO-A2-02/15951
- WO-A2-2006/026725
- CA-A1- 2 349 989
- CA-A1- 2 604 696
- CA-A1- 2 701 186
- CA-A1- 2 703 017
- JP-A- 2007 077 359
- US-A- 4 816 339
- US-A1- 2004 122 507
- US-A1- 2011 318 575

## Description

### Background of the Invention

Grafts are tubular constructs used to replace, repair, or bypass occluded or damaged vessels in the cardiovascular system. In addition, vascular grafts are used as access points for medical procedures such as hemodialysis. Grafts can be natural or synthetic. Synthetic grafts are routinely used for large vessel replacement (>7 mm), as they function well in these high-flow, low-resistance circuits. In small diameter vessel replacement, natural grafts, such as autologous veins, are preferred as they have superior biocompatibility and mechanical properties more closely matching those of the native vessel, thus resulting in higher patency rates. However, autologous grafts (from the same human) are not always available (e.g., morbid condition, inappropriate length or diameter) and their harvesting may lead to donor site complications. Allografts (from another human donor) or heterografts (from animal donors) are also used in some cases, but carry the risk of immunogenicity and are prone to degeneration over time. Often, synthetic or biosynthetic grafts remain the only alternative. However, some synthetic grafts perform well in a large, but not small, vessel repair or bypass. The most common causes of graft failure include inappropriate graft diameter. For example, a too large diameter can cause dilation, suture line failure, structural defects, bleeding, and infection. Small or medium diameters can cause thrombosis or intimal hyperplasia.

US 2004/122507 A1 discloses a vascular graft comprising a tubular body having an inner surface and a long axis wherein the inner surface comprises a base polymer comprising polyethylene terephthalate, polytetrafluoroethylene or polyurethane, wherein the tubular body has a first end and a second end adapted for an attachment to an artery or a vein, however this prior art document is silent on the presence of any oligofluorinated additive.

### Summary of the Invention

The invention features a vascular graft including a tubular body having an inner surface and a long axis wherein the inner surface includes an oligofluorinated additive admixed with a base polymer including polyethylene terephthalate, wherein the tubular body has a first end and a second end adapted for an attachment to an artery or a vein, in accordance with claim 1.

In a related aspect, the invention features a vascular graft including a tubular body having an inner surface and a long axis wherein the inner surface includes an oligofluorinated additive admixed with a base polymer including polytetrafluoroethylene, wherein the tubular body has a first end and a second end adapted for an attachment to an artery or a vein, in accordance with claim 2.

The invention further features a vascular graft including a tubular body having an inner surface and a long axis wherein the inner surface includes an oligofluorinated additive admixed with a base polymer including a polyurethane, wherein the tubular body has a first end and a second end adapted for an attachment to an artery or a vein, in accordance with claim 3. In particular embodiments, the polyurethane is selected from, without limitation, polycarbonate urethanes (e.g., BIONATE^{®}), polyurethane with a poly(dimethylsiloxane) soft segment (e.g., Elast-Eon^{™}), a polytetramethylene glycol-based polyurethane elastomer (e.g., Pellethane^{®} 2363-80AE elastomer), segmented polyurethanes (e.g., BIOSPAN^{™}) and polyetherurethanes (e.g., ELASTHANE^{™}).

In particular embodiments of the above aspects, the inner surface can include from 0.1% (w/w) to 15% (w/w), from 0.5% (w/w) to 15% (w/w), from 1% (w/w) to 15% (w/w), from 0.1% (w/w) to 5% (w/w), from 0.5% (w/w) to 5% (w/w), or from 1% (w/w) to 5% (w/w) of the oligofluorinated additive.

The oligofluorinated additives used in the prosthetic valves of the invention is described by the structure of any one of formulae (I), (II), (III), (IV), (V), (VI), (VII), (VIII), (IX), (X), (XI), (XII), (XIII), (XIV), (XV), (XVI), and (XVII) shown below. In certain embodiments, the oligofluorinated additive is selected from any one of compound 1-40. In particular embodiments, the oligofluorinated additive is selected from compound 11, compound 22, and compound 39. In one particular embodiment, the vascular graft of the invention exhibits reduced thrombogenicity in comparison to the vascular graft in the absence of the oligofluorinated material.

In some embodiments, the vascular graft includes a tubular body formed from polytetrafluoroethylene admixed with compound 11. In some embodiments, the vascular graft includes a tubular body formed from polycarbonate urethanes (e.g., BIONATE^{®}) admixed with compound 11. In some embodiments, the vascular graft includes a tubular body formed from polytetrafluoroethylene admixed with compound 11. In some embodiments, the vascular graft includes a tubular body formed from polyurethane with a poly(dimethylsiloxane) soft segment (e.g., Elast-Eon^{™}) admixed with compound 11. In some embodiments, the vascular graft includes a tubular body formed from a polytetramethylene glycol-based polyurethane elastomer (e.g., Pellethane^{®} 2363-80AE elastomer) admixed with compound 11. In some embodiments, the vascular graft includes a tubular body formed from segmented polyurethanes (e.g., BIOSPAN^{™}) admixed with compound 11. In some embodiments, the vascular graft includes a tubular body formed polyetherurethanes (e.g., ELASTHANE^{™}) admixed with compound 11.

In some embodiments, the vascular graft includes a tubular body formed from polytetrafluoroethylene admixed with compound 22. In some embodiments, the vascular graft includes a tubular body formed from polycarbonate urethanes (e.g., BIONATE^{®}) admixed with compound 22. In some embodiments, the vascular graft includes a tubular body formed from polytetrafluoroethylene admixed with compound 22. In some embodiments, the vascular graft includes a tubular body formed from polyurethane with a poly(dimethylsiloxane) soft segment (e.g., Elast-Eon^{™}) admixed with compound 22. In some embodiments, the vascular graft includes a tubular body formed from a polytetramethylene glycol-based polyurethane elastomer (e.g., Pellethane^{®} 2363-80AE elastomer) admixed with compound 22. In some embodiments, the vascular graft includes a tubular body formed from segmented polyurethanes (e.g., BIOSPAN^{™}) admixed with compound 22. In some embodiments, the vascular graft includes a tubular body formed polyetherurethanes (e.g., ELASTHANE^{™}) admixed with compound 22.

In some embodiments, the vascular graft includes a tubular body formed from polytetrafluoroethylene admixed with compound 39. In some embodiments, the vascular graft includes a tubular body formed from polycarbonate urethanes (e.g., BIONATE^{®}) admixed with compound 39. In some embodiments, the vascular graft includes a tubular body formed from polytetrafluoroethylene admixed with compound 39. In some embodiments, the vascular graft includes a tubular body formed from polyurethane with a poly(dimethylsiloxane) soft segment (e.g., Elast-Eon^{™}) admixed with compound 39. In some embodiments, the vascular graft includes a tubular body formed from a polytetramethylene glycol-based polyurethane elastomer (e.g., Pellethane^{®} 2363-80AE elastomer) admixed with compound 39. In some embodiments, the vascular graft includes a tubular body formed from segmented polyurethanes (e.g., BIOSPAN^{™}) admixed with compound 39. In some embodiments, the vascular graft includes a tubular body formed polyetherurethanes (e.g., ELASTHANE^{™}) admixed with compound 39.

In a particular embodiment of any of the above aspects, the first end and the second end adapted for an attachment to an artery or a vein include anchoring barbs or a material suitable for sewing onto a portion of an artery or of a vein.

As used herein, the term "reduced thrombogenicity" refers to the performance of the vascular graft in the assay of Example 4 in comparison to the vascular graft prepared without oligofluorinated additive.

The term "base polymer," as used herein, refers to a polymer having a theoretical molecular weight of greater than or equal to 20 kDa (e.g., greater than or equal to 50 kDa, greater than or equal to 75 kDa, greater than or equal to 100 kDa, greater than or equal to 150 kDa, or greater than 200 kDa). Non-limiting examples of base polymers include: silicone, polyolefin, polyester, polycarbonate, polysulfone, polyamide, polyether, polyurea, polyurethane, polyetherimide, cellulosic polymer, and copolymers thereof, and blends thereof. Further non-limiting examples of the base polymers include a silicone, polycarbonate, polypropylene (PP), polyvinylchloride (PVC), polyvinyl alcohol (PVA), polyvinylpyrrolidone (PVP), polyacrylamide (PAAM), polyethylene oxide, poly(ethylene oxide)-b-poly(propylene oxide)-b-poly(ethylene oxide), poly(hydroxyethylmethacrylate) (polyHEMA), polyethylene terephthalate (PET), polybutylene terephthalate (PBT), polymethylmethacrylate (PMMA), polyether ether ketone (PEEK), polyamide, polyurethane, cellulosic polymer, polysulfone, and copolymers thereof, and blends thereof. Base polymeric copolymers include, e.g., poly(ethylene oxide)-b-poly(propylene oxide)-b-poly(ethylene oxide) and polyether-b-polyamide (e.g., PEBAX).

The term "oligofluorinated additive," as used herein, refers to a segmented compound of any one of formulae (I), (II), (III), (IV), (V), (VI), (VII), (VIII), (IX), (X), (XI), (XII), (XIII), (XIV), (XV), (XVI), and (XVII). Certain oligofluorinated additives can have a theoretical molecular weight of less than or equal to 20 kDa (e.g., less than or equal to 10 kDa). Certain oligofluorinated additives can have a theoretical molecular weight of greater than or equal to 200 Da (e.g., greater than or equal to 300 Da). Non-limiting examples of oligofluorinated additives include those having a theoretical molecular weight of from 500 to 10,000 Da, from 500 to 9,000 Da, from 500 to 5,000 Da, from 1,000 to 10,000 Da, from 1,000 to 6,000 Da, or from 1,500 to 8,000 Da. One of skill in the art will recognize that these structural formulae represent idealized theoretical structures. Specifically, the segments are reacted in specific stoichiometries to furnish an oligofluorinated additive as a distribution of molecules having varying ratios of segments. Accordingly, the variable n in formulae (I)-(XVII) indicates the theoretical stoichiometry of the segments.

As used herein, "C" refers to a chain terminating group. Exemplary chain terminating groups include monofunctional groups containing an amine, alcohol, or carboxylic acid functionality.

The term "LinkB," as used herein, refers to a coupling segment linking two oligomeric segments and a surface-active group. Typically, LinkB has a molecular weight ranging from 40 to 700 Da. Preferably, LinkB can be selected from the group of functionalized diamines, diisocyanates, disulfonic acids, dicarboxylic acids, diacid chlorides, and dialdehydes, where the functionalized component has secondary functional group, through which a surface-active group is attached. Such secondary functional groups can be esters, carboxylic acid salts, sulfonic acid salts, phosphonic acid salts, thiols, vinyls, and primary or secondary amines. Terminal hydroxyls, amines, or carboxylic acids of an oligomeric segment intermediate can react with a diamine to form an oligo-amide; react with a diisocyanate to form an oligo-urethane, an oligo-urea, or an oligo-amide; react with a disulfonic acid to form an oligo-sulfonate or an oligo-sulfonamide; react with a dicarboxylic acid to form an oligo-ester or an oligo-amide; react with a diacyl dichloride to form an oligo-ester or an oligo-amide; or react with a dicarboxaldehyde to form an oligo-acetal or an oligo-imine.

The term "linker with two terminal carbonyls," as used herein, refers to a divalent group having a molecular weight of between 56 Da and 1,000 Da, in which the first valency belongs to a first carbonyl, and a second valency belongs to a second carbonyl. Within this linker, the first carbonyl is bonded to a first carbon atom, and the second carbonyl is bonded to a second carbon atom. The linker with two terminal carbonyls can be a small molecule dicarbonyl (e.g., norbornene-dicarbonyl, benzene-dicarbonyl, biphenyl-dicarbonyl, alkylene-dicarbonyl (e.g., succinoyl, glutaryl, adipoyl, pimeloyl, suberoyl, etc.))

The term "molecular weight," as used herein, refers to a theoretical weight of an Avogadro number of molecules of identical composition. As preparation of an oligofluorinated additive can involve generation of a distribution of compounds, the term "molecular weight" refers to a molar mass of an idealized structure determined by the stoichiometry of the reactive ingredients. Thus, the term "molecular weight," as used herein, refers to a theoretical molecular weight.

The term "oligomeric linker," as used herein, refers to a divalent group containing from two to fifty bonded to each other identical chemical moieties. The chemical moiety can be an alkylene oxide (e.g., ethylene oxide).

The term "oligomeric segment," as used herein, refers to a relatively short length of a repeating unit or units, generally less than about 50 monomeric units and theoretical molecular weights less than 10,000 Da, but preferably <7,000 Da and in some examples, <5,000 Da. In certain embodiments, oligo is selected from the group consisting of polyurethane, polyurea, polyamide, polyalkylene oxide, polycarbonate, polyester, polylactone, polysilicone, polyethersulfone, polyolefin, polyvinyl, polypeptide, polysaccharide, and ether and amine linked segments thereof.

The term "oxycarbonyl bond," as used herein, refers to a bond connecting an oxygen atom to a carbonyl group. Exemplary oxycarbonyl bonds can be found in esters and urethanes. Preferably, the oxycarbonyl bond is a bond in an ester.

The term "polyfluoroorgano group," as used herein, refers to a hydrocarbon group that may be optionally interrupted by one, two, or three non-contiguous oxygen atoms, in which from two to fifty nine hydrogen atoms were replaced with fluorine atoms. The polyfluoroorgano group contains one to thirty carbon atoms. The polyfluoroorgano group can contain linear alkyl, branched alkyl, or aryl groups, or any combination thereof. The polyfluoroorgano group (e.g., polyfluoroalkyl) can be a "polyfluoroacyl," in which the carbon atom, through which the polyfluoroorgano group (e.g., polyfluoroalkyl) is attached to the rest of the molecule, is substituted with oxo. The alkyl chain within polyfluoroorgano group (e.g., polyfluoroalkyl) can be interrupted by up to nine oxygen atoms, provided that two closest oxygen atoms within polyfluoroorgano are separated by at least two carbon atoms. When the polyfluoroorgano consists of a linear or branched alkyl optionally substituted with oxo and/or optionally interrupted with oxygen atoms, as defined herein, such group can be called a polyfluoroalkyl group. Some polyfluoroorgano groups (e.g., polyfluoroalkyl) can have a theoretical molecular weight of from 100 Da to 1,500 Da. A polyfluoroalkyl can be CF₃(CF₂)ᵣ(CH₂CH₂)ₚ-, where p is 0 or 1, r is from 2 to 20, or CF₃(CF₂)ₛ(CH₂CH₂O)_{χ}-, where χ is from 0 to 10, and s is from 1 to 20. Alternatively, polyfluoroalkyl can be CHₘF₍₃₋ₘ₎(CF₂)ᵣCH₂CH₂- or CHₘF₍₃₋ₘ₎(CF₂)ₛ(CH₂CH₂O)_{χ}-, where m is 0, 1, 2, or 3; χ is from 0 to 10; r is an integer from 2 to 20; and s is an integer from 1 to 20. In particular embodiments, χ is 0. In certain embodiments, polyfluoroalkyl is formed from 1H,1H,2H,2H-perfluoro-1-decanol; 1H,1H,2H,2H-perfluoro-1-octanol; 1H,1H,5H-perfluoro-1-pentanol; or 1H,1H, perfluoro-1-butanol, and mixtures thereof. In other embodiments, polyfluoroalkyl is perfluoroheptanoyl. In still other embodiments, polyfluoroalkyl is (CF₃)(CF₂)₅CH₂CH₂O-, (CF₃)(CF₂)₇CH₂CH₂O-, (CF₃)(CF₂)₅CH₂CH₂O-, CHF₂(CF₂)₃CH₂O-, (CF₃)(CF₂)₂CH₂O-, or (CF₃)(CF₂)₅-. In still other embodiments the polyfluoroalkyl group is (CF₃)(CF₂)₅-, e.g., where the polyfluoroalkyl group is bonded to a carbonyl of an ester group. In certain embodiments, polyfluoroorgano is -(O)_{q}-[C(=O)]ᵣ-(CH₂)ₒ(CF₂)ₚCF₃, in which q is 0 and r is 1, or q is 1 and r is 0; o is from 0 to 2; and p is from 0 to 10.

Other features and advantages of the invention will be apparent from the Drawings, Detailed Description, and the Claims.

### Brief Description of the Drawings

Figure 1A shows a structure of compound 1.
Figure 1B shows a structure of compound 2, wherein a = 0.225, b = 0.65, and c = 0.125.
Figure 2A shows a structure of compound 3, wherein a = 0.225, b = 0.65, and c = 0.125.
Figure 2B shows a structure of compound 4, wherein x and y are integers. The poly(ethylene-co-1,2-butylene) soft segment can be formed from poly(ethylene-co-1,2-butylene)diol of a pre-selected average molecular weight (e.g., CAS registry No. 68954-10-9).
Figure 3A shows a structure of compound 5.
Figure 3B shows a structure of compound 6.
Figure 4A shows a structure of compound 7.
Figure 4B shows a structure of compound 8, wherein a, b, and c are integers. The polybutadiene soft segment can be formed from hydroxyl terminated polybutadiene of a pre-selected average molecular weight (e.g., CAS registry No. 69102-90-5).
Figure 5A shows a structure of compound 9.
Figure 5B shows a structure of compound 10.
Figure 6A shows a structure of compound 11.
Figure 6B shows a structure of compound 12.
Figure 7 shows a structure of compound 13.
Figure 8 shows a structure of compound 14, wherein a = 0.225, b = 0.65, and c = 0.125.
Figure 9 shows a structure of compound 15, wherein a = 0.225, b = 0.65, and c = 0.125.
Figure 10 shows a structure of compound 16, wherein a = 0.225, b = 0.65, and c = 0.125.
Figure 11 shows a structure of compound 17.
Figure 12 shows a structure of compound 18.
Figure 13 shows a structure of compound 19.
Figure 14 shows a structure of compound 20, wherein m = 12-16, and n is an integer.
Figure 15 shows a structure of compound 21.
Figure 16 shows a structure of compound 22, wherein x, y, and z are integers. The poly(ethylene glycol)-block-poly(propylene glycol)-block-poly(ethylene glycol) can be, e.g., Pluronic^{®} L-35 (CAS registry No. 9003-11-6).
Figure 17 shows a structure of compound 23.
Figure 18 shows a structure of compound 24.
Figure 19 shows a structure of compound 25, m = 12-16, and n is an integer.
Figure 20 shows a structure of compound 26.
Figure 21A shows a structure of compound 27.
Figure 21B shows a structure of compound 28.
Figure 22 shows a structure of compound 29.
Figure 23A shows a structure of compound 30.
Figure 23B shows a structure of compound 31.
Figure 24A shows a structure of compound 32.
Figure 24B shows a structure of compound 33.
Figure 25 shows a structure of compound 34.
Figure 26 shows a structure of compound 35.
Figure 27 shows a structure of compound 36, wherein each of q, p, n, and m is an integer from 2 to 50.
Figure 28A shows a structure of compound 37.
Figure 28B shows a structure of compound 38.
Figure 29 shows a structure of compound 39, wherein m = 12-16, and n is an integer.
Figure 30 shows a structure of compound 40, wherein x = z = 40, and y = 20.

### Detailed Description

The invention features vascular grafts having an inner surface modified to reduce the risk of forming thrombi post implantation.

### Vascular grafts

Grafts can be classified based on their location of use, material, size, or specialized function. One type of grafts is hemodialysis arteriovenous (AV) access grafts that connect blood from an artery to a vein and are used to provide blood access for hemodialysis. They are often used when AV fistula access is not possible or when more rapid access is required (fistulas can take up to 6 months to mature and many dialysis patients have diabetes or other comorbidities that affect the quality of their veins, making them unsuitable for fistulas). Some of the key requirements for vascular grafts include biostability to resist degradation in vivo, biocompatibility, thromboresistance, and resistance to infection.

One exemplary material used to manufacture grafts includes polyethylene terephthalate (PET/Dacron). PET grafts are made from woven or knitted PET fibers. PET is a highly crystalline polymer with a melt temperature of 265 °C. The fibers are produced by melt-extrusion through a multi-capillary spinnerette die at 290-310 °C, followed by air quenching, and then drawing (stretching)/annealling of the fibers to improve tensile strength. Fiber properties are significantly affected by extrusion temperature and polymer viscosity, spinnerette capillary diameter, spin speed, quench air velocity and temperature, take-up roll speed, draw ratio, drawing temperature, etc. Woven grafts are made from fibers interlaced in over and under pattern to form almost nonporous graft with no stretch. These grafts are very strong (high burst strength and fatigue resistance) but they are also very stiff and tend to have poorer compliance, handling, suturability and tissue integration characteristics than their knitted counterparts. Knitted grafts are formed from fibers interlaced in looped configurations forming a continuous interconnecting chain with variable stretch and porosity. These grafts have better handling characteristics, suturability and tissue integration. They are more compliant then woven grafts, however, they may be more prone to dilation over time. Fabric can be manufactured to be "veloured" or have threads extending outwards from the fabric surface to give a 3-D texture, which can enhance pre-clotting or tissue incorporation. Knitted fabric is usually post-treated through compaction (heating or solvent soaking to shrink fabric and reduce porosity and impart dimensional stability) and cleaning (water or solvent-based). Knitted grafts often need to be made impervious to prevent blood leakage by pre-clotting with patient blood at time of implantation (which is cumbersome and time consuming), coating or "sealing" the graft with natural polymers (e.g., collagen or gelatin from bovine sources). The polymers slowly degrade allowing healing and tissue incorporation of the graft. Potential issues with this approach are coating immunogenicity, thrombogenicity, or presence of residual toxic cross-linking agents. Exemplary application of PET/Dacron is for large diameter, i.e. >7 mm, vessel repair (e.g., aorta, iliac, femoral, popliteal arteries).

A second material used to fabricate grafts is expanded polytetrafluoroethylene (ePTFE). PTFE has a very high melting point of 342 °C, and an extremely high viscosity even at 380 °C, and thus cannot be processed by standard-melt extrusion or injection molding techniques. General procedure for making ePTFE grafts consists of mixing the PTFE powder with a lubricant/solvent, compacting under pressure to form a billet, and then paste-extruding into a tubular shape using cold extrusion. Next, the tube is heated to remove the lubricant/solvent and heated to temperatures approaching the melting point (35-325 °C) while being stretched longitudinally. The raised temperature results in partial coalescence of the PTFE particles and the stretching produces a microporous structure (~30 µm pore size) of solid notes interconnected by fine fibrils oriented in the stretch direction. Then, the tube is "sintered" by heating the polymer to above its melt temperature, usually between 350-375 °C, for a few seconds to up to an h to permanently set the structure. Parameters for this process (heating temperatures, cooling rates, stretching rates, etc.) have a great impact on the formation of the structure and resulting mechanical properties. ePTFE grafts can be reinforced with a thin film of ePTFE with fibril orientation in the axial direction to improve radial tensile strength. ePTFE grafts find applications in medium and small diameter (4-7 mm) vessel repair (e.g., femoropopliteal and lower-extremity).

A third type of graft is based on polyurethanes. Polyurethanes that can be used in the AV grafts of the invention include, without limitation, polycarbonate urethanes (e.g., BIONATE^{®}), polyurethane with a poly(dimethylsiloxane) soft segment (e.g., Elast-Eon^{™}), a polytetramethylene glycol-based polyurethane elastomer (e.g., Pellethane^{®} 2363-80AE elastomer), segmented polyurethanes (e.g., BIOSPAN^{™}) and polyetherurethanes (e.g., ELASTHANE^{™}). There is no standard method for manufacturing polyurethane grafts and a variety of patented processes are used to prepare porous polyurethane grafts. Sample methods include melt spinning in which the fibers are extruded through spinnerette die followed by winding on rotating mandrel to form tubular structure. Alternatively, electrostatic spinning can be used in which fibers are solution spun from a charged nozzle onto an oppositely-charged rotating mandrel to form tubular structure. Another method is spray coating wherein a dilute polymer solution is sprayed onto a rotating mandrel. As the solution droplets land on the mandrel they are pulled into fine microfibers that adhere to previously laid down fibers as the spray nozzle moves back and forth along the length of the mandrel. This gives a nonwoven tubular graft. Coagulation/phase inversion method allows a solution of a polymer to coat a mandrel which is then immersed in a water bath to extract the solvent and induce polymer coagulation/precipitation. Extractable porogens may be used in this process to further control graft porosity. Alternatively, floatation method involves spraying a polymer solution onto the surface of a moving water bath to create a floating membrane or fibers that are then collected on a rotating mandrel. Temperature inversion, on the other hand, works by pouring a polymer dissolved in appropriate solvent/non-solvent mixture into a mold and then flash-frozen and freeze-dried to create a porous structure. Replamineform technique is based on porous choral or sea urchin spines that are shaped into a mold configuration and then a polymer solution or melt is forced in the mold and cooled or dried. A calcium solution is the used to dissolve the mold, leaving a porous graft.

### Oligofluorinated Additives

The oligofluorinated additives used in the vascular grafts of the invention are described by the structure of any one of formulae (I), (II), (III), (IV), (V), (VI), (VII), (VIII), (IX), (X), (XI), (XII), (XIII), (XIV), (XV), (XVI), and (XVII) shown below.
(1) Formula (I):

   F_{T}-[B-A]ₙ-B-F_{T} (I)

   where
   (i) A includes hydrogenated polybutadiene, poly((2,2-dimethyl)-1,3-propylene carbonate), polybutadiene, poly(diethylene glycol)adipate, poly(hexamethylene carbonate), poly(ethylene-co-butylene), (neopentyl glycol-ortho phthalic anhydride) polyester, (diethylene glycol-ortho phthalic anhydride) polyester, (1,6-hexanediol-ortho phthalic anhydride) polyester, or bisphenol A ethoxylate;
   (ii) B is a segment including a urethane; and
   (iii) F_{T} is a polyfluoroorgano group, and
   (iv) n is an integer from 1 to 10.
(2) Formula (II):

   F_{T}-[B-A]ₙ-B-F_{T} (II)

   where
   (i) B includes a urethane;
   (ii) A includes polypropylene oxide, polyethylene oxide, or polytetramethylene oxide;
   (iii) F_{T} is a polyfluoroorgano group; and
   (iv) n is an integer from 1 to 10.
(3) Formula (III) or Formula (IV): 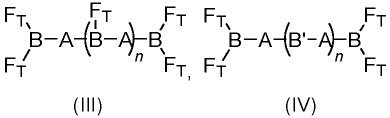 where
   (i) A is an oligomeric segment containing an ether linkage, an ester linkage, a carbonate linkage, or a polyalkylene and having a theoretical molecular weight of from 500 to 3,500 Da (e.g., from 500 to 2,000 Da, from 1,000 to 2,000 Da, or from 1,000 to 3,000 Da);
   (ii) B is a segment including a isocyanurate trimer or biuret trimer; B', when present, is a segment including a urethane;
   (iii) each F_{T} is a polyfluoroorgano group; and
   (iv) n is an integer between 0 to 10.
(4) Formula (V):

   F_{T}-[B-A]ₙ-B-F_{T} (V)

   where
   (i) A is an oligomeric segment including polypropylene oxide, polyethylene oxide, or polytetramethylene oxide and having a theoretical molecular weight of from 500 to 3,000 Da(e.g., from 500 to 2,000 Da, from 1,000 to 2,000 Da, or from 1,000 to 3,000 Da);
   (ii) B is a segment formed from a diisocyanate;
   (iii) F_{T} is a polyfluoroorgano group; and
   (iv) n is an integer from 1 to 10.
(5) 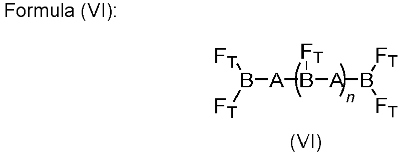 where
   (i) A is an oligomeric segment including polyethylene oxide, polypropylene oxide, polytetramethylene oxide, or a mixture thereof, and having a theoretical molecular weight of from 500 to 3,000 Da (e.g., from 500 to 2,000 Da, from 1,000 to 2,000 Da, or from 1,000 to 3,000 Da);
   (ii) B is a segment including an isocyanurate trimer or biuret trimer;
   (iii) F_{T} is a polyfluoroorgano group; and
   (iv) n is an integer from 0 to 10.
(6) Formula (VII):

   F_{T}-[B-A]ₙ-B-F_{T} (VII)

   where
   (i) A is a polycarbonate polyol having a theoretical molecular weight of from 500 to 3,000 Da(e.g., from 500 to 2,000 Da, from 1,000 to 2,000 Da, or from 1,000 to 3,000 Da);
   (ii) B is a segment formed from a diisocyanate;
   (iii) F_{T} is a polyfluoroorgano group; and
   (iv) n is an integer from 1 to 10.
(7) 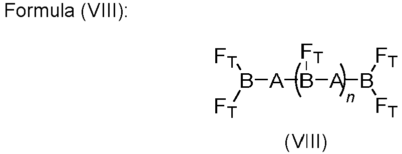 where
   (i) A is an oligomeric segment including a polycarbonate polyol having a theoretical molecular weight of from 500 to 3,000 Da (e.g., from 500 to 2,000 Da, from 1,000 to 2,000 Da, or from 1,000 to 3,000 Da);
   (ii) B is a segment including an isocyanurate trimer or biuret trimer;
   (iii) F_{T} is a polyfluoroorgano group; and
   (iv) n is an integer from 0 to 10.
(8) 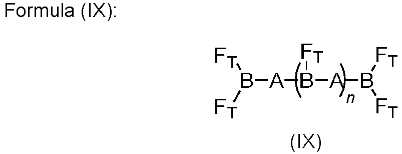 where
   (i) A includes a first block segment selected from polypropylene oxide, polyethylene oxide, polytetramethylene oxide, or a mixture thereof, and a second block segment including a polysiloxane or polydimethylsiloxane, where A has a theoretical molecular weight of from 1,000 to 5,000 Da (e.g., from 1,000 to 3,000 Da, from 2,000 to 5,000 Da, or from 2,500 to 5,000 Da);
   (ii) B is a segment including an isocyanurate trimer or biuret trimer;
   (iii) F_{T} is a polyfluoroorgano group; and
   (iv) n is an integer from 0 to 10.
(9) Formula (X):

   F_{T}-[B-A]ₙ-B-F_{T} (X)

   where
   (i) A is a segment selected from the group consisting of hydrogenated polybutadiene (e.g., HLBH), polybutadiene (e.g., LBHP), hydrogenated polyisoprene (e.g., HHTPI), polysiloxane-polyethylene glycol block copolymer, and polystyrene and has a theoretical molecular weight of from 750 to 3,500 Da(e.g., from 750 to 2,000 Da, from 1,000 to 2,500 Da, or from 1,000 to 3,500 Da);
   (ii) B is a segment formed from a diisocyanate;
   (iii) F_{T} is a polyfluoroorgano group; and
   (iv) n is an integer from 1 to 10.
(10) 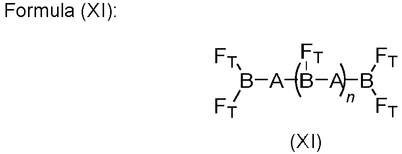 where
   (i) A is hydrogenated polybutadiene (e.g., HLBH), polybutadiene (e.g., LBHP), hydrogenated polyisoprene (e.g., HHTPI), or polystyrene and has a theoretical molecular weight of from 750 to 3,500 Da (e.g., from 750 to 2,000 Da, from 1,000 to 2,500 Da, or from 1,000 to 3,500 Da);
   (ii) B is a segment including an isocyanurate trimer or biuret trimer;
   (iii) F_{T} is a polyfluoroorgano group; and
   (iv) n is an integer from 0 to 10.
(11) 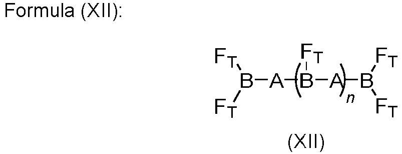 where
   (i) A is a polyester having a theoretical molecular weight of from 500 to 3,500 Da(e.g., from 500 to 2,000 Da, from 1,000 to 2,000 Da, or from 1,000 to 3,000 Da);
   (ii) B is a segment including an isocyanurate trimer or biuret trimer;
   (iii) F_{T} is a polyfluoroorgano group; and
   (iv) n is an integer from 0 to 10.
(12) Formula (XIII):

   F_{T}-A-F_{T} (XIII)

   where F_{T} is a polyfluoroorgano group and A is an oligomeric segment.
(13) 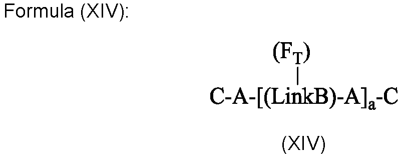 where
   (i) F_{T} is a polyfluoroorgano group covalently attached to LinkB;
   (ii) C is a chain terminating group;
   (iii) A is an oligomeric segment;
   (iv) LinkB is a coupling segment; and
   (v) a is an integer greater than 0.
(14) 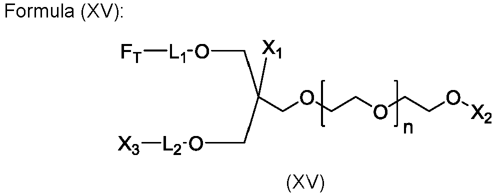 where
   (i) each F_{T} is a polyfluoroorgano group;
   (ii) X₁ is H, CH₃, or CH₂CH₃;
   (iii) each of X₂ and X₃ is independently H, CH₃, CH₂CH₃, or F_{T};
   (iv) each of L₁ and L₂ is independently a bond, an oligomeric linker, or a linker with two terminal carbonyls; and
   (v) n is an integer from 5 to 50.
(15) 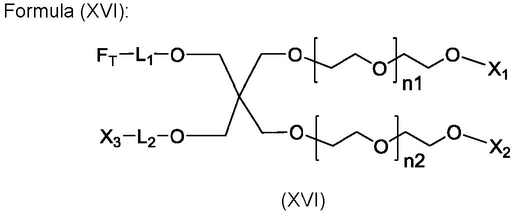 where
   (i) each F_{T} is a polyfluoroorgano;
   (ii) each of X₁, X₂, and X₃ is independently H, CH₃, CH₂CH₃, or F_{T};
   (iii) each of L₁ and L₂ is independently a bond, an oligomeric linker, a linker with two terminal carbonyls, or is formed from a diisocyanate; and
   (iv) each of n1 and n2 is independently an integer from 5 to 50.
(16) Formula (XVII):

   G-Aₘ-[B-A]ₙ-B-G (XVII)

   where
   (i) each A includes hydrogenated polybutadiene, poly ((2,2-dimethyl)-1,3-propylene carbonate), polybutadiene, poly (diethylene glycol)adipate, poly (hexamethylene carbonate), poly (ethylene-co-butylene), (diethylene glycol-ortho phthalic anhydride) polyester, (1,6-hexanediol-ortho phthalic anhydride) polyester, (neopentyl glycol-ortho phthalic anhydride) polyester, a polysiloxane, or bisphenol A ethoxylate;
   (ii) each B is independently a bond, an oligomeric linker, or a linker with two terminal carbonyls;
   (iii) each G is H or a polyfluoroograno, provided that at least one G is a polyfluoroorgano;
   (iv) n is an integer from 1 to 10; and
   (v) m is 0 or 1.

The oligofluorinated oligofluorinated additive of formula (I) can include B formed from a diisocyanate (e.g., 3-isocyanatomethyl-3,5,5-trimethyl-cyclohexylisocyanate; 4,4'-methylene bis(cyclohexyl isocyanate); 4,4'-methylene bis(phenyl isocyanate); toluene-2,4-diisocyanate; m-tetramethylxylene diisocyanate; or hexamethylene diisocyanate). The variable n may be 1 or 2. The vascular grafts of the invention may include a surface containing a base polymer and the oligofluorinated additive of formula (I).

The oligofluorinated additive of formulae (III) and (IV) can include A that is an oligomeric segment containing hydrogenated polybutadiene (HLBH), poly((2,2-dimethyl)-1,3-propylene carbonate) (PCN), polybutadiene (LBHP), polytetramethylene oxide (PTMO), polypropylene oxide (PPO), (diethyleneglycol-orthophthalic anhydride) polyester (PDP), hydrogenated polyisoprene (HHTPI), poly(hexamethylene carbonate), poly((2-butyl-2-ethyl)-1,3-propylene carbonate), or hydroxylterminated polydimethylsiloxane (C22). In the oligofluorinated additive of formulae (III) and (IV), B is formed by reacting a triisocyanate (e.g., hexamethylene diisocyanate (HDI) biuret trimer, isophorone diisocyanate (IPDI) trimer, or hexamethylene diisocyanate (HDI) trimer) with a diol including the oligomeric segment A. The vascular grafts of the invention may include a surface containing a base polymer and the oligofluorinated additive of formula (III). The vascular grafts of the invention may include a surface containing a base polymer and the oligofluorinated additive of formula (IV).

In the oligofluorinated additive of formula (V), B may be a segment formed from 3-isocyanatomethyl-3,5,5-trimethyl-cyclohexylisocyanate; 4,4'-methylene bis(cyclohexyl isocyanate); 4,4'-methylene bis(phenyl isocyanate); toluene-2,4-diisocyanate; m-tetramethylxylene diisocyanate; and hexamethylene diisocyanate. In the oligofluorinated additive of formula (V), segment A can be poly(ethylene oxide)-b-poly(propylene oxide)-b-poly(ethylene oxide). The variable n may be an integer from 1 to 3. The vascular grafts of the invention may include a surface containing a base polymer and the oligofluorinated additive of formula (V).

In the oligofluorinated additive of formula (VI), B is a segment formed by reacting a triisocyanate with a diol of A. The triisocyanate may be hexamethylene diisocyanate (HDI) biuret trimer, isophorone diisocyanate (IPDI) trimer, or hexamethylene diisocyanate (HDI) trimer. In the oligofluorinated additive of formula (VI), segment A can be poly(ethylene oxide)-b-poly(propylene oxide)-b-poly(ethylene oxide). The variable n may be 0, 1, 2, or 3. The vascular grafts of the invention may include a surface containing a base polymer and the oligofluorinated additive of formula (VI).

In the oligofluorinated additive of formula (VII), oligo can include poly((2,2-dimethyl)-1,3-propylene carbonate) (PCN). B may be a segment formed from 3-isocyanatomethyl-3,5,5-trimethyl-cyclohexylisocyanate; 4,4'-methylene bis(cyclohexyl isocyanate); 4,4'-methylene bis(phenyl isocyanate); toluene-2,4-diisocyanate; m-tetramethylxylene diisocyanate; and hexamethylene diisocyanate. The variable n may be 1, 2, or 3. The vascular grafts of the invention may include a surface containing a base polymer and the oligofluorinated additive of formula (VII).

In the oligofluorinated additive of formula (VIII), B is a segment formed by reacting a triisocyanate with a diol of A (e.g., the oligomeric segment). The triisocyanate may be hexamethylene diisocyanate (HDI) biuret trimer, isophorone diisocyanate (IPDI) trimer, or hexamethylene diisocyanate (HDI) trimer. The segment A can include poly((2,2-dimethyl)-1,3-propylene carbonate) (PCN) or poly(hexamethylene carbonate) (PHCN). The variable n may be 0, 1, 2, or 3. The vascular grafts of the invention may include a surface containing a base polymer and the oligofluorinated additive of formula (VIII).

In the oligofluorinated additive of formula (IX), B is a segment formed by reacting a triisocyanate with a diol of A. In segment A, the number of first block segments and second block segments can be any integer or non-integer to provide the approximate theoretical molecule weight of the segment. The segment A can include polypropylene oxide and polydimethylsiloxane. The triisocyanate may be hexamethylene diisocyanate (HDI) biuret trimer, isophorone diisocyanate (IPDI) trimer, or hexamethylene diisocyanate (HDI) trimer. The variable n may be 0, 1, 2, or 3. The vascular grafts of the invention may include a surface containing a base polymer and the oligofluorinated additive of formula (IX).

In oligofluorinated additive of formula (X), B is a segment formed from a diisocyanate. The segment A can include hydrogenated polybutadiene. Alternatively, the segment A can include polysiloxane-polyethylene glycol block copolymer (e.g., PEG-PDMS-PEG). The segment B may be formed from 3-isocyanatomethyl-3,5,5-trimethy-cyclohexylisocyanate; 4,4'-methylene bis(cyclohexyl isocyanate); 4,4'-methylene bis(phenyl isocyanate); toluene-2,4-diisocyanate; m-tetramethylxylene diisocyanate; and hexamethylene diisocyanate. The variable n may be 1, 2, or 3. The vascular grafts of the invention may include a surface containing a base polymer and the oligofluorinated additive of formula (X).

In the oligofluorinated additive of formula (XI), B is a segment formed by reacting a triisocyanate with a diol of A. The segment A may be hydrogenated polybutadiene (HLBH) or hydrogenated polyisoprene (HHTPI). The triisocyanate may be hexamethylene diisocyanate (HDI) biuret trimer, isophorone diisocyanate (IPDI) trimer, or hexamethylene diisocyanate (HDI) trimer. The variable n may be 0, 1, 2, or 3. The vascular grafts of the invention may include a surface containing a base polymer and the oligofluorinated additive of formula (XI).

In the oligofluorinated additive of formula (XII), B is a segment formed by reacting a triisocyanate with a diol of A (e.g., polyester). The segment A may be poly(diethylene glycol)adipate, (neopentyl glycol-ortho phthalic anhydride) polyester, (diethylene glycol-ortho phthalic) anhydride polyester, or (1,6-hexanediol-ortho phthalic anhydride) polyester. The triisocyanate may be hexamethylene diisocyanate (HDI) biuret trimer, isophorone diisocyanate (IPDI) trimer, and hexamethylene diisocyanate (HDI) trimer. The variable n may be 0, 1, 2, or 3. The vascular grafts of the invention may include a surface containing a base polymer and the oligofluorinated additive of formula (XII).

The oligofluorinated additive of formula (XIII) can include a segment A that is a branched or non-branched oligomeric segment of fewer than 20 repeating units (e.g., from 2 to 15 units, from 2 to 10 units, from 3 to 15 units, and from 3 to 10 units). In certain embodiments, the oligofluorinated additive of formula (XIII) includes an oligomeric segment selected from polyurethane, polyurea, polyamide, polyalkylene oxide, polycarbonate, polyester, polylactone, polysilicone, polyethersulfone, polyolefin, polyvinyl derivative, polypeptide, polysaccharide, polysiloxane, polydimethylsiloxane, polyethylene-butylene, polyisobutylene, polybutadiene, polypropylene oxide, polyethylene oxide, polytetramethylene oxide, or polyethylenebutylene segments. The vascular grafts of the invention may include a surface containing a base polymer and the oligofluorinated additive of formula (XIII).

The oligofluorinated additive of formula (XIV) can include a segment A that is a branched or non-branched oligomeric segment of fewer than 20 repeating units (e.g., from 2 to 15 units, from 2 to 10 units, from 3 to 15 units, and from 3 to 10 units). In certain embodiments, the oligofluorinated additive of formula (XIV) includes an oligomeric segment selected from polyurethane, polyurea, polyamide, polyalkylene oxide, polycarbonate, polyester, polylactone, polysilicone, polyethersulfone, polyolefin, polyvinyl derivative, polypeptide, polysaccharide, polysiloxane, polydimethylsiloxane, polyethylene-butylene, polyisobutylene, polybutadiene, polypropylene oxide, polyethylene oxide, or polytetramethylene oxide. The vascular grafts of the invention may include a surface containing a base polymer and the oligofluorinated additive of formula (XIV).

The oligofluorinated additive of formula (XV) can include a segment L₁ that is an oligomeric linker (e.g., of fewer than 50 repeating units (e.g., from 2 to 40 units, from 2 to 30 units, from 3 to 20 units, or from 3 to 10 units)). In some embodiments of formula (XV), L₂ is an oligomeric linker (e.g., of fewer than 50 repeating units (e.g., from 2 to 40 units, from 2 to 30 units, from 3 to 20 units, or from 3 to 10 units)). In particular embodiments of formula (XV), each of L₁ and L₂ is a bond. In certain embodiments of formula (XV), the oligofluorinated additive includes an oligomeric segment (e.g., in any one of L₁ and L₂) selected from the group consisting of polyurethane, polyurea, polyamide, polyalkylene oxide (e.g., polypropylene oxide, polyethylene oxide, or polytetramethylene oxide), polyester, polylactone, polysilicone, polyethersulfone, polyolefin, polyvinyl derivative, polypeptide, polysaccharide, polysiloxane, polydimethylsiloxane, poly(ethylene-co-butylene), polyisobutylene, and polybutadiene. In some embodiments of formula (XV), the oligofluorinated additive is a compound of formula (XV-A): 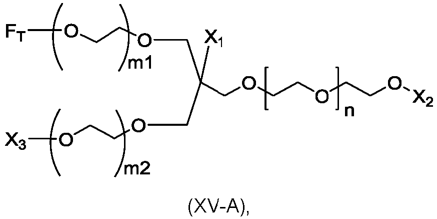 where each of m1 and m2 is independently an integer from 0 to 50. In particular embodiments of formula (XV-A), m1 is 5, 6, 7, 8, 9, or 10 (e.g., m1 is 6). In some embodiments of formula (XV-A), m2 is 5, 6, 7, 8, 9, or 10 (e.g., m2 is 6).

In certain embodiments of formula (XV) or (XV-A), X₂ is F_{T}. In other embodiments, X₂ is CH₃ or CH₂CH₃. In particular embodiments of formula (XV) or (XV-A), X₃ is F_{T}. In other embodiments, each F_{T} is independently a polyfluoroorgano (e.g., a polyfluoroacyl, such as -(O)_{q}-[C(=O)]ᵣ-(CH₂)ₒ(CF₂)ₚCF₃, in which q is 0, r is 1; o is from 0 to 2; and p is from 0 to 10). In certain embodiments of formula (XV) or (XV-A), n is an integer from 5 to 40 (e.g., from 5 to 20, such as from 5, 6, 7, 8, 9, or 10). In some embodiments of formula (XV) or (XV-A), each F_{T} includes (CF₂)₅CF₃. The vascular grafts of the invention may include a surface containing a base polymer and the oligofluorinated additive of formula (XV). The vascular grafts of the invention may include a surface containing a base polymer and the oligofluorinated additive of formula (XV-A).

The oligofluorinated additive of formula (XVI) can include a segment L₁ that is an oligomeric linker (e.g., of fewer than 50 repeating units (e.g., from 2 to 40 units, from 2 to 30 units, from 3 to 20 units, or from 3 to 10 units)). In some embodiments of formula (XVI), L₂ is an oligomeric linker (e.g., of fewer than 50 repeating units (e.g., from 2 to 40 units, from 2 to 30 units, from 3 to 20 units, or from 3 to 10 units)). In particular embodiments of formula (XVI), each of L₁ and L₂ is a bond. In certain embodiments of formula (XVI), the oligofluorinated additive includes an oligomeric segment (e.g., in any one of L₁ and L₂) selected from polyurethane, polyurea, polyamide, polyalkylene oxide (e.g., polypropylene oxide, polyethylene oxide, or polytetramethylene oxide), polyester, polylactone, polysilicone, polyethersulfone, polyolefin, polyvinyl derivative, polypeptide, polysaccharide, polysiloxane, polydimethylsiloxane, poly(ethylene-co-butylene), polyisobutylene, or polybutadiene. In some embodiments of formula (XVI), the oligofluorinated additive is a compound of formula (XVI-A): 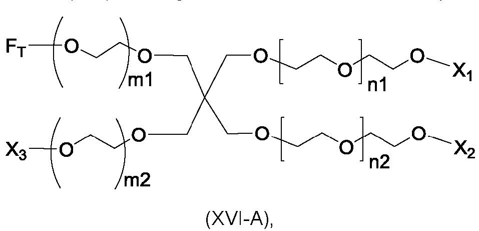 where each of m1 and m2 is independently an integer from 0 to 50. In particular embodiments of formula (XV-A), m1 is 5, 6, 7, 8, 9, or 10 (e.g., m1 is 6). In some embodiments of formula (XV-A), m2 is 5, 6, 7, 8, 9, or 10 (e.g., m2 is 6).

In certain embodiments of formula (XVI) or (XVI-A), X₂ is F_{T}. In other embodiments of formula (XVI) or (XVI-A), X₂ is CH₃ or CH₂CH₃. In particular embodiments of formula (XVI) or (XVI-A), X₃ is F_{T}. In other embodiments of formula (XVI) or (XVI-A), each F_{T} is independently a polyfluoroorgano (e.g., a polyfluoroacyl, such as -(O)_{q}-[C(=O)]ᵣ-(CH₂)ₒ(CF₂)ₚCF₃, in which q is 0, r is 1; o is from 0 to 2; and p is from 0 to 10). In some embodiments of formula (XVI) or (XVI-A), each F_{T} includes (CF₂)₅CF₃. The vascular grafts of the invention may include a surface containing a base polymer and the oligofluorinated additive of formula (XVI). The vascular grafts of the invention may include a surface containing a base polymer and the oligofluorinated additive of formula (XVI-A).

In some embodiments of formula (XVII), m is 1. The oligofluorinated additive of formula (XVII) can be a compound of formula (XVII-A):

G-A-[B-A]ₙ-G (XVII-A).

In other embodiments of formula (XVII), m is 0. The oligofluorinated additive of formula (XVII) can be a compound of formula (XVII-B):

G-[B-A]ₙ-B-G (XVII-B).

In particular embodiments of formula (XVII), (XVII-A), or (XVII-B), each B is a linker with two terminal carbonyls. In certain embodiments of formula (XVII), (XVII-A), or (XVII-B), each B is a bond. In some embodiments of Formula (XVII), (XVII-A), or (XVII-B), the bond connecting G and B is an oxycarbonyl bond (e.g., an oxycarbonyl bond in an ester). In other embodiments of formula (XVII), (XVII-A), or (XVII-B), n is 1 or 2.

The oligofluorinated additive of formula (XVII) can be a compound of formula (XVII-C):

G-A-G (XVII-C).

In formula (XVII), (XVII-A), (XVII-B), or (XVII-C), G can be a polyfluoroorgano group (e.g., a polyfluoroalkyl). In some embodiments of formula (XVII), (XVII-A), (XVII-B), or (XVII-C), G is F_{T} (e.g., each F_{T} is independently a polyfluoroorgano (e.g., a polyfluoroacyl, such as -(O)_{q}-[C(=O)]ᵣ-(CH₂)ₒ(CF₂)ₚCF₃, in which q is 0, r is 1; o is from 0 to 2; and p is from 0 to 10). In some embodiments of formula (XVII), (XVII-A), (XVII-B), or (XVII-C), each F_{T} includes (CF₂)₅CF₃. The vascular grafts of the invention may include a surface containing a base polymer and the oligofluorinated additive of formula (XVII). The vascular grafts of the invention may include a surface containing a base polymer and the oligofluorinated additive of formula (XVII-A). The vascular grafts of the invention may include a surface containing a base polymer and the oligofluorinated additive of formula (XVII-B). The vascular grafts of the invention may include a surface containing a base polymer and the oligofluorinated additive of formula (XVII-C).

For any of the oligofluorinated additives of the invention formed from a diisocyanate, the diisocyanate may be 3-isocyanatomethyl-3,5,5-trimethyl-cyclohexylisocyanate; 4,4'-methylene bis(cyclohexyl isocyanate) (HMDI); 2,2'-, 2,4'-, and 4,4'-methylene bis(phenyl isocyanate) (MDI); toluene-2,4-diisocyanate; aromatic aliphatic isocyanate, such 1,2-, 1,3-, and 1,4-xylene diisocyanate; meta-tetramethylxylene diisocyanate (m-TMXDI); para-tetramethylxylene diisocyanate (p-TMXDI); hexamethylene diisocyanate (HDI); ethylene diisocyanate; propylene-1,2-diisocyanate; tetramethylene diisocyanate; tetramethylene-1,4-diisocyanate; octamethylene diisocyanate; decamethylene diisocyanate; 2,2,4-trimethylhexamethylene diisocyanate; 2,4,4-trimethylhexamethylene diisocyanate; dodecane-1,12-diisocyanate; dicyclohexylmethane diisocyanate; cyclobutane-1,3-diisocyanate; cyclohexane-1,2-diisocyanate; cyclohexane-1,3-diisocyanate; cyclohexane-1,4-diisocyanate; methyl-cyclohexylene diisocyanate (HTDI); 2,4-dimethylcyclohexane diisocyanate; 2,6-dimethylcyclohexane diisocyanate; 4,4'-dicyclohexyl diisocyanate; 2,4'-dicyclohexyl diisocyanate; 1,3,5-cyclohexane triisocyanate; isocyanatomethylcyclohexane isocyanate; 1-isocyanato-3,3,5-trimethyl-5-isocyanatomethylcyclohexane; isocyanatoethylcyclohexane isocyanate; bis(isocyanatomethyl)-cyclohexane; 4,4'-bis(isocyanatomethyl) dicyclohexane; 2,4'-bis(isocyanatomethyl) dicyclohexane; isophoronediisocyanate (IPDI); 2,4-hexahydrotoluene diisocyanate; 2,6-hexahydrotoluene diisocyanate; 3,3'-dimethyl-4,4'-biphenylene diisocyanate (TODI); polymeric MDI; carbodiimide-modified liquid 4,4'-diphenylmethane diisocyanate; para-phenylene diisocyanate (PPDI); meta-phenylene diisocyanate (MPDI); naphthylene-1,5-diisocyanate; 2,4'-, 4,4'-, or 2,2'-biphenyl diisocyanate; polyphenyl polymethylene polyisocyanate (PMDI); mixtures of MDI and PMDI; mixtures of PMDI and TDI; dimerized uretdione of any isocyanate described herein, such as uretdione of toluene diisocyanate, uretdione of hexamethylene diisocyanate, or a mixture thereof; or a substituted or isomeric mixture thereof.

For any of the oligofluorinated additives of the invention formed from an isocyanate trimer, the isocyanate trimer can be hexamethylene diisocyanate (HDI) biuret or trimer, isophorone diisocyanate (IPDI) trimer, hexamethylene diisocyanate (HDI) trimer; 2,2,4-trimethyl-1,6-hexane diisocyanate (TMDI) trimer; a trimerized isocyanurate of any isocyanates described herein, such as isocyanurate of toluene diisocyanate, trimer of diphenylmethane diisocyanate, trimer of tetramethylxylene diisocyanate, or a mixture thereof; a trimerized biuret of any isocyanates described herein; modified isocyanates derived from the above diisocyanates; or a substituted or isomeric mixture thereof.

The oligofluorinated additive can include the group F_{T} that is a polyfluoroorgano group having a theoretical molecular weight of from 100 Da to 1,500 Da. For example, F_{T} may be CF₃(CF₂)ᵣ(CH₂CH₂)ₚ- wherein p is 0 or 1, r is 2-20, and CF₃(CF₂)ₛ(CH₂CH₂O)_{χ}, where χ is from 0 to 10 and s is from 1 to 20. Alternatively, F_{T} may be CHₘF₍₃₋ₘ₎(CF₂)ᵣCH₂CH₂- or CHₘF₍₃₋ₘ₎(CF₂)ₛ(CH₂CH₂O)_{χ}-, where m is 0, 1, 2, or 3; χ is an integer from 0 to 10; r is an integer from 2 to 20; and s is an integer from 1 to 20. In certain embodiments, F_{T} is 1H,1H,2H,2H-perfluoro-1-decanol; 1H,1H,2H,2H-perfluoro-1-octanol; 1H,1H,5H-perfluoro-1-pentanol; or 1H,1H-perfluoro-1-butanol, or a mixture thereof. In particular embodiments, F_{T} is (CF₃)(CF₂)₅CH₂CH₂O-, (CF₃)(CF₂)₇CH₂CH₂O-, (CF₃)(CF₂)₅CH₂CH₂O-, CHF₂(CF₂)₃CH₂O-, (CF₃)(CF₂)₂CH₂O-, or (CF₃)(CF₂)₅-. In still other embodiments the polyfluoroalkyl group is (CF₃)(CF₂)₅-, e.g., where the polyfluoroalkyl group is bonded to a carbonyl of an ester group. In certain embodiments, polyfluoroorgano is -(O)_{q}-[C(=O)]ᵣ-(CH₂)ₒ(CF₂)ₚCF₃, in which q is 0 and r is 1, or q is 1 and r is 0; o is from 0 to 2; and p is from 0 to 10.

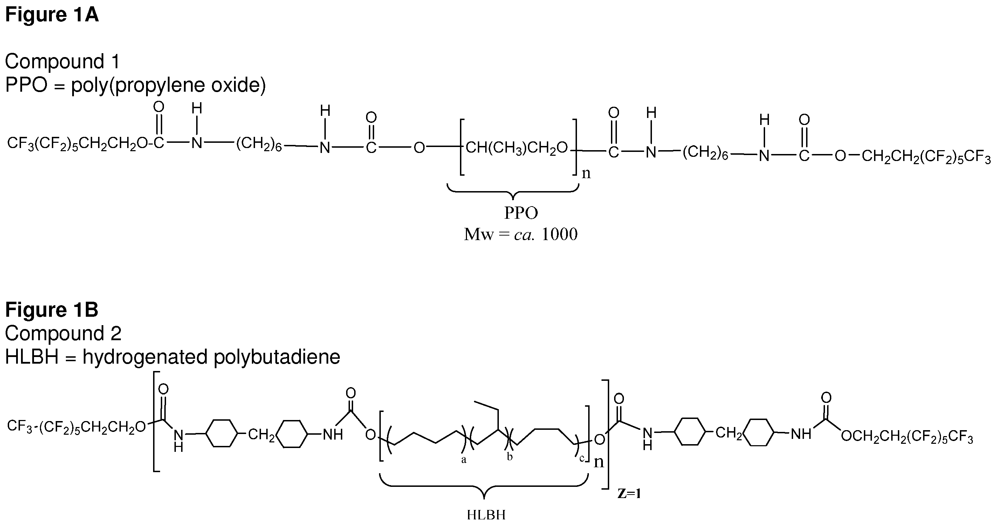

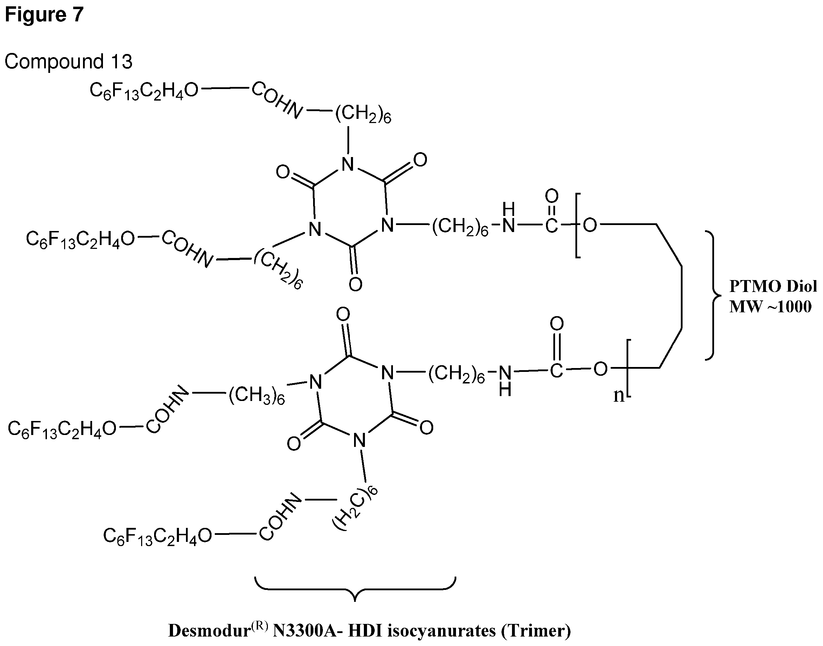

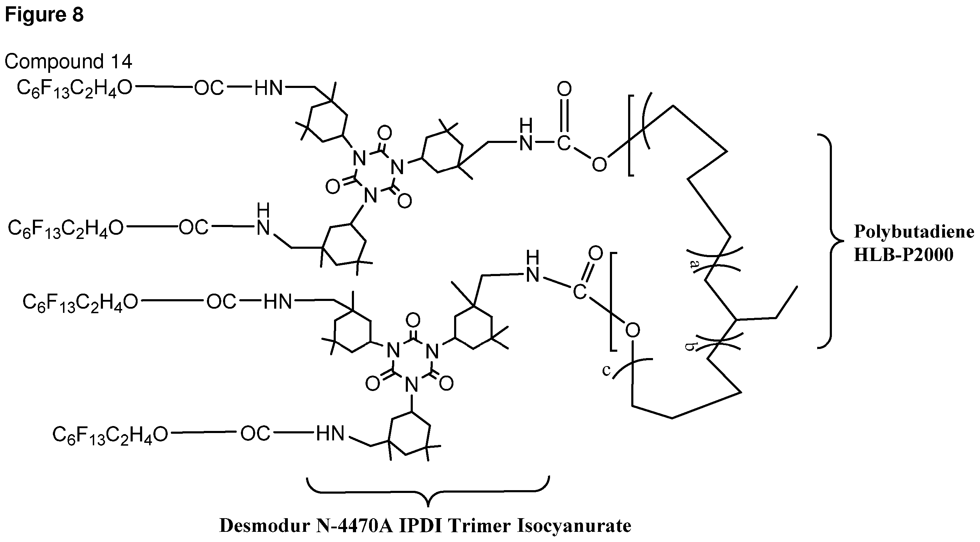

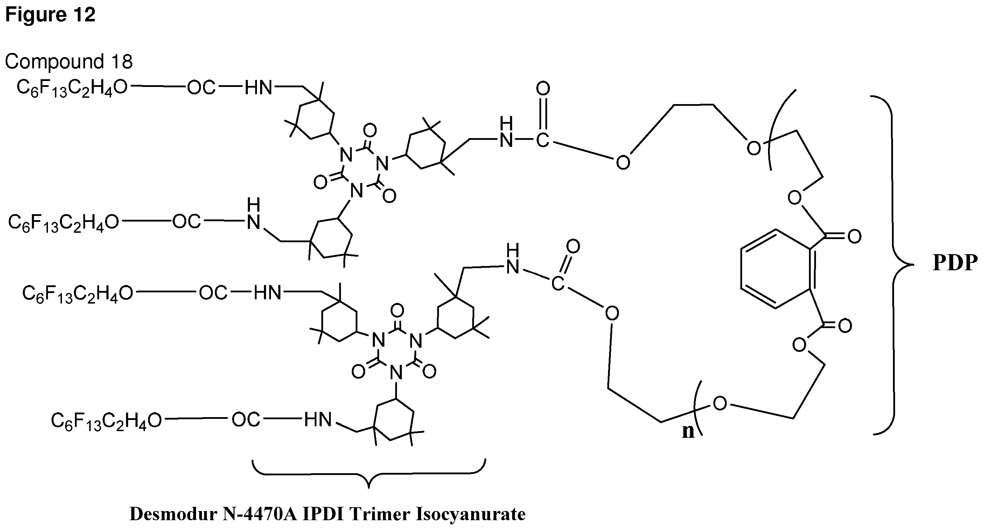

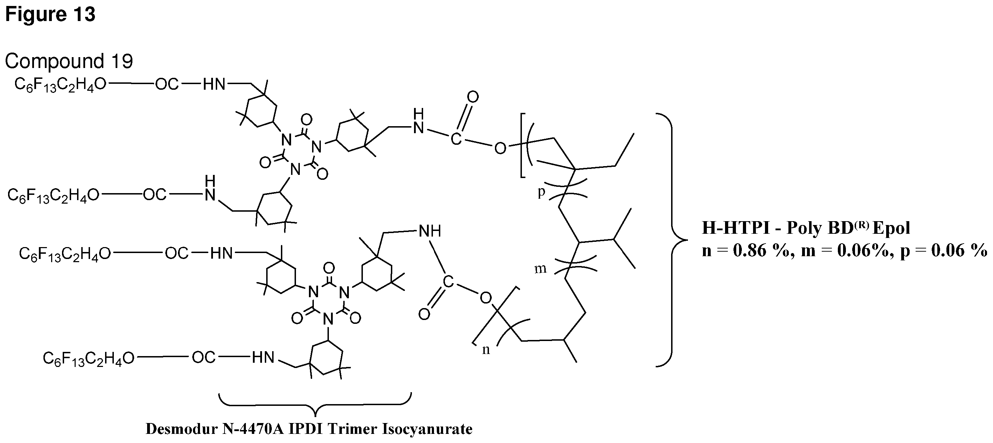

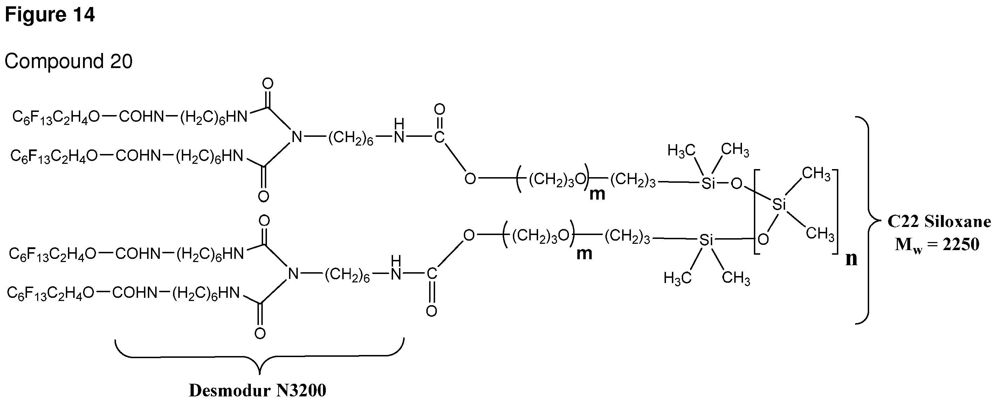

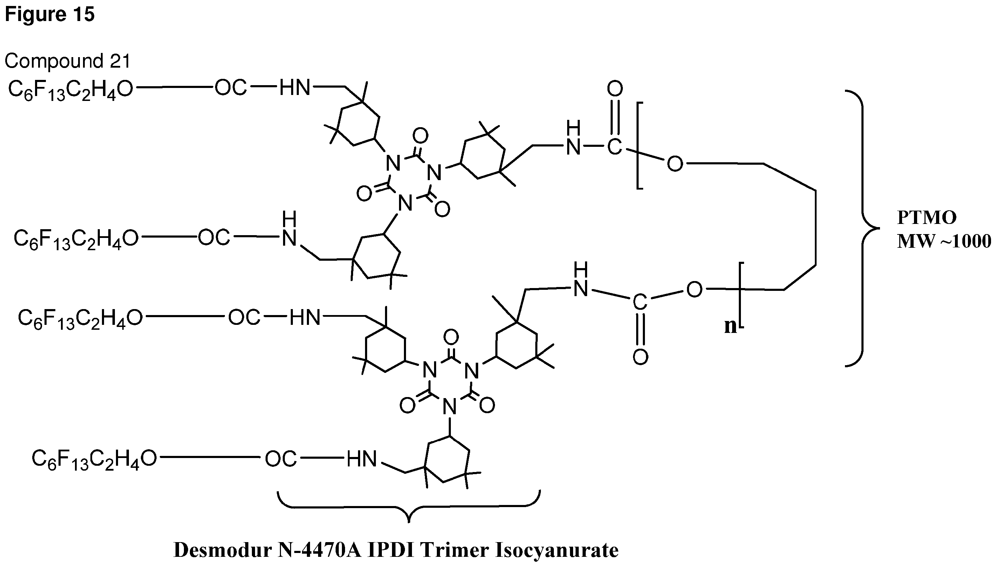

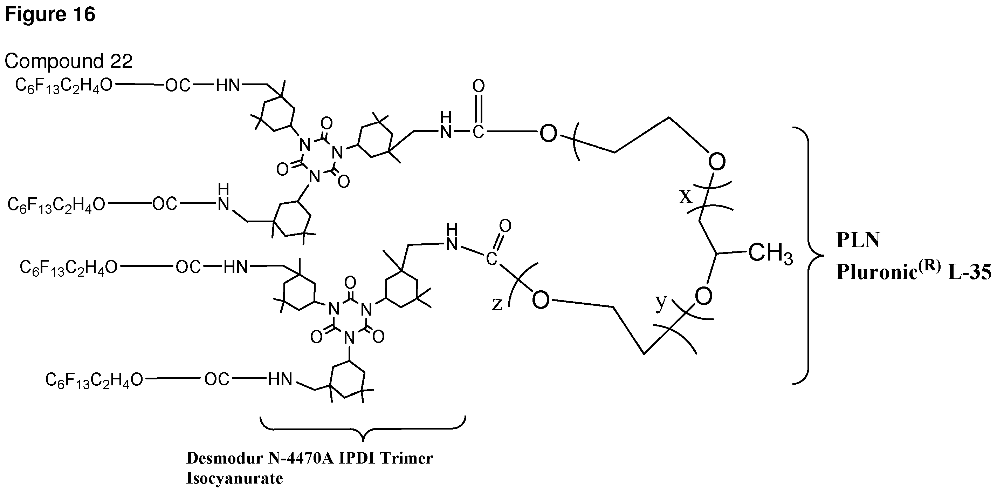

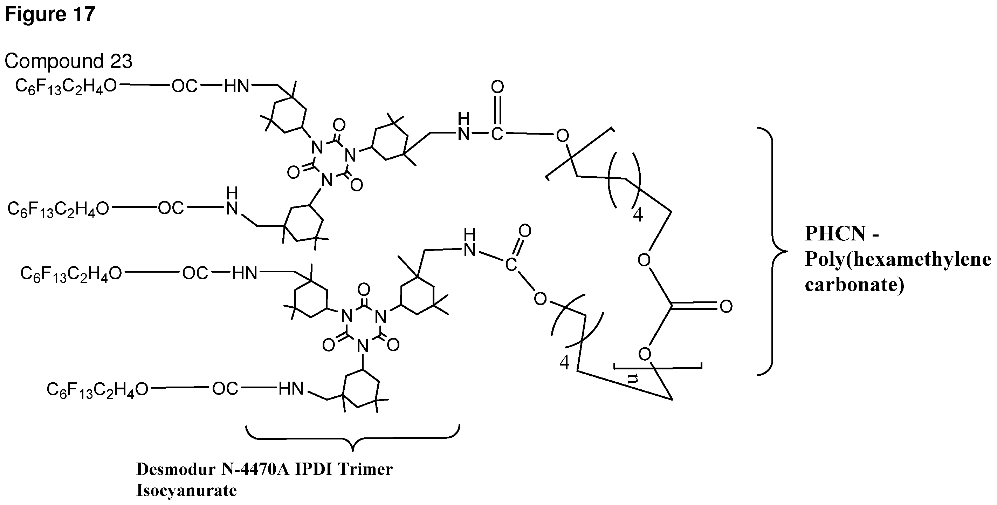

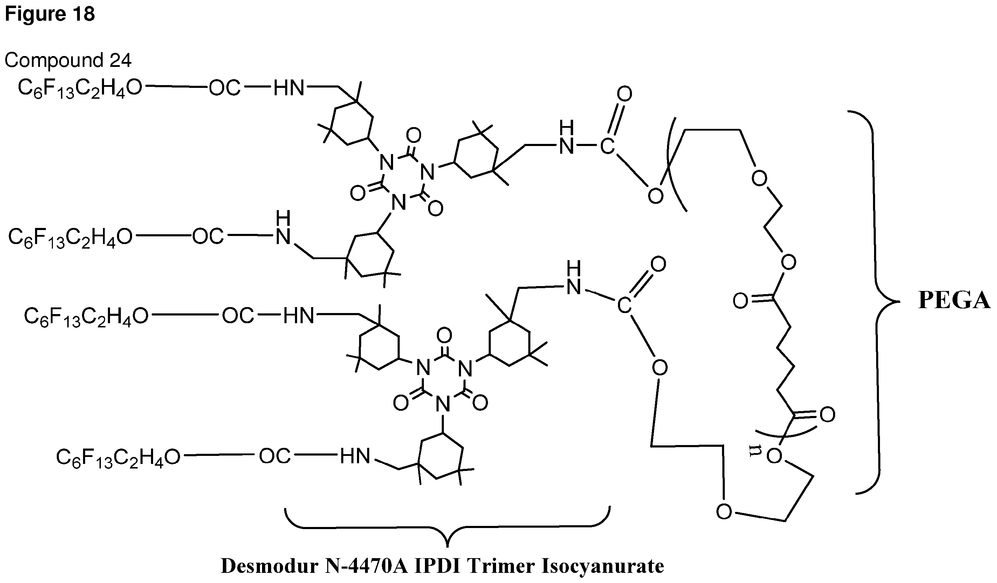

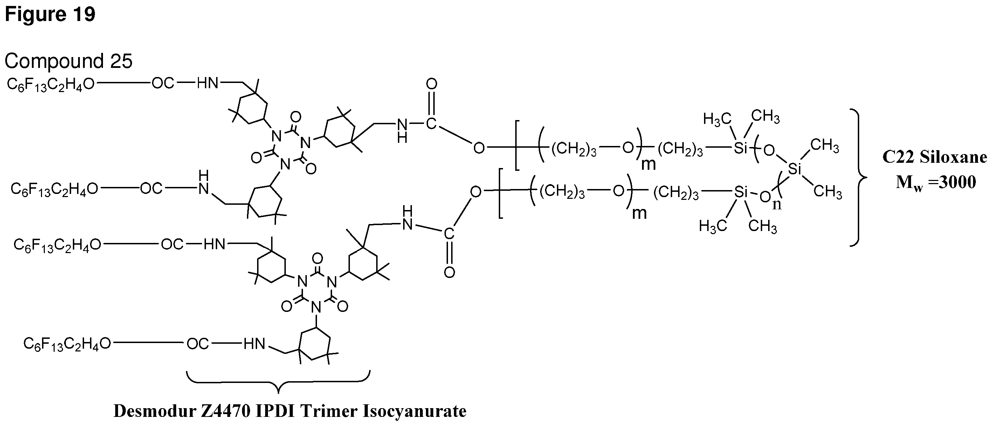

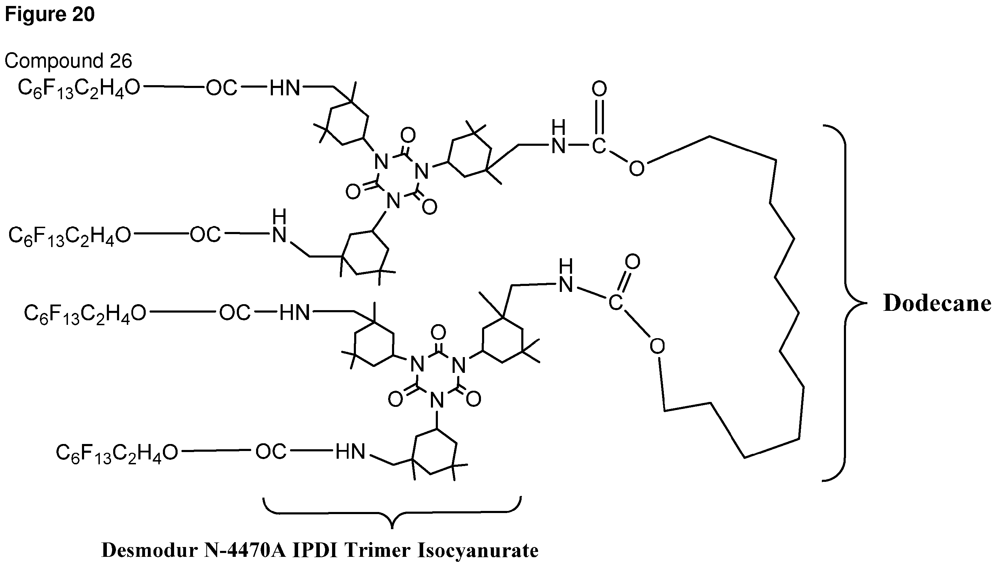

In some embodiments, the oligofluorinated additive is a structure described by any one of formulae (I)-(XVII). In certain embodiments, the oligofluorinated additive is any one of compounds 1-40. The theoretical structures of compounds 1-40 are illustrated in Figures 1-30.

The following examples are meant to illustrate the invention. They are not meant to limit the invention in any way.

### Examples

### Example 1. Preparation of Oligofluorinated Additives

The oligofluorinated additives used in the vascular grafts of the invention can be prepared using methods known in the art from the appropriately selected reagents, such as diisocyanates/triisocyanates, dicarboxylic acids, diols, and fluorinated alcohols to form a wide range of oligofluorinated additives. The reagents include but are not limited to the component reagents mentioned below.

### Diisocyanates

HMDI = 4,4'-methylene bis(cyclohexyl isocyanate)
IPDI = isophorone diisocyanate
TMXDI = m-tetramethylenexylene diisocyanate
HDI = hexamethylene diisocyanate

### Triisocyanates

Desmodur N3200 or Desmodur N-3200 = hexamethylene diisocyanate (HDI) biuret trimer
Desmodur Z4470A or Desmodur Z-4470A = isophorone diisocyanate (IPDI) trimer
Desmodur N3300 = hexamethylene diisocyanate (HDI) trimer

### Diols/Polyols

HLBH = hydrogenated-hydroxyl terminated polybutadiene,
PCN = poly(2,2-dimethyl-1-3-propylenecarbonate)diol
PHCN = poly(hexamethylene carbonate)diol
PEB = poly(ethylene-co-butylene)diol
LBHP = hydroxyl terminated polybutadiene polyol
PEGA = poly(diethylene glycol)adipate
PTMO = poly(tetramethylene oxide)diol
PDP = diethylene glycol-ortho phthalic anhydride polyester polyol
HHTPI = hydrogenated hydroxyl terminated polyisoprene
C22 = hydroxylterminated polydimethylsiloxanes block copolymer
C25 (diol) = hydroxy-terminated polidimethylsiloxane (ethylene oxide-PDMS-ethylene oxide) block copolymer
C10 (diol) = hydroxy-terminated polidimethylsiloxane (ethylene oxide-PDMS-ethylene oxide) block copolymer
PLN = poly(ethylene glycol)-*block*-poly(propylene glycol))-*block*-poly(ethylene glycol) polymer (PEO-PPO-PEO pluronic polymers)
PLN8K = poly(ethylene glycol)-*block*-poly(propylene glycol))-*block*-poly(ethylene glycol) polymer (PEO-PPO-PEO pluronic polymers)
DDD = 1,12-dodecanediol
SPH = 1,6-hexanediol-ortho phthalic anhydride polyester polyol
SPN = neopentyl glycol-ortho phthalic anhydride polyester polyol
BPAE = bisphenol A ethoxylate diol
YMer (diol) = hydroxy-terminated polyethylene glycol monomethyl ether
YMerOH(triol) = trimethylolpropane ethoxylate
XMer (tetraol) = pentaerythritol ethoxylate

### FLUORINATED END-CAPPING GROUPS

C6-FOH = (CF₃)(CF₂)₅CH₂CH₂OH (1H,1H,2H,2H perfluorooctanol)
C8-FOH = 1H,1H,2H,2H perfluorooctanol
C6-C8 FOH = (CF₃)(CF₂)₇CH₂CH₂OH and (CF₃)(CF₂)₅CH₂CH₂OH (mixtures of C6-FOH and C8-FOH; also designated as BAL-D)
C10-FOH = 1H,1H,2H,2H perfluorodecanol
C8-C10 FOH = mixtures of C8-FOH and C10-FOH
C5-FOH = 1 H,1H,5H-perfluoro-1-pentanol
C4-FOH = 1H,1H-perfluorobutanol
C3-FOH = (CF₃)(CF₂)₂CH₂OH (1H,1H perfluorobutanol)

### NON-TIN BASED CATALYST

Bi348 - bismuth carboxylate Type 1
Bi221- bismuth carboxylate Type 2
Bi601- bismuth carboxylate Type 3

The bismuth catalysts listed above can be purchased from King Industries (Norwalk CT). Any bismuth catalyst known in the art can be used to synthesize the oligofluorinated additives described herein. Also, tin-based catalysts (e.g., dibutyltin dilaurate) useful in the synthesis of polyurethanes may be used instead of the bismuth-based catalysts for the synthesis of the oligofluorinated additives described herein.

### Compound 1

Compound 1 was synthesized with PPO diol (MW = 1000 Da), 1,6-hexamethylene diisocyanate (HDI), and the low boiling fraction of the fluoroalcohol (BA-L). The conditions of the synthesis were as follows: 10 g of PPO were reacted with 3.36 g of HDI for 2 h, and then 5 g of BA-L (low boiling fraction) were added to the reaction. The mixture was reacted with 42.5 mg of the catalyst, dibutyltin dilaurate, in 130 mL of dimethylacetamide, and the reaction temperature for the prepolymer step was maintained within 60-70 °C. The polystyrene equivalent weight average molecular weight is 1.6+/-0.2×10⁴ Da and its total fluorine content is 18.87+/-2.38% by weight. Thermal transitions for compound 1 are detectable by differential scanning calorimetry. Two higher order thermal transitions at approximately 14 °C and 85 °C were observed. The theoretical chemical structure of the compound 1 is shown Figure 1A.

### Compound 2

All glassware used for the synthesis was dried in an oven at 110 °C overnight. To a 3-neck 1000 mL oven dried flask equipped with a stir bar was added 175 g (72 mmol) of hydrogenated-hydroxyl terminated polybutadiene (HLBH polyol, MW = 2000 Da). The flask with the polyol was degassed overnight and then purged with dry N₂. A 1000 mL graduated cylinder was filled with 525 mL anhydrous Toluene, sealed by a rubber septa and purged with dry N₂. The toluene was transferred to the 3-neck flask via a double-edged needle and the polyol stirred vigorously to dissolve in the solvent. The flask was placed in an oil bath at 65-70 °C. 39.70 g (151 mmol) of 4,4'-methylene bis(cyclohexyl isocyanate) (HMDI) was added to a degassed 250 mL flask equipped with a stir bar. To this flask was added 150 mL of anhydrous toluene from a degassed, N₂ purged 250 mL septasealed cylinder also using a double-edged needle and the mixture was stirred to dissolve the HMDI in the solvent. To a degassed 50 mL round bottom flask was added 8.75 g (5.00% w/w based on diol) of the bismuth carboxylate catalyst followed by 26 mL of toluene to dissolve the catalyst. The HMDI solution was transferred to the 1000 mL flask containing the polyol. The bismuth catalyst solution was added (20 mL) immediately following the addition of the HMDI. The reaction mixture was allowed to stir for 5 h at 70 °C to produce a HMDI-HLBH prepolymer.

In another 50 mL round bottom flask 74.95 g (180 mmol) of C8-C10 FOH (mixture of C8-FOH and C10-FOH) was added, capped with a septa, degassed and then purged with N₂. This was added to the 1000 mL flask containing prepolymer. All additions and transfers were conducted carefully in an atmosphere of dry N₂ to avoid any contact with air. The resulting mixture was heated to 45 °C for 18 h to produce SMM (1) with the end-capped C8-C10 FOH. The SMM solution was allowed to cool to ambient temperature and formed a milky solution. The milky solution was precipitated in MeOH (methanol) and the resulting precipitate was washed repeatedly with MeOH to form a white viscous material with dough-like consistency. This viscous, semi-solid material was washed twice in THF/EDTA (ethylene diamine tetraacetic acid) to remove residual catalyst followed by two more successive washes in THF/MeOH to remove unreacted monomers, low molecular weight byproducts, and catalyst residues. The SMM was first dried in a flow oven from at 40-120 °C in a period of 10 h gradually raising the temperature and finally dried under vacuum at 120 °C (24 h) and stored in a desiccator as a colorless rubbery semi-solid. The theoretical chemical structure of compound 2 is shown Figure 1B.

### Compound 3

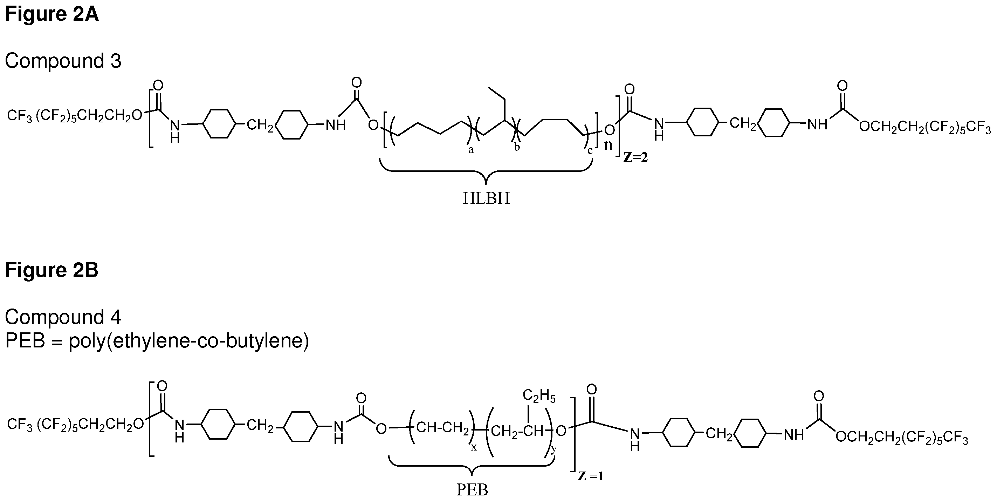

The reaction was carried out as described for compound 2 using 180 g (74 mmol) hydrogenated-hydroxyl terminated polybutadiene (HLBH polyol, MW = 2000 Da) and 30.14 g (115 mmol) of 4,4'-methylene-bis(cyclohexyl isocyanate) (HMDI) to form the prepolymer. The prepolymer was end-capped with 40.48 g (111.18 mmol) of 1H,1H,2H,2H-perfluoro-1-octanol (C8-FOH) to form compound 3 as a colorless rubbery semi-solid. As described above, the couplings were carried out in the presence of bismuth carboxylate catalyst, and compound 3 was washed similarly to compound 2 and dried prior to use. The theoretical chemical structure of compound 3 is shown in Figure 2a.

### Compound 4

The reaction was carried out as described for compound 3 using 10 g (4 mmol) poly(ethylene-co-butylene (PEB polyol, MW = 2500 Da) and 2.20 g (8.4 mmol) of 4,4'-methylene-bis(cyclohexyl isocyanate) (HMDI) to form the prepolymer. The prepolymer was capped with 3.64 g (10 mmol) of 1H, 1H, 2H, 2H-perfluoro-1-octanol (C8-FOH) to form compound 4. As described above, the couplings were carried out in the presence of bismuth carboxylate catalyst, and the compound 4 was washed similarly to compound 2 and dried prior to use. The theoretical chemical structure of compound 4 is shown in Figure 2B.

### Compound 5

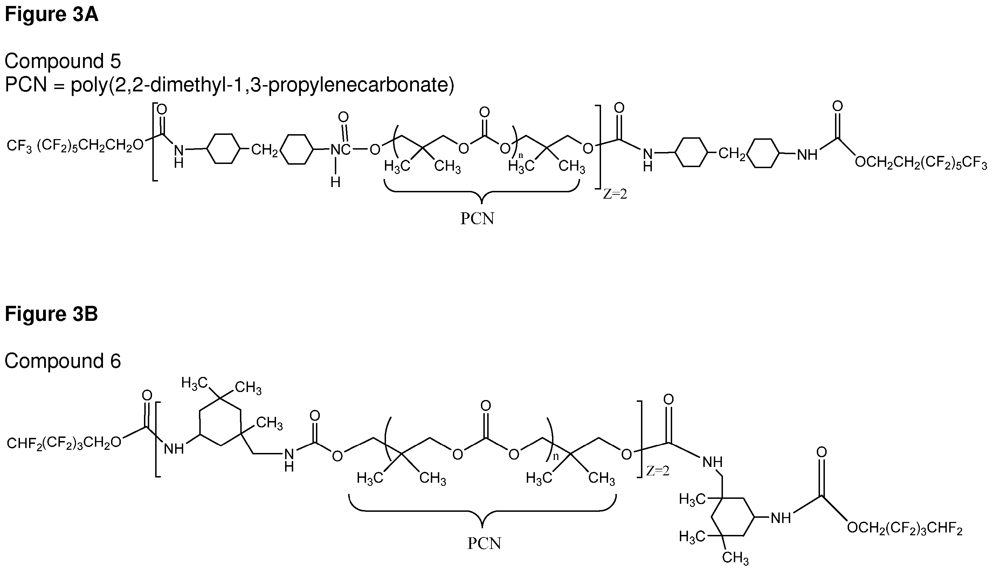

The reaction was carried out as described for compound 4, except the solvent was changed from toluene to DMAc. Here, 100 g (100 mmol) poly(2,2-dimethyl-1,3-propylenecarbonate) diol (PCN, MW = 1000 Da) and 40.7 g (155 mmol) of 4,4'-methylene-bis(cyclohexyl isocyanate) (HMDI) to form a prepolymer. The prepolymer was end-capped with 45.5 g (125 mmol) of 1H,1H,2H,2H-perfluoro-1-octanol (C8-FOH) to form compound 5. The work-up after the reaction and the subsequent washing procedures are modified from the compound 4 synthesis as follows. Compound 5 from the reaction mixture in DMAc was precipitated in distilled water and washed successively in IPA/EDTA (isopropanol/ethylene diamine tetraacetic acid) solution followed by another wash in IPA/hexanes to remove unreacted monomers, low molecular weight byproducts, and catalyst residues to yield compound 5 as a white amorphous powder. As described above, the couplings were carried out in the presence of bismuth carboxylate catalyst and dried under vacuum prior to use. The theoretical chemical structure of compound 5 is shown in Figure 3A.

### Compound 6

The reaction was carried out as described for compound 5 using 60 g (6.0 mmol) poly(2,2 dimethyl-1,3-propylenecarbonate) diol (MW = 1000 Da) and 1.90 g (8.5 mmol) of isophorone diisocyanate (IPDI) to form the prepolymer. The prepolymer was end-capped with 1.4 g (6.0 mmol) of 1H,1H,5H-perfluoro-1-pentanol (C5-FOH) to form compound 6 as a white amorphous solid. As described above, the couplings were carried out in the presence of bismuth carboxylate catalyst, and compound 6 was washed similarly to compound 5 and dried prior to use. The theoretical chemical structure of compound 6 is shown in Figure 3B.

### Compound 7

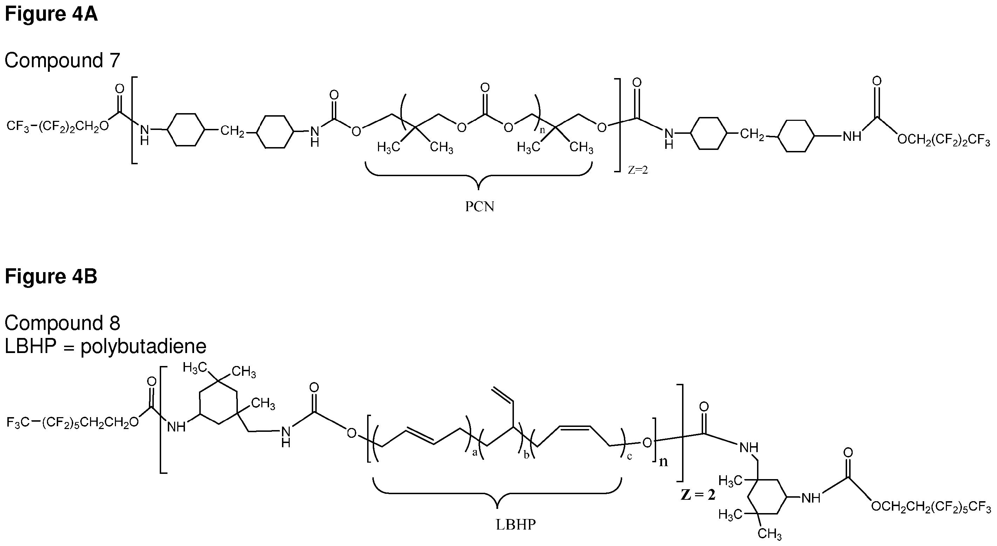

The reaction was carried out as described for compound 5 using 10.0 g (10.0 mmol) poly(2,2-dimethyl-1,3-propylenecarbonate) diol (MW = 1000 Da) and 4.07 g (15.5 mmol) of 4,4'-methylene-bis(cyclohexyl isocyanate) (HMDI) to form the prepolymer. The prepolymer was capped with 2.5 g (12.5 mmol) of 1H, 1H-perfluoro-1-butanol (C4-FOH) to form compound 7 as a white amorphous solid. As described above, the couplings were carried out in the presence of bismuth carboxylate catalyst, and compound 7 was washed similar to compound 5 and dried prior to use. The theoretical chemical structure of compound 7 is shown in Figure 4A.

### Compound 8

The reaction was carried out as described for compound 5 using 180 g (84.8 mmol) hydroxylterminated polybutadiene (LBHP polyol, MW = 2000 Da) and 29.21 g (131.42 mmol) of isophorone diisocyanate (IPDI) to form the prepolymer. The prepolymer was capped with 46.31 g (127.18 mmol) of 1H,1H,2H,2H-perfluoro-1-octanol (C8-FOH) to form compound 8 as an off-white opaque viscous liquid. As described above, the couplings were carried out in the presence of bismuth carboxylate catalyst, and compound 8 was washed similarly to compound 5 and dried prior to use. The theoretical chemical structure of compound 8 is shown in Figure 4B.

### Compound 9

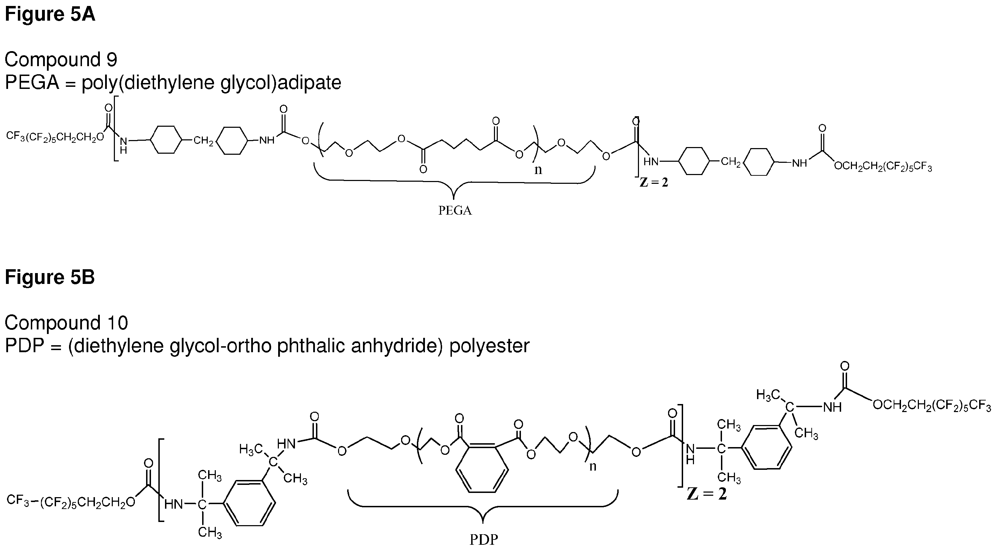

The reaction was carried out as described for compound 5 using 10 g (3.92 mmol) poly(diethyhlene glycol adipate) (PEGA polyol, MW = 2500 Da) and 1.59 g (6.08 mmol) of 4,4'-methylene-bis(cyclohexyl isocyanate) (HMDI) to form a prepolymer. The prepolymer was capped with 2.14 g (5.88 mmol) of 1H,1H,2H,2H-perfluoro-1-octanol (C8-FOH) to form compound 9 as an off-white opaque viscous liquid. As described above, the couplings were carried out in the presence of bismuth carboxylate catalyst, and compound 9 was washed similarly to compound 5 and dried prior to use. The theoretical chemical structure of compound 9 is shown in Figure 5A.

### Compound 10

The reaction was carried out as described for compound 5 using 10 g (5.06 mmol), ortho phthalate-diethylene glycol-based polyester polyol (PDP polyol, MW = 2000 Da) and 1.92 g (7.85 mmol) of m-tetramethylenexylene diisocyanate (TMXDI) to form a prepolymer. The prepolymer was capped with 2.76 g (7.59 mmol) of 1H,1H,2H,2H-perfluoro-1-octanol (C8-FOH) to form compound 10 as a colorless solid. As described above, the couplings were carried out in the presence of bismuth carboxylate catalyst, and compound 10 was washed similarly to compound 5 and dried prior to use. The theoretical chemical structure of compound 10 is shown in Figure 5B.

### Compound 11

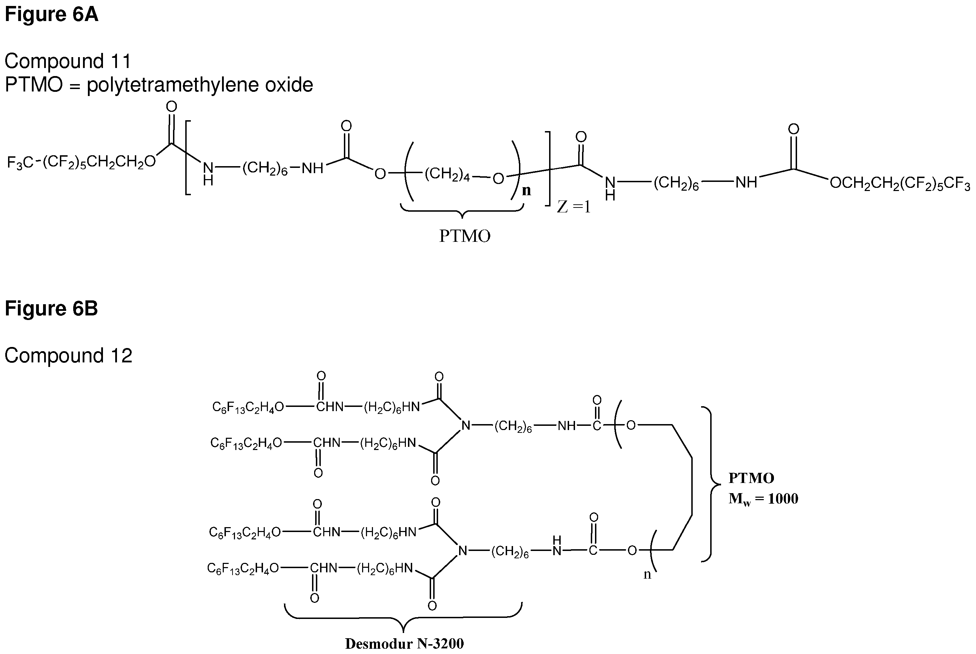

Compound 11 was synthesized with PTMO diol (MW = 1000 Da), 1,6-hexamethylene diisocyanate (HDI), and the low boiling fraction of the fluoroalcohol (BA-L). The conditions of the synthesis were as follows: 10 g of PTMO were reacted with 3.36 g of HDI for 2 h and then 9 g of BA-L (low boiling fraction) were added to the reaction. The mixture was reacted with 60 mL of the catalyst, dibutyltin dilaurate, in 70 mL of dimethyl-acetamide (DMAc), and the reaction temperature for the prepolymer step was maintained within 60-70 °C. The polystyrene equivalent weight average molecular weight is 3.0×10⁴ Da and its total fluorine content is 7.98% by weight. The theoretical chemical structure of compound 11 is shown in Figure 6A.

### Compounds 12-26

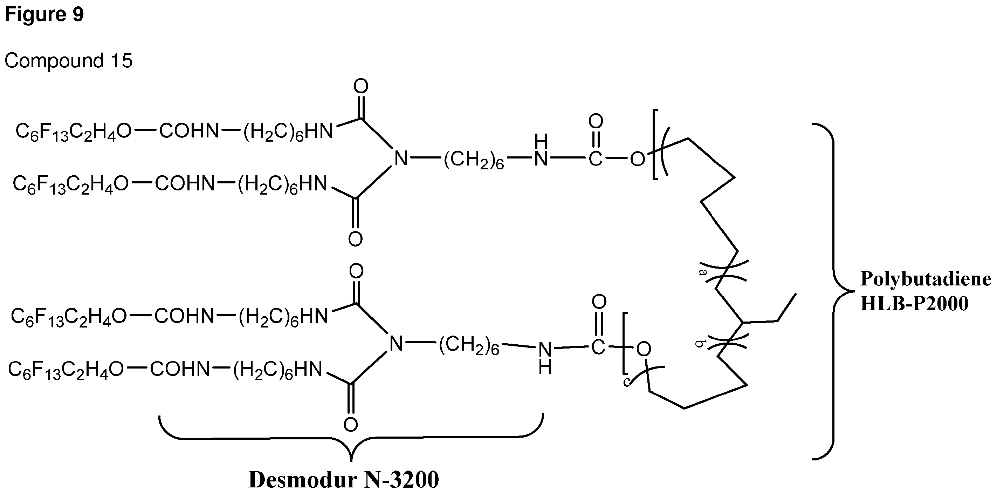

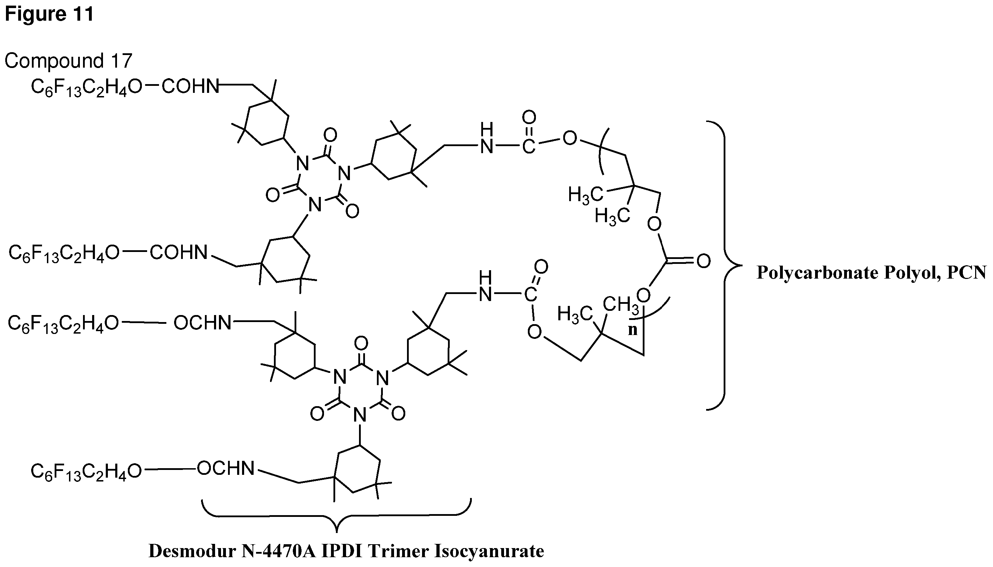

Surface modifiers of the invention such as compound 15 and compound 17 may be synthesized by a 2-step convergent method according to the schemes depicted in schemes 1 and 2. Briefly, the polyisocyanate such as Desmodur N3200 or Desmodur 4470 is reacted dropwise with the surface-active group (e.g., a fluoroalcohol) in an organic solvent (e.g., anhydrous THF or dimethylacetamide (DMAc)) in the presence of a catalyst at 25 °C for 2 h. After addition of the fluoroalcohol, stirring is continued for 1 h at 50 °C and for a further 1 h at 70 °C. These steps lead to the formation of a partially fluorinated intermediate that is then coupled with the polyol (e.g., hydrogenated-hydroxyl terminated polybutadiene, or poly(2,2-dimethyl-1,3-propylenecarbonate)diol) at 70 °C over a period of 14 h to provide the SMM. Because the reactions are moisture sensitive, they are carried out under an inert N₂ atmosphere and anhydrous conditions. The temperature profile is also maintained carefully, especially during the partial fluorination, to avoid unwanted side reactions. The reaction product is precipitated in MeOH and washed several times with additional MeOH. The catalyst residues are eliminated by first dissolving the oligofluorinated additive in hot THF or in hot IPA followed by reacting the oligofluorinated additive with EDTA solution, followed by precipitation in MeOH. Finally, the oligofluorinated additive is dried in a rotary evaporator at 120-140 °C prior to use. The theoretical chemical structure of compounds 15 and 17 is shown in Figures 9 and 11, respectively.

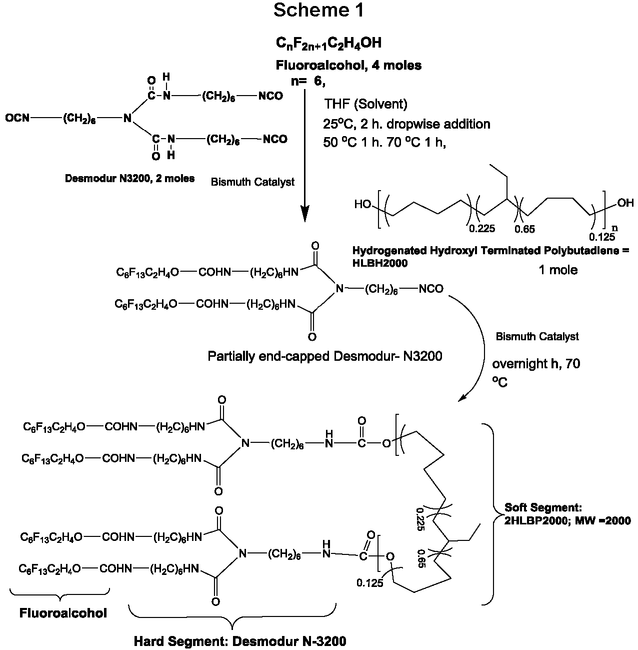

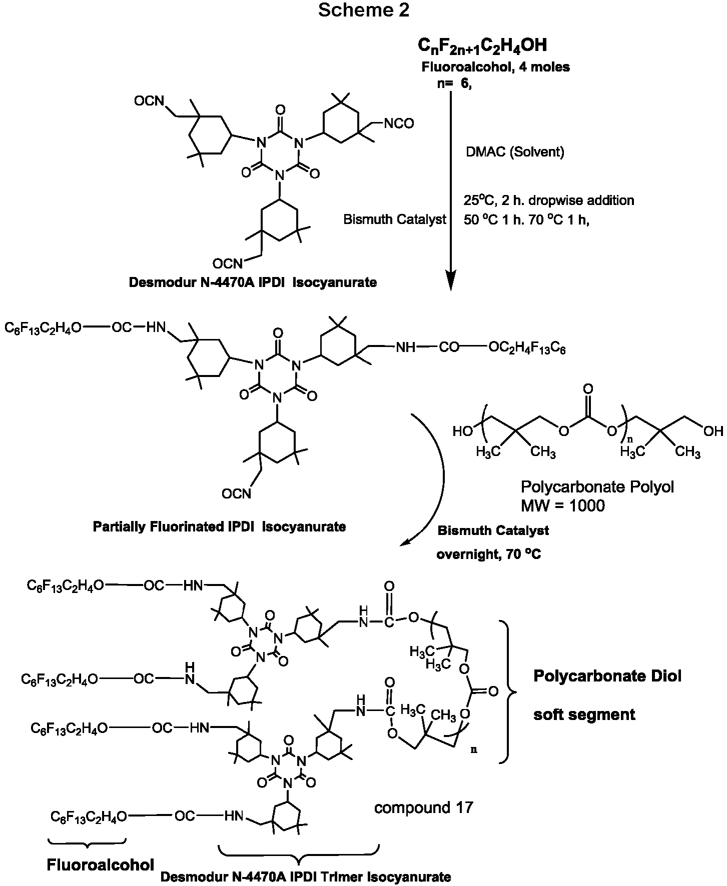

All glassware were dried in the oven overnight at 110 °C. To a 3-neck 5000 mL reactor equipped with a stir bar and a reflux condenser was added 300 g (583 mmol) of Desmodur N3300. The mixture was degassed overnight at ambient temperature. Hydrogenated-hydroxyl terminated polybutadiene (HLBH polyol MW = 2000 Da) was measured into a 2000 mL flask and degassed at 60 °C overnight. The bismuth catalyst K-Kat 348 (a bismuth carboxylate; available from King Industries) was measured out into a 250 mL flask and degassed overnight at ambient temperature. The perfluorinated alcohol was measured into a 1000 mL flask and degassed for 30 minutes at ambient temperature. After degassing, all the vessels were purged with N₂.

300 mL of THF (or DMAc) was then added to the Desmodur N3300 containing vessel, and the mixture was stirred to dissolve the polyisocyanate. Similarly, 622 mL of THF was added to the HLBH polyol, and the mixture was stirred to dissolve the polyol. Likewise, 428 mL of THF (or DMAC) was added to the perfluorinated alcohol and the mixture was stirred to dissolve. Similarly for K-Kat 348

which was dissolved in 77 mL of THF or DMAC. Stirring was continued to ensure all the reagents were dissolved in their respective vessels.

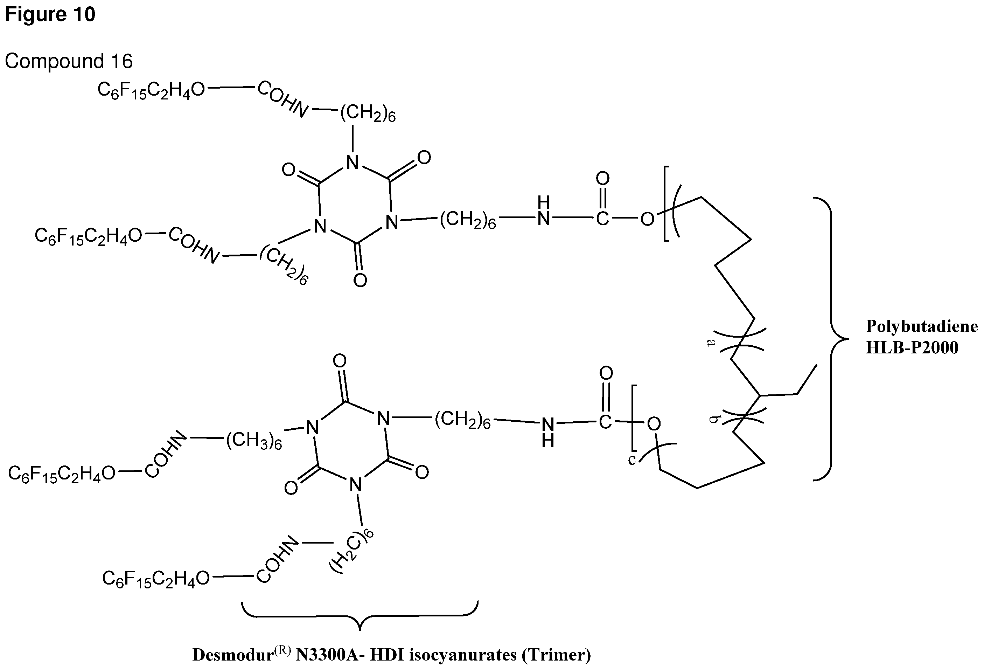

Half the K-Kat solution was transferred to the perfluorinated solution which was stirred for 5 minutes. This solution was added to the reaction vessel containing the Desmodur N3300 solution dropwise over a period of 2 h at ambient (25 °C) temperature through a cannula (double ended needle) under positive N₂ pressure. After addition, the temperature was raised to 50 °C for 1 h and 70 °C for another 1 h. Proper stirring was maintained throughout. The remaining K-Kat 348 catalyst was transferred to the HLBH-2000 flask; after stirring to dissolve, this was added to the reactor containing the N3300. The reaction mixture was allowed to react overnight for 14 h at 70 °C to produce compound 16 with four fluorinated end groups. The theoretical chemical structure of compound 16 is shown in Figure 10.

Exemplary oligofluorinated additives that can be prepared according to the procedures described for compounds 15-17 are illustrated in Figures 6B and 11-20.

### General Synthesis Description for Ester-based Oligofluorinated additives

A diol such as Ymer diol, hydroxyl terminated polydimethylsiloxane, or polyols such as trimethylolpropane ethoxylate or pentaerythritol ethoxylate are reacted in a one-step reaction with a surface-active group precursor (e.g., perfluoroheptanoyl chloride) at 40 °C in a chlorinated organic solvent e.g., chloroform or methylene chloride in the presence of an acid scavenger like pyridine or triethylamine for 24 h. This reaction end-caps the hydroxyl groups with polyfluoroorgano groups. Because the reactions are moisture sensitive, the reactions are carried out under a N₂ atmosphere using anhydrous solvents. After the reaction the solvent is rotary evaporated and the product is dissolved in Tetrahydrofuran (THF) which dissolves the product and precipitates the pyridine salts which are filtered off and the filtrate rotary evaporated further to dryness. The product is then purified by dissolving in minimum THF and precipitating in hexanes. This is performed three times and after which the final product is again rotary evaporated and finally dried in a vacuum oven at 60 °C overnight.

### Compound 27

Glassware used for the synthesis was dried in an oven at 110 °C overnight. To a 2-neck 1000 mL oven dried round bottom flask equipped with a stir bar was added 85 g (24 mmol) of C25-diol (MW = 3500 Da). The flask with the diol was degassed overnight at 60 °C with gentle stirring and then purged with dry N₂ the following day. The heating was turned off. A 1000 mL graduated cylinder was charged with 320 mL anhydrous CHCl₃, sealed by a rubber septa and purged with dry N₂. The CHCl₃ was transferred to the 2-neck flask via a cannula and the diol stirred vigorously to dissolve in the solvent. Anhydrous pyridine (11.53 g, 146 mmol) was added to the C25-diol solution using a plastic syringe, and the resulting mixture was stirred to dissolve all materials. Another oven dried 2-neck 1000 mL flask was charged with 32.51 g (85 mmol) of perfluoroheptanoyl chloride. The flask was sealed with rubber septa and degassed for 5 minutes, then purge with N₂. At this time 235 mL of anhydrous CHCl₃ were added via cannula to the 1000 mL 2-neck flask containing the perfluoroheptanoyl chloride. Stir at room temperature to dissolve the acid chloride. This flask was fitted with an addition funnel and the C25-diol-pyridine solution in CHCl₃ was transferred via a cannula into the addition funnel. N₂ flow through the reactor was adjusted to a slow and steady rate. Continuous drop-wise addition of C25-diol-pyridine solution to the acid chloride solution was started at room temperature and was continued over a period of ~4 h. Stirring was maintained at a sufficient speed to achieve good mixing of reagents. After completing addition of the C25-diol-pyridine solution, the addition funnel was replaced with an air condenser, and the 2-neck flask was immerses in an oil bath placed on a heater fitted with a thermocouple unit. The temperature was raised to 40 °C, and the reaction continued at this temperature under N₂ for 24 h.

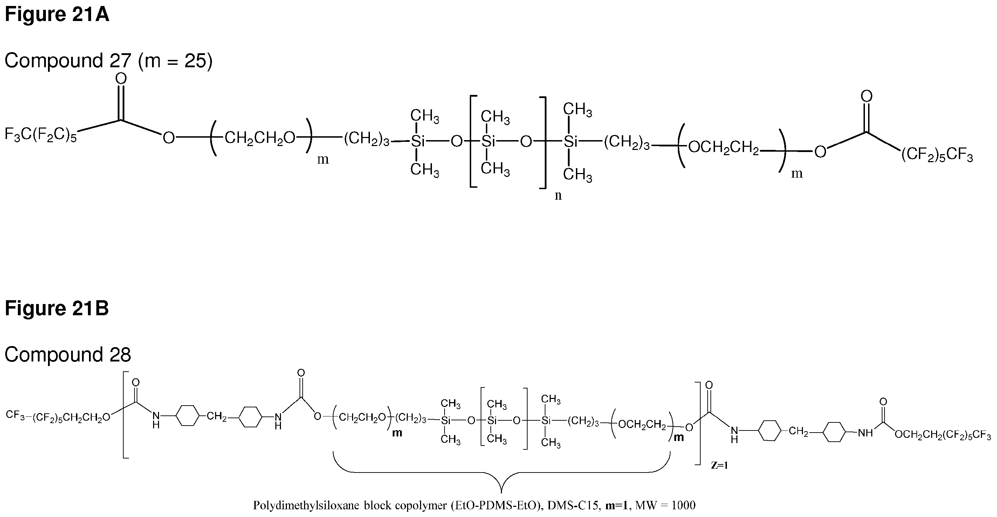

The product was purified by evaporating CHCl₃ in a rotary evaporator and by filtering the pyridine salts after addition of THF. The crude product was then precipitated in isopropanol/hexanes mixture twice. The oil from the IPA/Hexane that precipitated was subjected to further washing with hot hexanes as follows. About 500 mL of Hexanes was added to the oil in a 1 L beaker with a stir bar. The mixture was stirred while the Hexanes was heated to boiling. The heating was turned off, and the mixture was allowed to cool for 5 minutes. The oil settles at the bottom at which point the Hexane top layer is decanted. The isolated oil is further dissolved in THF, transferred to a round bottom flask and then the solvents rotary evaporated. The oil is finally dried in a vacuum oven at 40 °C for 24 h. The purified product (a mixture of di- and mono-substituted products) was characterized by GPC (molecular weight based on polystyrene standards), elemental analysis for fluorine, ¹⁹F NMR, ¹H NMR, FTIR, and TGA. Appearance: viscous oil. Weight average molecular weight (using polystyrene standards) = 5791 g/mol. Polydispersity: 2.85. Elemental analysis: F: 7.15% (theory: 10.53%). ¹⁹F NMR (CDCl₃, 400 MHz, ppm): δ -80.78 (m, CF₃), -118.43 (m, CF₂), -121.85 (m, CF₂), -122.62 (m, CF₂), -126.14 (m, CF₂). ¹H NMR (CDCl₃, 400 MHz, ppm): δ 0.0 (m, CH₃Si), 0.3 (br m, CH₂Si), 1.4 (br m, CH₂), 3.30 (m, CH₂'s), 4.30 (m, CH₂COO-). FTIR, neat (cm⁻¹): 3392 (OH), 2868 (CH₂), 1781 (O-C=O, ester), 1241, 1212, 1141, 1087 (CF₃, CF₂,). The theoretical chemical structure of compound 27 is shown in Figure 21A.

### Compound 29

Glassware used for the synthesis was dried in an oven at 110 °C overnight. To a 2-neck 100 mL oven dried round bottom flask equipped with a stir bar was added 10 g (5 mmol) of PDMS C22-diol (C22 diol, MW = 3000 Da). The flask with the diol was degassed overnight at 60 °C with gentle stirring and then purged with dry N₂ the following day. Heating was turned off. A 100 mL graduated cylinder was filled with 50 mL anhydrous CHCl₃, sealed with a rubber septum, and purged with dry N₂. The CHCl₃ was transferred to the 2-neck flask via a cannula, and the diol was stirred vigorously to dissolve in the solvent. Anhydrous pyridine (0.53 g, 7 mmol) was then added to the C22-diol solution using a plastic syringe, and the resulting mixture was stirred to dissolve all materials. Another oven-dried 2-neck 250 mL flask was charged with 3.19 g (8 mmol) perfluoroheptanoyl chloride. The flask was then sealed with a rubber septum, and the mixture in the flask was degassed for 5 minutes and purged with N₂. Then, 22 mL of anhydrous CHCl₃ were added using a graduated cylinder and a cannula to transfer the solvent to the 250 mL 2-neck flask containing the perfluoroheptanoyl chloride. The resulting mixture was stirred at room temperature to dissolve the acid chloride. The flask was then equipped with an addition funnel, and the C22-diol-pyridine solution in CHCl₃ was transferred to the addition funnel using a cannula. N₂ flow through the reactor was adjusted to a slow and steady rate. C22-diol-pyridine solution was then added continuously drop-wise to the acid chloride solution at room temperature over a period of ~4 h. Stirring was maintained at a sufficient speed to achieve good mixing of reagents. After completing the addition of the C22 diol, the addition funnel was replaced with an air condenser, and the 2-neck flask was immersed in an oil bath placed on a heater fitted with a thermocouple unit. The temperature was raised to 50 °C, and the reaction mixture was left at this temperature under N₂ for 24 h.

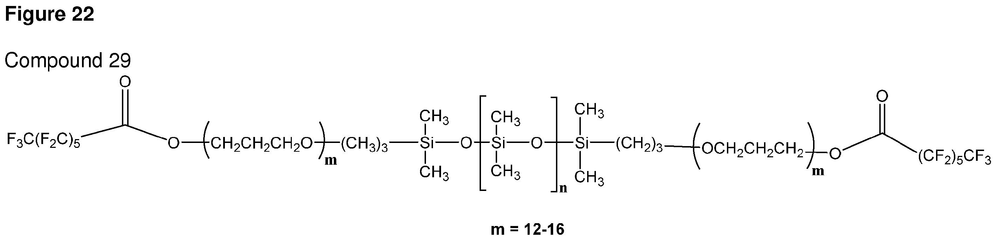

Then, heating and stirring were turned off. The flask was removed and its contents were poured into a round bottom flask. Volatiles were removed by rotary evaporation. Upon concentration, a dense precipitate (pyridine salts) formed. THF was added to dissolve the product, and the precipitated pyridine salts were removed by filtration using a coarse Whatman filter paper (No 4), as the pyridine salts are insoluble in THF. Volatiles were removed by rotary evaporation. The crude product was then dissolved in 100 mL of CHCl₃ and poured into a separatory funnel. 150 mL of water and 5 mL of 5 N HCl were added to neutralize any remaining pyridine. The funnel was shaken, and the product was extracted into CHCl₃. The bottom CHCl₃ layer containing product was then washed in a separatory funnel sequentially with water, 5 mL of 5% (w/v) NaHCO₃ solution to neutralize any remaining HCI, and with distilled water. The CHCl₃ layer was separated and concentrated by rotary evaporation to obtain crude product, which was then dissolved in 10 mL of isopropanol. The resulting solution was added dropwise to a 1 L beaker containing 200 mL of DI Water with 1% (v/v) MeOH with continuous stirring. The product separated out as oil, at which time the solution was kept in an ice bath for 20 minutes, and the top aqueous layer was decanted. The oil was dissolved in THF and transferred into a 200 mL round bottom flask. The volatiles were removed by rotary evaporation at a maximum of 80 °C and 4 mbar to remove residual solvents. The resulting product was dried in a vacuum oven at 60 °C for 24 h to give a purified product as a light yellow, clear oil (~64% yield). The purified product was characterized by GPC (molecular weight based on polystyrene standards), and elemental analysis (for fluorine). Appearance: light yellow clear oil. Weight average molecular weight (using polystyrene standards) Mw = 5589 Da, Polydispersity PD = 1.15. Elemental Analysis F: 12.86% (theory: 13.12%). The theoretical chemical structure of compound 29 is shown in Figure 22.

### Compound 30

Glassware used for the synthesis was dried in an oven at 110 °C overnight. To a 2-neck 250 mL oven dried round bottom flask equipped with a stir bar was added 20 g (8.0 mmol) of hydrogenated-hydroxyl terminated polybutadiene (HLBH diol, MW = 2000 Da). The flask with the diol was degassed overnight at 60 °C with gentle stirring and then purged with dry N₂ the following day. At this time, the heating was turned off. A 200 mL graduated cylinder was charged with 104 mL anhydrous CHCl₃, sealed by a rubber septa, and purged with dry N₂. The CHCl₃ was transferred to the 2-neck flask via a cannula, and the diol was stirred vigorously to dissolve in the solvent. At this time, anhydrous pyridine (3.82 g, 48 mmol) was added to the HLBH diol solution using a plastic syringe, and the resulting mixture was stirred to dissolve all materials. Another oven dried 2-neck 100 mL flask was charged with trans-5-norbornene-2,3-dicarbonyl chloride ("NCl"; 3.70 g, 17 mmol), sealed with rubber septa, and degassed for 5 minutes, and then purged with N₂. At this time, 52 mL of anhydrous CHCl₃ were added using a graduated cylinder and a cannula to transfer the solvent to the 100 mL 2-neck flask containing NCI. The resulting mixture was stirred to dissolve NCI. The 250 mL 2-neck flask was then fitted with an addition funnel, and the solution of NCI in CHCl₃ was transferred to the addition funnel using a cannula. N₂ flow was adjusted through the reactor to a slow and steady rate. The solution of NCI was added continuously drop-wise to the HLBH-pyridine solution at room temperature over a period of ~1 h to form a pre-polymer. Stirring was maintained at a sufficient speed to achieve good mixing of reagents.

In parallel, another oven-dried 50 mL flask was charged with Capstone^{™} Al-62 perfluorinated reagent (5.45 g, 15 mmol). The flask was sealed with rubber septa, degassed for 15 minutes, and purged with N₂. Anhydrous CHCl₃ (17 mL) and anhydrous pyridine (1.9 g, 24 mmol) were added. The mixture was stirred to dissolve all reagents. After the addition of the NCI solution to the 250 mL 2-neck flask was complete, the Capstone^{™} Al-62 perfluorinated reagent solution was added to this flask using a cannula with stirring. The addition funnel was replaced with an air condenser, and the 250 mL 2-neck flask was immersed in an oil bath placed on a heater fitted with a thermocouple unit. The temperature was raised to 50 °C, and the reaction continued at this temperature under N₂ for 24 h.

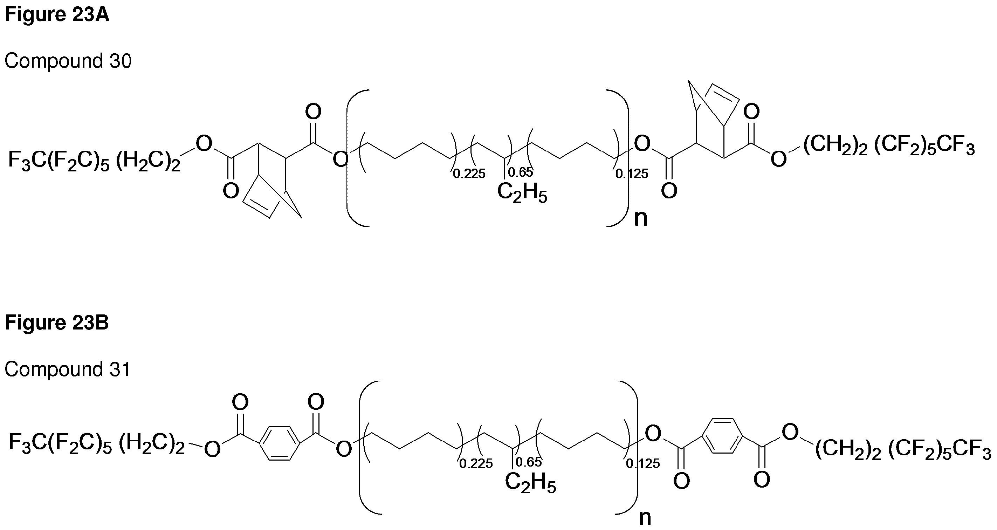

After the reaction, heating and stirring were turned off. The reaction flask was removed, and its contents were poured into a round bottom flask. CHCl₃ was removed by rotary evaporation. Upon concentration, a dense precipitate (pyridine salts) formed. THF was added to dissolve the product, and the precipitated pyridine salts were removed by filtration using a coarse Whatman filter paper (No 4). Pyridine salts are insoluble in THF. THF was removed by rotary evaporation. The crude product was dissolved in 100 mL of CHCl₃ and was poured into a separatory funnel. 100 mL of water were added, followed by the addition of 5 mL of 5 N HCI to neutralize any remaining pyridine. The funnel was shaken, and the product was extracted into CHCl₃. The bottom CHCl₃ layer containing product was isolated and washed in a separatory funnel with water (5 mL of 5% NaHCO₃ aqueous solution were added to neutralize any remaining HCI). The organic layer was then washed once more with plain distilled water. Isolated CHCl₃ layer was concentrated by rotary evaporation to obtain crude product. The crude product was dissolved in 10 mL of isopropanol (IPA) and was then added dropwise to a beaker containing 200 mL of deionized water containing 1% (v/v) MeOH with continuous stirring. Product separated out as an oil. The mixture was kept in ice bath for 20 minutes, and the top water layer was decanted. The oil was dissolved in THF and transferred into 200 mL round bottom flask. THF was removed by rotary evaporation at a maximum temperature of 80 °C and 4 mbar to remove all residual solvents. The resulting product was dried in a vacuum oven at 60 °C for 24 h to give a purified product as a viscous oil (~55% yield). The purified product (a mixture of di- and mono-substituted products) was characterized by GPC, elemental analysis, for fluorine, and Hi-Res TGA. Appearance: light yellow viscous liquid. Weight average molecular weight (using polystyrene standards) = 12389 g/mol. Polydispersity, PD: 1.43. Elemental analysis: F: 10.6% (theory: 14.08%). The theoretical chemical structure of compound 30 is shown in Figure 23A.

### Compound 31

Compound 31 was prepared according to a procedure similar to compound 30. Glassware used for the synthesis was dried in an oven at 110 °C overnight. To a 2-neck 250 mL oven dried round bottom flask equipped with a stir bar was added 15 g (6.0 mmol) of hydrogenated-hydroxyl terminated polybutadiene (HLBH diol, MW = 2000 Da). The flask with the diol was degassed overnight at 60 °C with gentle stirring and then purged with dry N₂ the following day. At this time, the heating was turned off. A 100 mL graduated cylinder was charged with 12 mL anhydrous CHCl₃, sealed by a rubber septa, and purged with dry N₂. The CHCl₃ was transferred to the 2-neck flask via a cannula, and the diol was stirred vigorously to dissolve in the solvent. At this time, anhydrous pyridine (0.95 g, 12 mmol) was added to the HLBH diol solution using a plastic syringe, and the resulting mixture was stirred to dissolve all materials. Another oven dried 2-neck 100 mL flask was charged with terephthaloyl chloride (2.57 g, 13 mmol), sealed with rubber septa, and degassed for 5 minutes, and then purged with N₂. At this time, 85 mL of anhydrous CHCl₃ were added using a graduated cylinder and a cannula to transfer the solvent to the 100 mL 2-neck flask. The resulting mixture was stirred to dissolve terephthaloyl chloride. The 250 mL 2-neck flask was then fitted with an addition funnel, and the solution of terephthaloyl chloride in CHCl₃ was transferred to the addition funnel using a cannula. N₂ flow was adjusted through the reactor to a slow and steady rate. The solution of terephthaloyl chloride was added continuously drop-wise to the HLBH-pyridine solution at room temperature over a period of ~1 h to form a pre-polymer. Stirring was maintained at a sufficient speed to achieve good mixing of reagents.

In parallel, another oven-dried 50 mL flask was charged with Capstone^{™} Al-62 perfluorinated reagent (5.45 g, 15 mmol). The flask was sealed with rubber septa, degassed for 15 minutes, and purged with N₂. Anhydrous CHCl₃ (12 mL) and anhydrous pyridine (0.95 g, 12 mmol) were added. The mixture was stirred to dissolve all reagents. After the addition of the terephthaloyl chloride solution to the 250 mL 2-neck flask was complete, the Capstone^{™} Al-62 perfluorinated reagent solution was added to this flask with stirring. The addition funnel was replaced with an air condenser, and the 250 mL 2-neck flask was immersed in an oil bath placed on a heater fitted with a thermocouple unit. The temperature was raised to 50 °C, and the reaction continued at this temperature under N₂ for 24 h.

After the reaction, heating and stirring were turned off. The reaction flask was removed, and its contents were poured into a round bottom flask. CHCl₃ was removed by rotary evaporation. Upon concentration, a dense precipitate (pyridine salts) formed. THF was added to dissolve the product, and the precipitated pyridine salts were removed by filtration using a coarse Whatman filter paper (No 4). Pyridine salts are insoluble in THF. THF was removed by rotary evaporation. The crude product was dissolved in 100 mL of CHCl₃ and was poured into a separatory funnel. 100 mL of water were added, followed by the addition of 5 mL of 5 N HCI to neutralize any remaining pyridine. The funnel was shaken, and the product was extracted into CHCl₃. The bottom CHCl₃ layer containing product was isolated and washed in a separatory funnel with water (5 mL of 5% NaHCO₃ aqueous solution were added to neutralize any remaining HCI). The organic layer was then washed once more with plain distilled water. Isolated CHCl₃ layer was concentrated by rotary evaporation to obtain crude product. The crude product was dissolved in 10 mL of isopropanol (IPA) and was then added dropwise to a beaker containing 200 mL of deionized water containing 1% (v/v) MeOH with continuous stirring. Product separated out as an oil. The mixture was kept in ice bath for 20 minutes, and the top water layer was decanted. The oil was dissolved in THF and transferred into 200 mL round bottom flask. THF was removed by rotary evaporation at a maximum temperature of 80 °C and 4 mbar to remove all residual solvents. The resulting product was dried in a vacuum oven at 60 °C for 24 h to give a purified product as a viscous oil (~87% yield). The purified product (a mixture of di- and mono-substituted products) was characterized by GPC, elemental analysis, for fluorine, and Hi-Res TGA. Appearance: off-white viscous liquid. Weight average molecular weight (using polystyrene standards) = 10757 g/mol. Polydispersity, PD: 1.33. Elemental analysis: F: 11.29% (theory: 14.21%). The theoretical chemical structure of compound 31 is shown in Figure 23B.

### Compound 33

Glassware used for the synthesis was dried in an oven at 110 °C overnight. To a 2-neck 100 mL oven dried round bottom flask equipped with a stir bar was added 10 g (5 mmol) of hydrogenated-hydroxyl terminated polyisoprene (HHTPI diol, MW = 2000 Da). The flask with the diol was degassed overnight at 60 °C with gentle stirring and then purged with dry N₂ the following day. At this time, the heating was turned off. A 100 mL graduated cylinder was charged with 50 mL anhydrous CHCl₃, sealed by a rubber septa, and purged with dry N₂. The CHCl₃ was transferred to the 2-neck flask via a cannula, and the diol was stirred vigorously to dissolve in the solvent. At this time, excess anhydrous pyridine (0.75 g, 9 mmol) was added to the HHTPI diol solution using a plastic syringe, and the resulting mixture was stirred to dissolve all materials. Another oven dried 2-neck 250 mL flask was charged with perfluoroheptanoyl chloride (4.51 g, 12 mmol), sealed with rubber septa, and degassed for 5 minutes, and then purged with N₂. At this time, 22 mL of anhydrous CHCl₃ was added using a graduated cylinder and a cannula to transfer the solvent to the 250 mL 2-neck flask containing the perfluoroheptanoyl chloride. The resulting mixture was stirred at room temperature to dissolve the acid chloride. An addition funnel was fitted to this flask, and the HHTPI-pyridine solution in CHCl₃ was added into the addition funnel. N₂ flow was adjusted through the reactor to a slow and steady rate. HHTPI-Pyridine solution was added continuously drop-wise to the acid chloride solution at room temperature over a period of ~4 h. Stirring was maintained at a sufficient speed to achieve good mixing of reagents. After completing addition of the HHTPI diol, the addition funnel was replaced with an air condenser, and the 2-neck flask was immersed in an oil bath on a heater fitted with a thermocouple unit. The temperature was raised to 50 °C, and the reaction continued at this temperature under N₂ for 24 h.

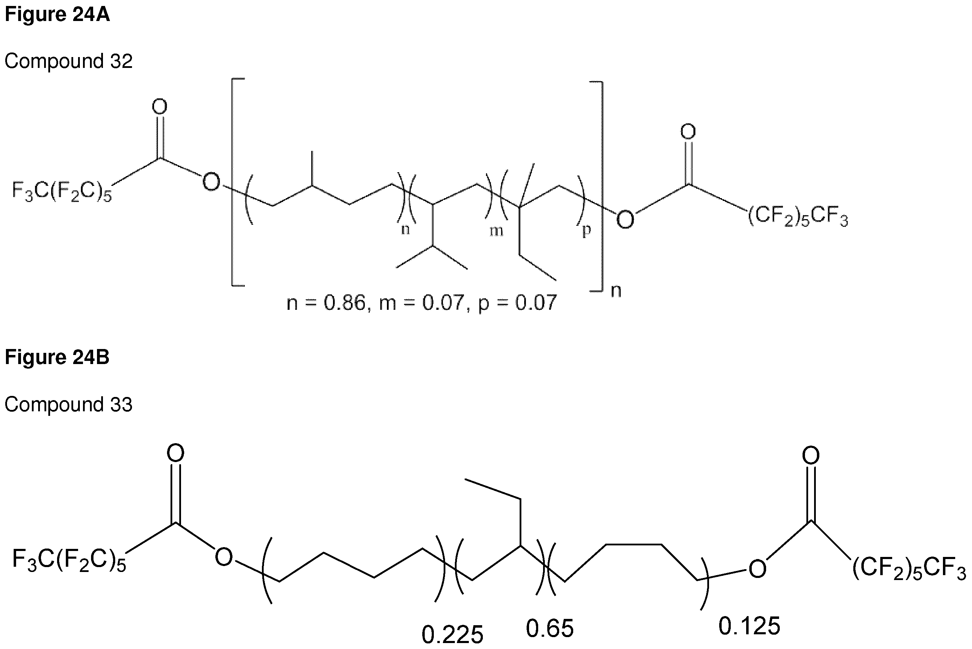

After the reaction, heating and stirring were turned off. The reaction flask was removed, and its contents were poured into a round bottom flask. CHCl₃ was removed by rotary evaporation. Upon concentration, a dense precipitate (pyridine salts) formed. THF was added to dissolve the product, and the precipitated pyridine salts were removed by filtration using a coarse Whatman filter paper (No 4). Pyridine salts are insoluble in THF. THF was removed by rotary evaporation. The crude product was dissolved in 100 mL of CHCl₃ and was poured into a separatory funnel. 150 mL of water were added, followed by the addition of 5 mL of 5 N HCI to neutralize any remaining pyridine. The funnel was shaken, and the product was extracted into CHCl₃. The bottom CHCl₃ layer containing product was isolated and washed in separatory funnel with water (5 mL of 5% NaHCO₃ aqueous solution were added to neutralize any remaining HCI). The organic layer was then washed once more with plain distilled water. Isolated CHCl₃ layer was concentrated by rotary evaporation to obtain crude product. The crude product was dissolved in 10 mL of isopropanol (IPA) and was added dropwise to a 1L beaker containing 200 mL of deionized water containing 1% (v/v) MeOH with continuous stirring. Product separated out as an oil. The mixture was kept in ice bath for 20 minutes, and the top water layer was decanted. The oil was dissolved in THF and transferred into 200 mL round bottom flask. THF was removed by rotary evaporation at a maximum temperature of 80 °C and 4 mbar to remove all residual solvents. The resulting product was dried in a vacuum oven at 60 °C for 24 h to give a purified product as a colorless viscous oil (~99% yield). The purified product (a mixture of di- and mono-substituted products) was characterized by GPC, elemental analysis, for fluorine, and Hi-Res TGA. Appearance: colorless viscous liquid. Weight average molecular weight (using polystyrene standards) = 12622 g/mol. Polydispersity, PD: 1.53. Elemental analysis: F: 13.50% (theory: 17.13%). The theoretical chemical structure of compound 32 is shown in Figure 24A.

### Compound 33

Glassware used for the synthesis was dried in an oven at 110 °C overnight. To a 2-neck 1000 mL oven dried round bottom flask equipped with a stir bar was added 100 g (40 mmol) of Hydrogenated-hydroxyl terminated polybutadiene (HLBH diol, MW = 2000 Da). The flask with the diol was degassed overnight at 60 °C with gentle stirring and then purged with dry N₂ the following day. At this time, the heating was turned off. A 1000 mL graduated cylinder was charged with 415 mL anhydrous CHCl₃, sealed by a rubber septa, and purged with dry N₂. The CHCl₃ was transferred to the 2-neck flask via a cannula, and the diol was stirred vigorously to dissolve in the solvent. Now excess anhydrous pyridine (19.08 g, 241 mmol) was added to the HLBH diol solution using a plastic syringe, and the resulting mixture was stirred to dissolve all materials. Another oven dried 2-neck 1000 mL flask was charged with 38.45 g, (101 mmol) perfluoroheptanoyl chloride, sealed with rubber septa, and degassed for 5 minutes, and then purged with N₂. At this time, 277 mL of anhydrous CHCl₃ was added using a graduated cylinder and a cannula to transfer the solvent to the 1000 mL 2-neck flask containing the perfluoroheptanoyl chloride. The resulting mixture was stirred at room temperature to dissolve the acid chloride. An addition funnel was fitted to this flask, and the HLBH-pyridine solution in CHCl₃ was added into the addition funnel using a cannula. N₂ flow was adjusted through the reactor to a slow and steady rate. Continuous drop-wise addition of HLBH-Pyridine solution to the acid chloride solution was started at room temperature over a period of ~4 h. Stirring was maintained at a sufficient speed to achieve good mixing of reagents. After completing addition of the HLBH, the addition funnel was replaced with an air condenser, and the 2-neck flask was immersed in an oil bath on a heater fitted with a thermocouple unit. The temperature was raised to 50 °C, and the reaction continued at this temperature under N₂ for 24 h.

After the reaction, heating and stirring were turned off. The reaction flask was removed, and its contents were poured into a round bottom flask. CHCl₃ was removed by rotary evaporation. Upon concentration, a dense precipitate (pyridine salts) formed. THF was added to dissolve the product, and the precipitated pyridine salts were removed by filtration using a coarse Whatman filter paper (No 4). Pyridine salts are insoluble in THF. THF was removed by rotary evaporation. The crude product was dissolved in 400 mL of CHCl₃ and was poured into a separatory funnel. 500 mL of water were added, followed by the addition of 20 mL of 5 N HCI to neutralize any remaining pyridine. The funnel was shaken, and the product was extracted into CHCl₃. The bottom CHCl₃ layer containing product was isolated, and washed in a separatory funnel with water (20 mL of 5% NaHCO₃ aqueous solution were added to neutralize any remaining HCI). The organic layer was then washed once more with plain distilled water. Isolated CHCl₃ layer was concentrated by rotary evaporation to obtain crude product. The crude product was dissolved in 20 mL of THF and was then added dropwise to a 4 L beaker containing 1200 mL of deionized water containing 1% (v/v) MeOH with continuous stirring. Product separated out as an oil. The mixture was kept in ice bath for 20 minutes, and the top hexane layer was decanted. The oil was dissolved in THF and transferred into 500 mL round bottom flask. THF was removed by rotary evaporation at a maximum temperature of 80 °C and 4 mbar to remove all residual solvents. The resulting product was dried in a vacuum oven at 60 °C for 24 h to give a purified product as a yellow viscous oil (~80% yield). The purified product (a mixture of di- and mono-substituted products) was characterized by GPC, elemental analysis for fluorine and Hi-Res TGA. Appearance: light yellow viscous liquid. Weight average molecular weight (using polystyrene standards) = 6099 g/mol. Polydispersity, PD: 1.08. Elemental analysis: F: 12.84% (theory: 15.54%). The theoretical chemical structure of compound 33 is shown in Figure 24B.

### Compound 34

Glassware used for the synthesis was dried in an oven at 110 °C overnight. To a 2-neck 1000 mL oven dried round bottom flask equipped with a stir bar was added 65 g (63 mmol) of YMer-diol (MW = 1000 Da). The flask with the diol was degassed overnight at 60 °C with gentle stirring and then purged with dry N₂ the following day. At this time, heating was turned off. A 1000 mL graduated cylinder was charged with 374 mL anhydrous CHCl₃, sealed by rubber septa, and purged with dry N₂. The CHCl₃ was transferred to the 2-neck flask via a cannula, and the diol was stirred vigorously to dissolve in the solvent. Excess anhydrous pyridine (30 g, 375 mmol) was added to the YMer-diol solution using a plastic syringe, the resulting stir to dissolve all materials. Another oven dried 2-neck 1000 mL flask was charged with 59.82 g (156 mmol) of perfluoroheptanoyl chloride, sealed with rubber septa, and degassed for 5 minutes, then purged with N₂. At this time 250 mL of anhydrous CHCl₃ were added using a graduated cylinder and cannula to transfer the solvent to the 1000 mL 2-neck flask containing the perfluoroheptanoyl chloride. The resulting mixture was stirred at room temperature to dissolve the acid chloride. An addition funnel was fitted to this flask and using a cannula transfer the YMer-diol-pyridine solution in CHCl₃ into the addition funnel. N₂ flow through the reactor was adjusted to a slow and steady rate. YMer-diol-pyridine solution was added drop-wise, continuously to the acid chloride solution at room temperature over a period of ~4 h. Stirring was maintained at a sufficient speed to achieve good mixing of reagents. After completing the addition of the YMer-diol-pyridine solution, the addition funnel was replaced with an air condenser, and the 2-neck flask was immersed in an oil bath placed on a heater fitted with a thermocouple unit. The temperature was raised to 40 °C, and the reaction continued at this temperature under N₂ for 24 h.

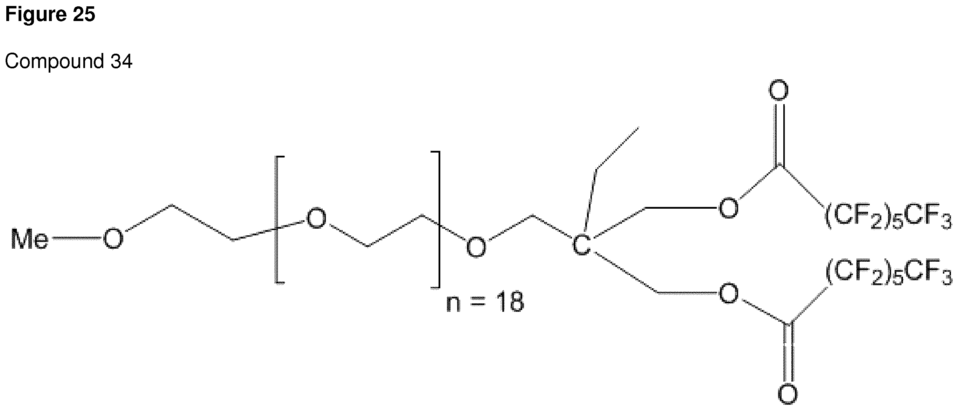

After the reaction, heating and stirring were turned off. The reaction flask was removed, and the contents were poured into a round bottom flask. CHCl₃ was removed by rotary evaporation. Upon concentration, a dense precipitate (pyridine salts) formed. THF was added to dissolve the product. The flask was cooled in an ice bath for 20 minutes, at which time, the precipitated pyridine salts were removed by gravity filtration using a coarse Whatman filter paper (No 4). Pyridine salts are insoluble in THF. THF was removed by rotary evaporation. The resulting crude product was dissolved in a minimum quantity of Isopropanol (IPA), and this solution was added to 700 mL of hexanes in a beaker with a stir bar. An oil separated out. The top layer was decanted and washed once with 200 mL of hexanes. The residue was then dissolved in 200 mL of THF and transferred to a 500 mL round bottom flask. Rotary evaporation of the solvents at a maximum temperature of 75 °C and 4 mbar vacuum furnished an oil, which was then transferred to a wide mouth jar and further dried for 24 h at 60 °C under vacuum to yield the pure product which solidifies upon cooling at room temperature to an off white waxy semi-solid (82% yield). The purified product was characterized by GPC (molecular weight based on polystyrene standards), elemental analysis for fluorine, ¹⁹F NMR, ¹H NMR, FTIR and TGA. Appearance: waxy semi-solid. Weight average molecular weight (using polystyrene standards) = 2498 g/mol. Polydispersity: 1.04. Elemental Analysis: F: 27.79% (theory: 28.54%). ¹⁹F NMR (CDCl₃, 400 MHz, ppm): δ -81.3 (m, CF₃), -118.88 (m, CF₂), -122.37 (m, CF₂), - 123.28 (m, CF₂), -126 (m, CF₂). ¹H NMR (CDCl₃, 400 MHz, ppm): δ 0.83 (t, CH₃CH₂), 1.44 (q, CH₂CH₃), 3.34 (m, CH₂), 3.51 (m, CH₂), 3.54 (m, CH₂), 4.30 (m, CH₂COO-). FTIR, neat (cm⁻¹): 2882 (CH2), 1783 (O-C=O, ester), 1235, 1203, 1143, 1104 (CF₃, CF₂). The theoretical chemical structure of compound 34 is shown in Figure 25.

### Compound 35

Compound 35 was prepared according to a procedure similar to that used for the preparation of compound 34.

Glassware used for the synthesis was dried in an oven at 110 °C overnight. To a 2-neck 1000 mL oven dried round bottom flask equipped with a stir bar was added 60 g (59 mmol) of YMerOH-triol (MW = 1014 Da). The flask with the triol was degassed overnight at 60 °C with gentle stirring and then purged with dry N₂ the following day. Heating was turned off. A 1000 mL graduated cylinder was charged with 435 mL anhydrous CHCl₃, sealed with rubber septa, and purged with dry N₂. The CHCl₃ liquid was transferred to the 2-neck flask via a cannula, and the triol was stirred vigorously to dissolve in the solvent. Excess anhydrous pyridine (37 g, 473 mmol) was added to the YMer-triol solution using a plastic syringe, the resulting mixture was stirred to dissolve all materials. Another oven dried 2-neck 1000 mL flask was charged with 84.88 g (222 mmol) of perfluoroheptanoyl chloride, sealed with rubber septa, and degassed for 5 minutes, then purged with N₂. 290 mL of anhydrous CHCl₃ were added using a graduated cylinder and cannula to transfer the solvent to the 1000 mL 2-neck flask containing the perfluoroheptanoyl chloride. The mixture was stirred at room temperature to dissolve the acid chloride. An addition funnel was fitted to this flask, and the YMerOH-triol-pyridine solution in CHCl₃ was transferred to the addition funnel using a cannula. N₂ flow through the reactor was adjusted to a slow and steady rate. YMerOH-triol-pyridine solution was added continuously drop-wise to the acid chloride solution at room temperature over a period of ~4 h. Stirring was maintained at a sufficient speed to achieve good mixing of reagents. After completing the addition of the YMer-triol-pyridine solution, the addition funnel was replaced with an air condenser, and the 2-neck flask was immersed in an oil bath placed on a heater fitted with a thermocouple unit. The temperature was raised to 40 °C, and the reaction was continued at this temperature under N₂ for 24 h.

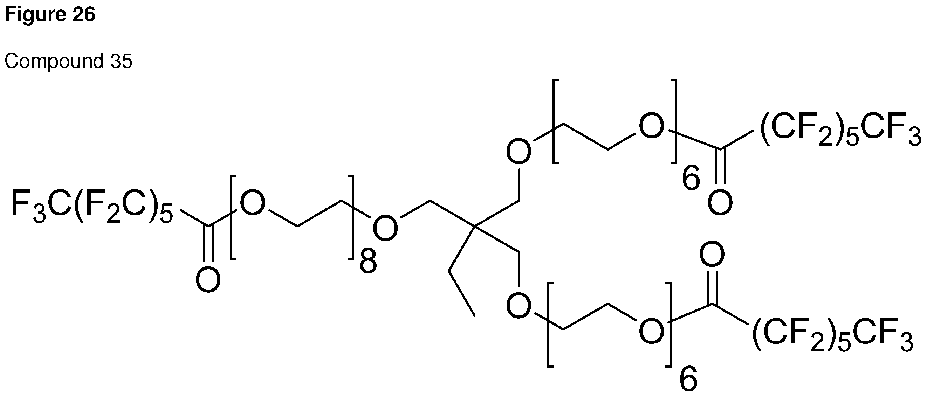

The resulting product was purified in a similar manner to compound 7 described above. The purification involved rotary evaporation of CHCl₃, addition of THF, and separation of the pyridine salts by filtration. The product was then precipated in isopropanol (IPA)/Hexanes, washed as described above for compound 7, and dried at 75 °C and 4 mbar. Final drying was also done under vacuum at 60 °C for 24 h to yield an oil (78% yield). The purified product was characterized by GPC (molecular weight based on polystyrene standards), elemental analysis for fluorine, ¹⁹F NMR, ¹H NMR, FTIR, and TGA. Appearance: light yellow, viscous oil. Weight average molecular weight (using polystyrene standards) = 2321g/mol. Polydispersity: 1.06. Elemental Analysis: F: 35.13% (theory: 36.11%). ¹⁹F NMR (CDCl₃, 400 MHz, ppm): δ -81.30 (m, CF₃), -118.90 (m, CF₂), -122.27 (m, CF₂), -123.07 (m, CF₂), -126.62 (m, CF₂). ¹H NMR (CDCl₃, 400 MHz, ppm): δ 0.83 (t, CH₃CH₂), 1.44 (q, CH₂CH₃), 3.34 (m, CH₂O), 3.41 (m, CH₂'s), 3.74 (m, CH₂), 4.30 (m, CH₂COO-). FTIR, neat (cm⁻¹): 2870 (CH₂), 1780 (O-C=O, ester), 1235, 1202, 1141, 1103 (CF₃, CF₂). The theoretical chemical structure of compound 35 is shown in Figure 26.

### Compound 36

Compound 36 was prepared according to a procedure similar to that used for the preparation of compound 34.

Glassware used for the synthesis was dried in an oven at 110 °C overnight. To a 2-neck 1000 mL oven dried round bottom flask equipped with a stir bar was added 50 g (65 mmol) of XMer-tetraol (MW = 771 Da). The flask with the tetraol was degassed overnight at 60 °C with gentle stirring and then purged with dry N₂ the following day. Heating was turned off. A 1000 mL graduated cylinder was charged with 400 mL anhydrous CHCl₃, sealed with rubber septa, and purged with dry N₂. CHCl₃ was transferred to the 2-neck flask via a cannula, and the tetraol was stirred vigorously to dissolve in the solvent. Excess anhydrous pyridine (51.30 g, 649 mmol) was added to the XMer-tetraol solution using a plastic syringe, and the resulting mixture was stirred to dissolve all materials. Another oven dried 2-neck 1000 mL flask was charged with 111.63 g (292 mmol) of perfluoroheptanoyl chloride, sealed with rubber septa, and degassed for 5 minutes, and then purged with N₂. 300 mL of anhydrous CHCl₃ were added using a graduated cylinder and cannula to transfer the solvent to the 1000 mL 2-neck flask containing perfluoroheptanoyl chloride. The resulting mixture was stirred at room temperature to dissolve the acid chloride. An addition funnel was attached to this flask, and the XMer-tetraol-pyridine solution in CHCl₃ was transferred into the addition funnel via a cannula. N₂ flow through the reactor was adjusted to a slow and steady rate. XMer-tetraol-pyridine solution was added continuously drop-wise to the acid chloride solution at room temperature over a period of ~4 h. Stirring was maintained at a sufficient speed to achieve good mixing of reagents. After completing addition of the XMer-tetraol-pyridine solution, the addition funnel was replaced with an air condenser, and the 2-neck flask was immersed in an oil bath placed on a heater fitted with a thermocouple unit. The temperature was raised to 40 °C, and the reaction continued at this temperature under N₂ for 24 h.

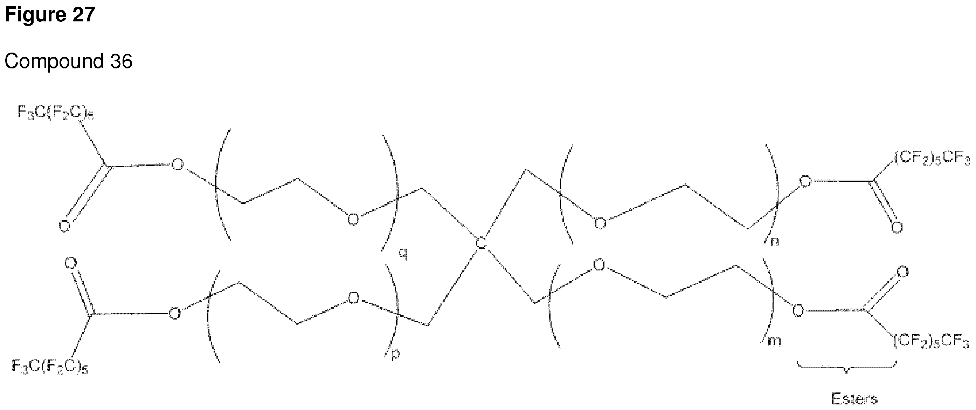

The resulting product was purified in a similar manner to compound 7 described above, where the CHCl₃ was removed by rotary evaporation, addition of THF, and the separation of pyridine salts by filtration after adding THF. The product was then precipitated in isopropanol (IPA)/hexanes, washed as described for compound 7, and dried at 75 °C and 4 mbar. Final drying was also done under vacuum at 60 °C for 24 h to yield an oil (81% yield). The purified product was characterized by GPC (molecular weight based on polystyrene standards), elemental analysis for fluorine, ¹⁹F NMR, ¹H NMR, FTIR, and TGA. Appearance: light yellow, viscous oil. Weight average molecular weight (using polystyrene standards) = 2410 g/mol. Polydispersity: 1.04. Elemental Analysis: F: 44.07% (theory: 45.85%). ¹⁹F NMR (CDCl₃, 400 MHz, ppm): δ -81.37 (m, CF₃), -118.89 (m, CF₂), -122.27 (m, CF₂), - 123.06 (m, CF₂), -26.64 (m, CF₂). ¹H NMR (CDCl₃, 400 MHz, ppm): δ 3.36 (m, CH₂'s), 3.75 (m, CH₂O), 4.39 (m, CH₂O), 4.49 (m, CH₂COO-). FTIR, neat (cm⁻¹): 2870 (CH₂), 1780 (O-C=O, ester), 1235, 1202, 1141, 1103 (CF₃, CF₂). TGA: N₂, at ca. 10% (w/w) loss = 327 °C. The theoretical chemical structure of compound 36 is shown in Figure 27.

### Compounds 37 and 38

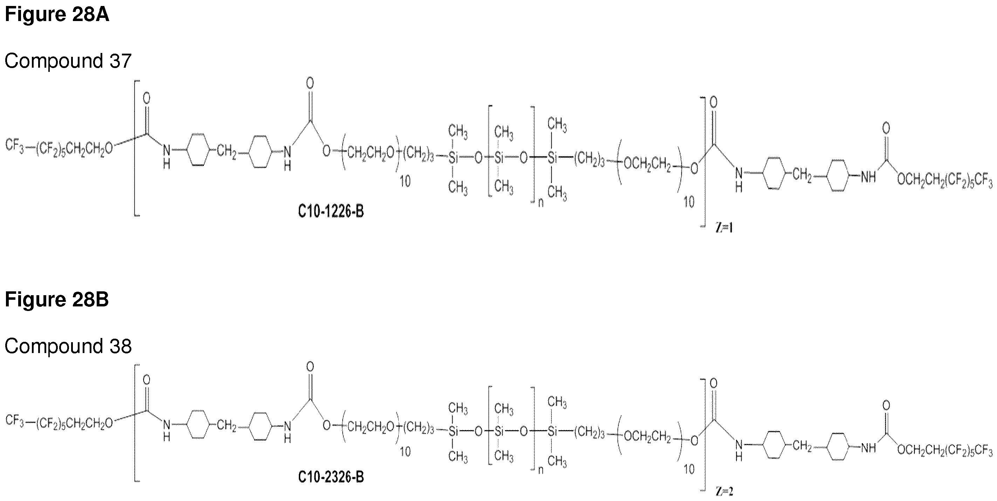

Glassware used for the synthesis was dried in an oven at 110 °C overnight. 25.04 g (9.7 mmol) of pegylated polydimethylsiloxane diol (C10-diol) was weighed out in a 250 mL 2-neck flask, heated to 50 °C, and degassed overnight with stirring. The diol was then purged with N₂ and dissolved in 25 mL of anhydrous THF. To the resulting mixture was added 36 mg of bismuth carboxylate catalyst in THF (concentration of 0.02 g/mL) followed by a solution of HMDI diisocyanate in THF (5.34 g, 20.4 mmol) which was previously degassed for 30 minutes followed by N₂ purge. The addition was performed using a syringe. The reaction vessel was fitted with an air condenser, and the mixture was allowed to react at 60 °C with stirring for 4 h. While the pre-polymer reaction was under way, capstone C6-FOH (fluoroalcohol) (8.82 g, 24.2 mmol) was degassed for 15 minutes in a separate flask and then purged with N₂. The fluoroalcohol was dissolved in THF, and a further 24 mg of bismuth carboxylate catalyst in THF was added to it. This mixture was then added to the prepolymer reaction vessel via syringe. After the addition was completed, the reaction mixture was allowed to react overnight at 45 °C under a N₂ atmosphere. After the reaction, the THF solvent was removed on a rotary evaporator, and the crude residue was dissolved in chloroform. The bismuth catalyst residues were extracted using EDTA solution (pH ~9). The solution containing EDTA was washed with DI water in a separatory funnel, and the organic layer was concentrated in a rotary evaporator to give the product as an amber viscous liquid. Final drying was done under vacuum at 60 °C for 24 h to yield a viscous oil (74% yield). The purified product was characterized by GPC (molecular weight based on polystyrene standards), elemental analysis for fluorine, and TGA. Appearance: amber, viscous oil. Weight average molecular weight (using polystyrene standards) = 13583 g/mol. Polydispersity: 1.73. Elemental Analysis: F: 12.20% (theory: 12.88%). TGA: N₂, at ca. <5% (w/w) loss = 231 °C. The theoretical chemical structure of compound 37 is shown in Figure 28A.

### Compound 38

Compound 38 is synthesized following a procedure similar to that which was used in the preparation of compound 37. Thus, 25.01 g (9.7 mmol) of C10-diol was reacted with 4.07 g (15.5 mmol) of HMDI in THF in the presence of Bismuth Carboxylate catalyst to form the prepolymer. The prepolymer was then endcapped with 5.29 g (14.5 mmol) Capstone C6-FOH (fluoroalcohol) to yield the product as a viscous oil (59% yield). The purified product was characterized by GPC (molecular weight based on polystyrene standards), elemental analysis for fluorine, and TGA. Appearance: amber, viscous oil. Weight average molecular weight (using polystyrene standards) = 19279 g/mol. Polydispersity: 1.79. Elemental Analysis: F: 6.51% (theory: 7.39%). TGA: N₂, at ca. <5% (w/w) loss = 244 °C. The theoretical chemical structure of compound 38 is shown in Figure 28B.

### Compound 39

Compound 39 was synthesized by a 2-step convergent method according to scheme 2. Briefly, the polyisocyanate desmodur 4470 (11.45 g, 11 mmol) was reacted with capstone C6-FOH (7.65 g, 21 mmol) in anhydrous THF in the presence of Bismuth Carboxylate catalyst at 25 °C for 10 minutes. After the dropwise addition of the fluoroalcohol to the polyisocyanate, stirring was continued for 4 h at 40 °C. These steps lead to the formation of a partially fluorinated intermediate that is then coupled with the PLN8K diol (40 g, 5 mmol) at 70 °C over a period of 14 h to provide compound 39. Because the reactions are moisture sensitive, they are carried out under an inert atmosphere (N₂) and anhydrous conditions. The temperature profile is also maintained carefully, especially during the partial fluorination, to avoid unwanted side reactions. Over the course of the reaction, the reaction mixture becomes very viscous, and continuous stirring must be maintained to prevent localized heating.

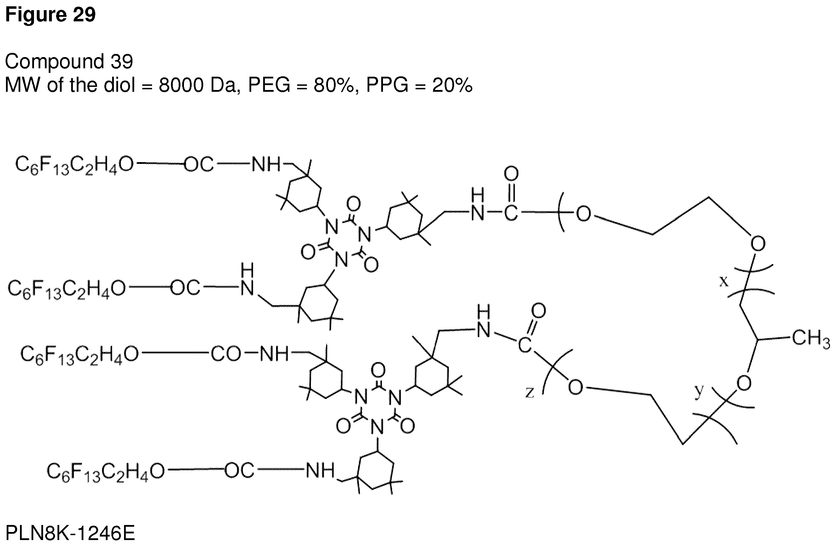

After the reaction, the THF solvent was evaporated on a rotary evaporator to yield the crude product. The product was purified by dissolving in chloroform and adding the EDTA solution (pH ~9.0). The mixture was then transferred to a separatory funnel, and the catalyst residues were separated with the aqueous layer. The organic layer was concentrated, and the product was dissolved in isopropanol and precipated in hexanes to yield a white chunky solid which was dried under vacuum (66% yield). The purified product was characterized by GPC (molecular weight based on polystyrene standards), elemental analysis for fluorine, and TGA. Appearance: white chunky solid. Weight average molecular weight (using polystyrene standards) = 31806 g/mol. Polydispersity: 1.32. Elemental Analysis: F: 3.6% (theory: 8.0%). TGA: N₂, at ca. <5% (w/w) loss = 295 °C. The theoretical chemical structure of compound 39 is shown in Figure 29.

### Compound 40

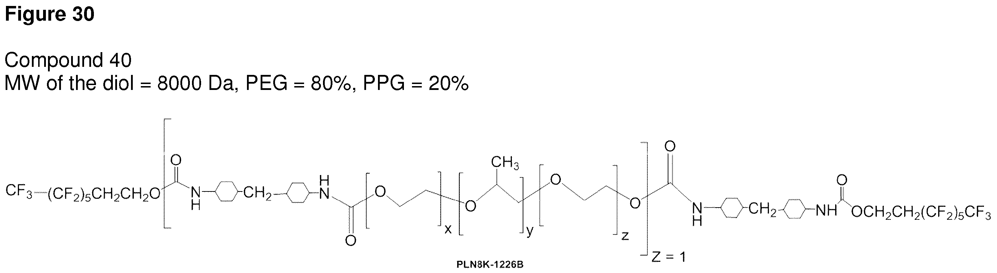

Compound 40 was synthesized following a procedure similar to that which was used in the preparation of compound 37. Thus, 50.0 g (5.7 mmol) of PLN8K diol were reacted with 4.5 g (17.1 mmol) of HMDI in THF in the presence of bismuth carboxylate catalyst to form the prepolymer. The prepolymer was then endcapped with 7.28 g (20 mmol) capstone C6-FOH (fluoroalcohol) to yield the crude product. The EDTA washes to eliminate the catalyst residues were similar. Final purification was performed by dissolving in isopropanol and precipitating with hexanes to yield a white solid (86% yield). The purified product was characterized by GPC (molecular weight based on polystyrene standards), elemental analysis for fluorine, and TGA. Appearance: while solid. Weight average molecular weight (using polystyrene standards) = 9253 g/mol. Polydispersity: 1.28. Elemental Analysis: F: 3.14% (theory: 4.94%). TGA: N₂, at ca. <5% (w/w) loss = 303 °C. The theoretical chemical structure of compound 40 is shown in Figure 30.

### Compound 41

Compound 41 was synthesized following a procedure similar to that which was used in the preparation of compound 27. The theoretical chemical structure of compound 41 is shown in Figure 21A, with the exception that the middle triblock copolymer is formed from a C10-diol.

The purified product was characterized by GPC (molecular weight based on polystyrene standards), elemental analysis for fluorine, and TGA. Appearance: colorless viscous liquid. Weight average molecular weight (using polystyrene standards) = 5858 g/mol. Polydispersity: 1.21. Elemental Analysis: F: 18.39% (theory: 15.08%). TGA: N₂, at ca. <10% (w/w) loss = 310 °C.

### Example 2. Preparation of a Vascular Graft Bearing a Modified Surface

### Electrospinning

A vascular graft of the invention may be electrospun from a liquid mixture for coating a structural support in the form of a tube. In one example, the liquid mixture is prepared by mixing a solution of, e.g., dimethylacetamide (DMAc), tetrahydrofuran (THF), isopropyl alcohol (IPA), and an oligofluorinated additive (e.g., a compound of any one of formulae (I)-(XVII) or any one of compounds 1-41; targeted dry weight percentage of an oligofluorinated additive in the final coating is from 0.05% (w/w) to 15% (w/w)) with a solution of a suitable base polymer (e.g., Bionate^{™}, Elast-Eon^{™}, Pellethane^{®} 2363-80AE elastomer, BIOSPAN^{™}, or ELASTHANE^{™}). Electrospinning creates a fine stream or jet of liquid that upon proper evaporation of a solvent or liquid to solid transition state yields a non-woven structure. The fine stream of liquid is produced by pulling a small amount of polymer solution through space by using electrical forces, followed by a hardening procedure, e.g., cooling, chemical hardening (e.g., polymerization), solvent evaporation. The produced fibers are collected on a suitably located precipitation device and subsequently stripped therefrom. The sedimentation device is typically shaped in a desired geometry of the final product, which may be tubular in the case of vascular grafts

### Wet spinning

A vascular graft of the invention may be formed by wet spinning of an admixture of an additive (e.g., a compound of any one of formulae (I)-(XVII) or any one of compounds 1-41; targeted dry weight percentage of an oligofluorinated additive in the final coating is from 0.05% (w/w) to 15% (w/w)) with a base polymer (e.g., Bionate^{™}, Elast-Eon^{™}, Pellethane^{®} 2363-80AE elastomer, BIOSPAN^{™}, or ELASTHANE^{™}) extruded with a syringe pump. The resulting fibers are collected using a fiber collecting system.

### Example 3. BCA Assay for Protein Deposition

A reference vascular graft of the invention is prepared (e.g., as described in Example 2) and incubated in protein solutions of varying concentrations. Examples of proteins that may be used in this assay include fibrinogen, albumin, and lysozyme. The concentrations of proteins typically fall within the range from 1 mg/mL to 5 mg/mL. The incubation time is typically from about 2 h to about 3 h. After the incubation is complete, the film samples are rinsed with PBS. Protein adhesion onto the samples may then be quantified using methods known in the art, e.g., a bicinchoninic acid (BCA) assay kit (Pierce, Rockford, IL). Briefly, the samples are incubated in a solution of sodium dodecyl sulfate (SDS) solution for up to about 24 h (with sonication if needed) in order to remove the proteins from the surfaces. A working solution is then prepared using the kit that facilitates the reduction of copper ions and interaction with the BCA. The sample protein solutions are added to the working solution, and the proteins from the sample solutions form a purple complex that is quantifiable using a spectrophotometer at a wavelength of 570 nm. A calibration curve of known protein concentrations is prepared in a similar manner for quantification. Based on the sample surface area, the results are typically reported as µg/cm².

### Example 4. Assay for Deposition in Blood

A reference vascular graft surface of the invention is prepared (e.g., as described in Example 2) and exposed to fresh bovine blood with a heparin concentration of 0.75 to 1 U/mL in a circulating blood loop. To quantify thrombosis on the sample tubes, the autologous platelets are radiolabeled with ¹¹¹In oxyquinoline (oxine) prior to the commencement of the experiment. Samples are placed inside a segment of circuit tubing, or they can be attached as a segment, and both ends of the circuit are placed in the blood reservoir. The blood is then circulated at a flow rate of 200 mL/min, and the temperature kept at 37 °C. The blood circulation is maintained for 60 to 120 minutes. When the experiment is terminated, the tubing section containing the sample is detached from the test circuit and rinsed gently with saline. The sample is removed from the tubing and further analyzed for visual and radioactive count.

## Claims

1. A vascular graft comprising a tubular body having an inner surface and a long axis wherein the inner surface comprises an oligofluorinated additive admixed with a base polymer comprising polyethylene terephthalate, wherein the tubular body has a first end and a second end adapted for an attachment to an artery or a vein,
wherein the inner surface comprises from 0.05% (w/w) to 15% (w/w) of the oligofluorinated additive selected from the structure of any one of formulas (I)-(XVII):
Formula (I):
F_{T}-[B-A]ₙ-B-F_{T} (I)
where
(i) A includes hydrogenated polybutadiene, poly((2,2-dimethyl)-1,3-propylene carbonate), polybutadiene, poly(diethylene glycol)adipate, poly(hexamethylene carbonate), poly(ethylene-co-butylene), (neopentyl glycol-ortho phthalic anhydride) polyester, (diethylene glycol-ortho phthalic anhydride) polyester, (1,6-hexanediol-ortho phthalic anhydride) polyester, or bisphenol A ethoxylate;
(ii) B is a segment including a urethane; and
(iii) F_{T} is a polyfluoroorgano group, and
(iv) n is an integer from 1 to 10; or
Formula (II):
F_{T}-[B-A]ₙ-B-F_{T} (II)
where
(i) B includes a urethane;
(ii) A includes polypropylene oxide, polyethylene oxide, or polytetramethylene oxide;
(iii) F_{T} is a polyfluoroorgano group; and
(iv) n is an integer from 1 to 10; or 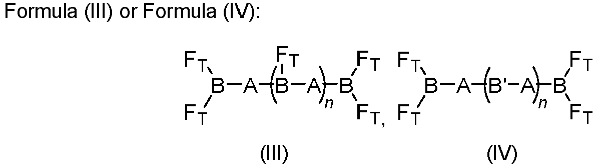
where
(i) A is an oligomeric segment containing an ether linkage, an ester linkage, a carbonate linkage, or a polyalkylene and having a theoretical molecular weight of from 500 to 3,500 Da;
(ii) B is a segment including a isocyanurate trimer or biuret trimer; B', when present, is a segment including a urethane;
(iii) each F_{T} is a polyfluoroorgano group; and
(iv) n is an integer between 0 to 10; or
Formula (V):
F_{T}-[B-A]ₙ-B-F_{T} (V)
where
(i) A is an oligomeric segment including polypropylene oxide, polyethylene oxide, or polytetramethylene oxide and having a theoretical molecular weight of from 500 to 3,000 Da;
(ii) B is a segment formed from a diisocyanate;
(iii) F_{T} is a polyfluoroorgano group; and
(iv) n is an integer from 1 to 10; or 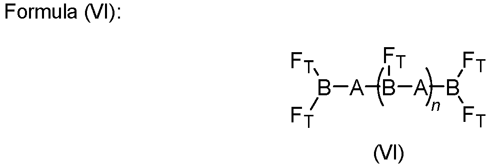
where
(i) A is an oligomeric segment including polyethylene oxide, polypropylene oxide, polytetramethylene oxide, or a mixture thereof, and having a theoretical molecular weight of from 500 to 3,000 Da;
(ii) B is a segment including an isocyanurate trimer or biuret trimer;
(iii) F_{T} is a polyfluoroorgano group; and
(iv) n is an integer from 0 to 10; or
Formula (VII):
F_{T}-[B-A]ₙ-B-F_{T} (VII)
where
(i) A is a polycarbonate polyol having a theoretical molecular weight of from 500 to 3,000 Da;
(ii) B is a segment formed from a diisocyanate;
(iii) F_{T} is a polyfluoroorgano group; and
(iv) n is an integer from 1 to 10; or 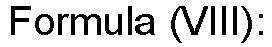 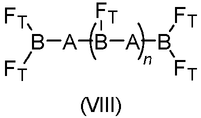
where
(i) A is an oligomeric segment including a polycarbonate polyol having a theoretical molecular weight of from 500 to 3,000 Da;
(ii) B is a segment including an isocyanurate trimer or biuret trimer;
(iii) F_{T} is a polyfluoroorgano group; and
(iv) n is an integer from 0 to 10; or 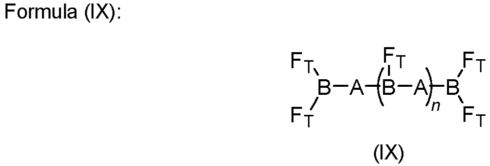
where
(i) A includes a first block segment selected from polypropylene oxide, polyethylene oxide, polytetramethylene oxide, or a mixture thereof, and a second block segment including a polysiloxane or polydimethylsiloxane, where A has a theoretical molecular weight of from 1,000 to 5,000 Da;
(ii) B is a segment including an isocyanurate trimer or biuret trimer;
(iii) F_{T} is a polyfluoroorgano group; and
(iv) n is an integer from 0 to 10; or
Formula (X):
F_{T}-[B-A]ₙ-B-F_{T} (X)
where
(i) A is a segment selected from the group consisting of hydrogenated polybutadiene, polybutadiene, hydrogenated polyisoprene, polysiloxane-polyethylene glycol block copolymer, and polystyrene and has a theoretical molecular weight of from 750 to 3,500 Da;
(ii) B is a segment formed from a diisocyanate;
(iii) F_{T} is a polyfluoroorgano group; and
(iv) n is an integer from 1 to 10; or 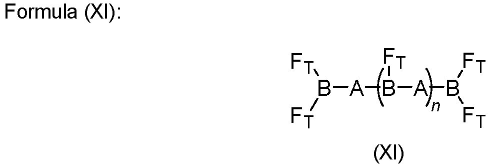
where
(i) A is hydrogenated polybutadiene, polybutadiene, hydrogenated polyisoprene, or polystyrene and has a theoretical molecular weight of from 750 to 3,500 Da;
(ii) B is a segment including an isocyanurate trimer or biuret trimer;
(iii) F_{T} is a polyfluoroorgano group; and
(iv) n is an integer from 0 to 10; or 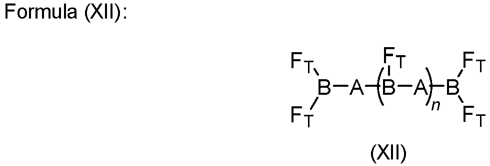
where
(i) A is a polyester having a theoretical molecular weight of from 500 to 3,500 Da;
(ii) B is a segment including an isocyanurate trimer or biuret trimer;
(iii) F_{T} is a polyfluoroorgano group; and
(iv) n is an integer from 0 to 10; or
Formula (XIII):
F_{T}-A-F_{T} (XIII)
where F_{T} is a polyfluoroorgano group and A is an oligomeric segment.
(13) 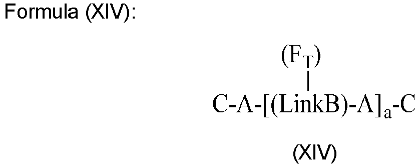 where
(i) F_{T} is a polyfluoroorgano group covalently attached to LinkB;
(ii) C is a chain terminating group;
(iii) A is an oligomeric segment;
(iv) LinkB is a coupling segment; and
(v) a is an integer greater than 0; or 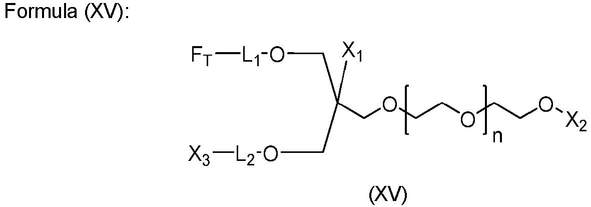
where
(i) each F_{T} is a polyfluoroorgano group;
(ii) X₁ is H, CH₃, or CH₂CH₃;
(iii) each of X₂ and X₃ is independently H, CH₃, CH₂CH₃, or F_{T};
(iv) each of L₁ and L₂ is independently a bond, an oligomeric linker, or a linker with two terminal carbonyls; and
(v) n is an integer from 5 to 50; or 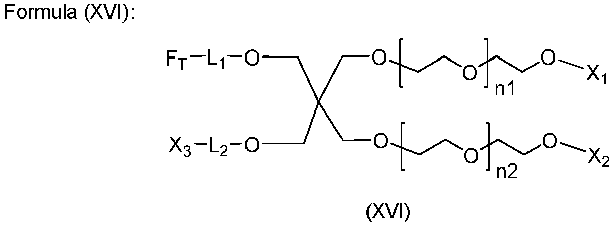
where
(i) each F_{T} is a polyfluoroorgano;
(ii) each of X₁, X₂, and X₃ is independently H, CH₃, CH₂CH₃, or F_{T};
(iii) each of L₁ and L₂ is independently a bond, an oligomeric linker, a linker with two terminal carbonyls, or is formed from a diisocyanate; and
(iv) each of n1 and n2 is independently an integer from 5 to 50; or
Formula (XVII):
G-Am-[B-A]n-B-G (XVII)
where
(i) each A includes hydrogenated polybutadiene, poly ((2,2-dimethyl)-1,3-propylene carbonate), polybutadiene, poly (diethylene glycol)adipate, poly (hexamethylene carbonate), poly (ethylene-co-butylene), (diethylene glycol-ortho phthalic anhydride) polyester, (1,6-hexanediol-ortho phthalic anhydride) polyester, (neopentyl glycol-ortho phthalic anhydride) polyester, a polysiloxane, or bisphenol A ethoxylate;
(ii) each B is independently a bond, an oligomeric linker, or a linker with two terminal carbonyls;
(iii) each G is H or a polyfluoroograno, provided that at least one G is a polyfluoroorgano;
(iv) n is an integer from 1 to 10; and
(v) m is 0 or 1.

2. A vascular graft comprising a tubular body having an inner surface and a long axis wherein the inner surface comprises an oligofluorinated additive admixed with a base polymer comprising polytetrafluoroethylene, wherein the tubular body has a first end and a second end adapted for an attachment to an artery or a vein,
wherein the inner surface comprises from 0.05% (w/w) to 15% (w/w) of the oligofluorinated additive selected from the structure of any one of formulas (I)-(XVII):
Formula (I):
F_{T}-[B-A]ₙ-B-F_{T} (I)
where
(i) A includes hydrogenated polybutadiene, poly((2,2-dimethyl)-1,3-propylene carbonate), polybutadiene, poly(diethylene glycol)adipate, poly(hexamethylene carbonate), poly(ethylene-co-butylene), (neopentyl glycol-ortho phthalic anhydride) polyester, (diethylene glycol-ortho phthalic anhydride) polyester, (1,6-hexanediol-ortho phthalic anhydride) polyester, or bisphenol A ethoxylate;
(ii) B is a segment including a urethane; and
(iii) F_{T} is a polyfluoroorgano group, and
(iv) n is an integer from 1 to 10; or
Formula (II):
F_{T}-[B-A]ₙ-B-F_{T} (II)
where
(v) B includes a urethane;
(vi) A includes polypropylene oxide, polyethylene oxide, or polytetramethylene oxide;
(vii) F_{T} is a polyfluoroorgano group; and
(viii) n is an integer from 1 to 10; or 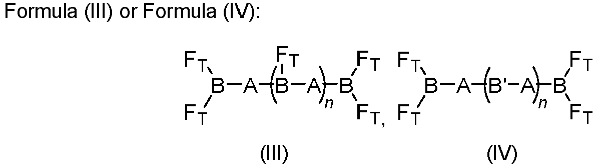
where
(v) A is an oligomeric segment containing an ether linkage, an ester linkage, a carbonate linkage, or a polyalkylene and having a theoretical molecular weight of from 500 to 3,500 Da;
(vi) B is a segment including a isocyanurate trimer or biuret trimer; B', when present, is a segment including a urethane;
(vii) each F_{T} is a polyfluoroorgano group; and
(viii) n is an integer between 0 to 10; or
Formula (V):
F_{T}-[B-A]ₙ-B-F_{T} (V)
where
(v) A is an oligomeric segment including polypropylene oxide, polyethylene oxide, or polytetramethylene oxide and having a theoretical molecular weight of from 500 to 3,000 Da;
(vi) B is a segment formed from a diisocyanate;
(vii) F_{T} is a polyfluoroorgano group; and
(viii) n is an integer from 1 to 10; or 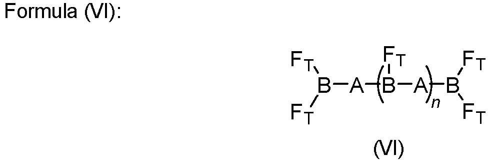
where
(v) A is an oligomeric segment including polyethylene oxide, polypropylene oxide, polytetramethylene oxide, or a mixture thereof, and having a theoretical molecular weight of from 500 to 3,000 Da;
(vi) B is a segment including an isocyanurate trimer or biuret trimer;
(vii) F_{T} is a polyfluoroorgano group; and
(viii) n is an integer from 0 to 10; or
Formula (VII):
F_{T}-[B-A]ₙ-B-F_{T} (VII)
where
(v) A is a polycarbonate polyol having a theoretical molecular weight of from 500 to 3,000 Da;
(vi) B is a segment formed from a diisocyanate;
(vii) F_{T} is a polyfluoroorgano group; and
(viii) n is an integer from 1 to 10; or 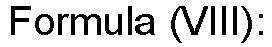 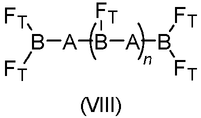
where
(v) A is an oligomeric segment including a polycarbonate polyol having a theoretical molecular weight of from 500 to 3,000 Da;
(vi) B is a segment including an isocyanurate trimer or biuret trimer;
(vii) F_{T} is a polyfluoroorgano group; and
(viii) n is an integer from 0 to 10; or 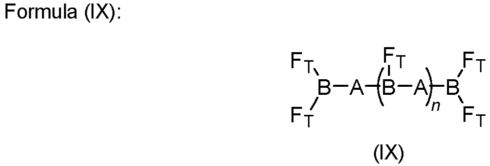
where
(v) A includes a first block segment selected from polypropylene oxide, polyethylene oxide, polytetramethylene oxide, or a mixture thereof, and a second block segment including a polysiloxane or polydimethylsiloxane, where A has a theoretical molecular weight of from 1,000 to 5,000 Da;
(vi) B is a segment including an isocyanurate trimer or biuret trimer;
(vii) F_{T} is a polyfluoroorgano group; and
(viii) n is an integer from 0 to 10; or
Formula (X):
F_{T}-[B-A]ₙ-B-F_{T} (X)
where
(v) A is a segment selected from the group consisting of hydrogenated polybutadiene, polybutadiene, hydrogenated polyisoprene, polysiloxane-polyethylene glycol block copolymer, and polystyrene and has a theoretical molecular weight of from 750 to 3,500 Da;
(vi) B is a segment formed from a diisocyanate;
(vii) F_{T} is a polyfluoroorgano group; and
(viii) n is an integer from 1 to 10; or 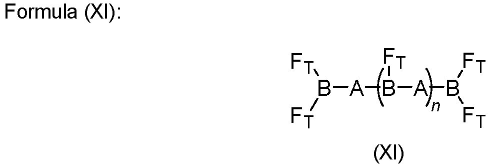
where
(v) A is hydrogenated polybutadiene, polybutadiene, hydrogenated polyisoprene, or polystyrene and has a theoretical molecular weight of from 750 to 3,500 Da;
(vi) B is a segment including an isocyanurate trimer or biuret trimer;
(vii) F_{T} is a polyfluoroorgano group; and
(viii) n is an integer from 0 to 10; or 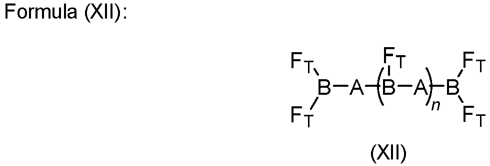
where
(v) A is a polyester having a theoretical molecular weight of from 500 to 3,500 Da;
(vi) B is a segment including an isocyanurate trimer or biuret trimer;
(vii) F_{T} is a polyfluoroorgano group; and
(viii) n is an integer from 0 to 10; or
Formula (XIII):
F_{T}-A-F_{T} (XIII)
where F_{T} is a polyfluoroorgano group and A is an oligomeric segment.
(13) 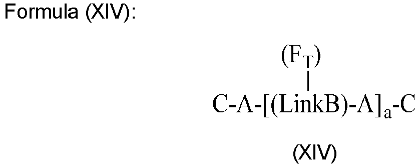 where
(i) F_{T} is a polyfluoroorgano group covalently attached to LinkB;
(ii) C is a chain terminating group;
(vi) A is an oligomeric segment;
(vii) LinkB is a coupling segment; and
(viii) a is an integer greater than 0; or 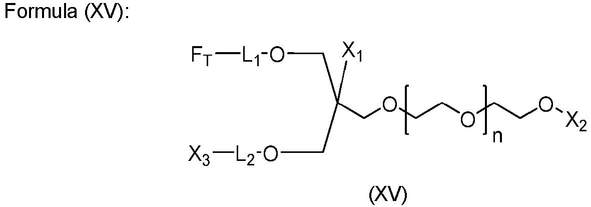
where
(i) each F_{T} is a polyfluoroorgano group;
(ii) X₁ is H, CH₃, or CH₂CH₃;
(iii) each of X₂ and X₃ is independently H, CH₃, CH₂CH₃, or F_{T};
(iv) each of L₁ and L₂ is independently a bond, an oligomeric linker, or a linker with two terminal carbonyls; and
(v) n is an integer from 5 to 50; or 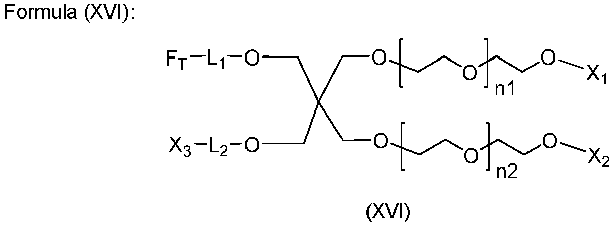
where
(i) each F_{T} is a polyfluoroorgano;
(ii) each of X₁, X₂, and X₃ is independently H, CH₃, CH₂CH₃, or F_{T};
(iii) each of L₁ and L₂ is independently a bond, an oligomeric linker, a linker with two terminal carbonyls, or is formed from a diisocyanate; and
(iv) each of n1 and n2 is independently an integer from 5 to 50; or
Formula (XVII):
G - Am - [B - A]ₙ - B - G (XVII)
where
(i) each A includes hydrogenated polybutadiene, poly ((2,2-dimethyl)-1,3-propylene carbonate), polybutadiene, poly (diethylene glycol)adipate, poly (hexamethylene carbonate), poly (ethylene-co-butylene), (diethylene glycol-ortho phthalic anhydride) polyester, (1,6-hexanediol-ortho phthalic anhydride) polyester, (neopentyl glycol-ortho phthalic anhydride) polyester, a polysiloxane, or bisphenol A ethoxylate;
(ii) each B is independently a bond, an oligomeric linker, or a linker with two terminal carbonyls;
(iii) each G is H or a polyfluoroograno, provided that at least one G is a polyfluoroorgano;
(iv) n is an integer from 1 to 10; and
(v) m is 0 or 1.

3. A vascular graft comprising a tubular body having an inner surface and a long axis wherein the inner surface comprises an oligofluorinated additive admixed with a base polymer comprising a polyurethane, wherein the tubular body has a first end and a second end adapted for an attachment to an artery or a vein,
wherein the inner surface comprises from 0.05% (w/w) to 15% (w/w) of the oligofluorinated additive selected from the structure of any one of formulas (I)-(XVII):
Formula (I):
F_{T}-[B-A]ₙ-B-F_{T} (I)
where
(i) A includes hydrogenated polybutadiene, poly((2,2-dimethyl)-1,3-propylene carbonate), polybutadiene, poly(diethylene glycol)adipate, poly(hexamethylene carbonate), poly(ethylene-co-butylene), (neopentyl glycol-ortho phthalic anhydride) polyester, (diethylene glycol-ortho phthalic anhydride) polyester, (1,6-hexanediol-ortho phthalic anhydride) polyester, or bisphenol A ethoxylate;
(ii) B is a segment including a urethane; and
(iii) F_{T} is a polyfluoroorgano group, and
(iv) n is an integer from 1 to 10; or
Formula (II):
F_{T}-[B-A]ₙ-B-F_{T} (II)
where
(ix) B includes a urethane;
(x) A includes polypropylene oxide, polyethylene oxide, or polytetramethylene oxide;
(xi) F_{T} is a polyfluoroorgano group; and
(xii) n is an integer from 1 to 10; or 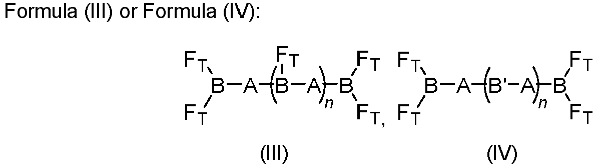
where
(ix) A is an oligomeric segment containing an ether linkage, an ester linkage, a carbonate linkage, or a polyalkylene and having a theoretical molecular weight of from 500 to 3,500 Da;
(x) B is a segment including a isocyanurate trimer or biuret trimer; B', when present, is a segment including a urethane;
(xi) each F_{T} is a polyfluoroorgano group; and
(xii) n is an integer between 0 to 10; or
Formula (V):
F_{T}-[B-A]ₙ-B-F_{T} (V)
where
(ix) A is an oligomeric segment including polypropylene oxide, polyethylene oxide, or polytetramethylene oxide and having a theoretical molecular weight of from 500 to 3,000 Da;
(x) B is a segment formed from a diisocyanate;
(xi) F_{T} is a polyfluoroorgano group; and
(xii) n is an integer from 1 to 10; or 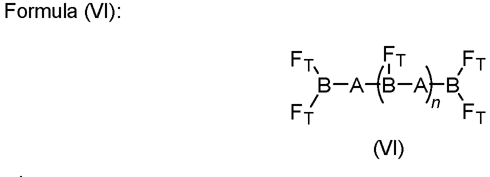
where
(ix) A is an oligomeric segment including polyethylene oxide, polypropylene oxide, polytetramethylene oxide, or a mixture thereof, and having a theoretical molecular weight of from 500 to 3,000 Da;
(x) B is a segment including an isocyanurate trimer or biuret trimer;
(xi) F_{T} is a polyfluoroorgano group; and
(xii) n is an integer from 0 to 10; or
Formula (VII):
F_{T}-[B-A]ₙ-B-F_{T} (VII)
where
(ix) A is a polycarbonate polyol having a theoretical molecular weight of from 500 to 3,000 Da;
(x) B is a segment formed from a diisocyanate;
(xi) F_{T} is a polyfluoroorgano group; and
(xii) n is an integer from 1 to 10; or 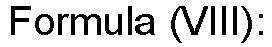 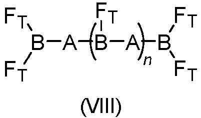
where
(ix) A is an oligomeric segment including a polycarbonate polyol having a theoretical molecular weight of from 500 to 3,000 Da;
(x) B is a segment including an isocyanurate trimer or biuret trimer;
(xi) F_{T} is a polyfluoroorgano group; and
(xii) n is an integer from 0 to 10; or 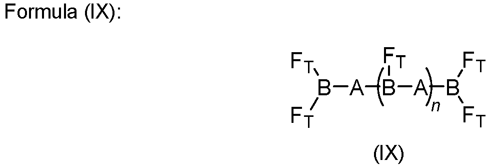
where
(ix) A includes a first block segment selected from polypropylene oxide, polyethylene oxide, polytetramethylene oxide, or a mixture thereof, and a second block segment including a polysiloxane or polydimethylsiloxane, where A has a theoretical molecular weight of from 1,000 to 5,000 Da;
(x) B is a segment including an isocyanurate trimer or biuret trimer;
(xi) F_{T} is a polyfluoroorgano group; and (xii) n is an integer from 0 to 10; or
Formula (X):
F_{T}-[B-A]ₙ-B-F_{T} (X)
where
(ix) A is a segment selected from the group consisting of hydrogenated polybutadiene, polybutadiene, hydrogenated polyisoprene, polysiloxane-polyethylene glycol block copolymer, and polystyrene and has a theoretical molecular weight of from 750 to 3,500 Da;
(x) B is a segment formed from a diisocyanate;
(xi) F_{T} is a polyfluoroorgano group; and
(xii) n is an integer from 1 to 10; or 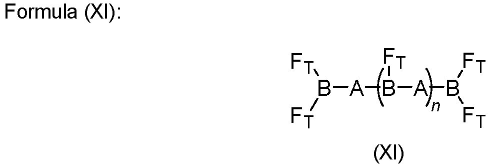
where
(ix) A is hydrogenated polybutadiene, polybutadiene, hydrogenated polyisoprene, or polystyrene and has a theoretical molecular weight of from 750 to 3,500 Da;
(x) B is a segment including an isocyanurate trimer or biuret trimer;
(xi) F_{T} is a polyfluoroorgano group; and
(xii) n is an integer from 0 to 10; or 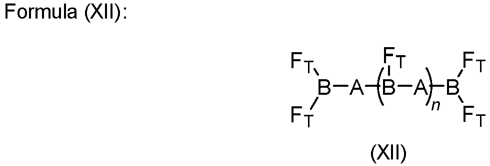
where
(ix) A is a polyester having a theoretical molecular weight of from 500 to 3,500 Da;
(x) B is a segment including an isocyanurate trimer or biuret trimer;
(xi) F_{T} is a polyfluoroorgano group; and
(xii) n is an integer from 0 to 10; or
Formula (XIII):
F_{T}-A-F_{T} (XIII)
where F_{T} is a polyfluoroorgano group and A is an oligomeric segment.
(13) 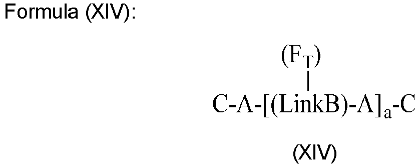 where
(i) F_{T} is a polyfluoroorgano group covalently attached to LinkB;
(ii) C is a chain terminating group;
(ix) A is an oligomeric segment;
(x) LinkB is a coupling segment; and
(xi) a is an integer greater than 0; or 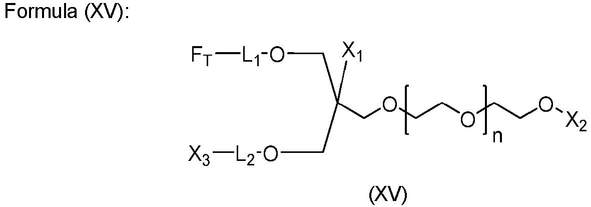
where
(i) each F_{T} is a polyfluoroorgano group;
(ii) X₁ is H, CH₃, or CH₂CH₃;
(iii) each of X₂ and X₃ is independently H, CH₃, CH₂CH₃, or F_{T};
(iv) each of L₁ and L₂ is independently a bond, an oligomeric linker, or a linker with two terminal carbonyls; and
(v) n is an integer from 5 to 50; or 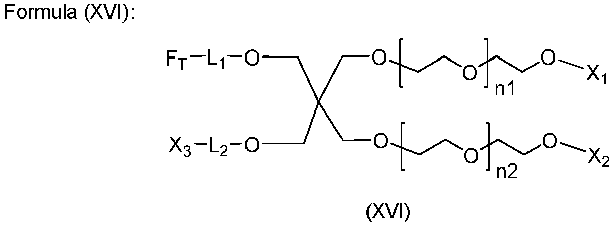
where
(i) each F_{T} is a polyfluoroorgano;
(ii) each of X₁, X₂, and X₃ is independently H, CH₃, CH₂CH₃, or F_{T};
(iii) each of L₁ and L₂ is independently a bond, an oligomeric linker, a linker with two terminal carbonyls, or is formed from a diisocyanate; and
(iv) each of n1 and n2 is independently an integer from 5 to 50; or
Formula (XVII):
G - Am - [B - A]ₙ - B - G (XVII)
where
(i) each A includes hydrogenated polybutadiene, poly ((2,2-dimethyl)-1,3-propylene carbonate), polybutadiene, poly (diethylene glycol)adipate, poly (hexamethylene carbonate), poly (ethylene-co-butylene), (diethylene glycol-ortho phthalic anhydride) polyester, (1,6-hexanediol-ortho phthalic anhydride) polyester, (neopentyl glycol-ortho phthalic anhydride) polyester, a polysiloxane, or bisphenol A ethoxylate;
(ii) each B is independently a bond, an oligomeric linker, or a linker with two terminal carbonyls;
(iii) each G is H or a polyfluoroograno, provided that at least one G is a polyfluoroorgano;
(iv) n is an integer from 1 to 10; and
(v) m is 0 or 1.

4. The vascular graft of claim 3, wherein the polyurethane is selected from polycarbonate urethanes, polyurethane with a poly(dimethylsiloxane) soft segment, a polytetramethylene glycol-based polyurethane elastomer, segmented polyurethanes, and polyetherurethanes.

5. The vascular graft of claims 1 to 4, wherein the oligofluorinated additive is selected from 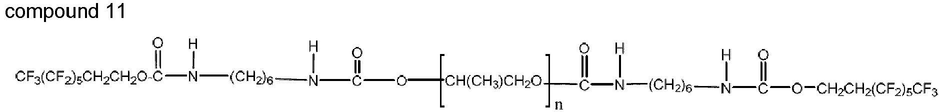 wherein the molecular weight is 800 to 1200 g/mol; 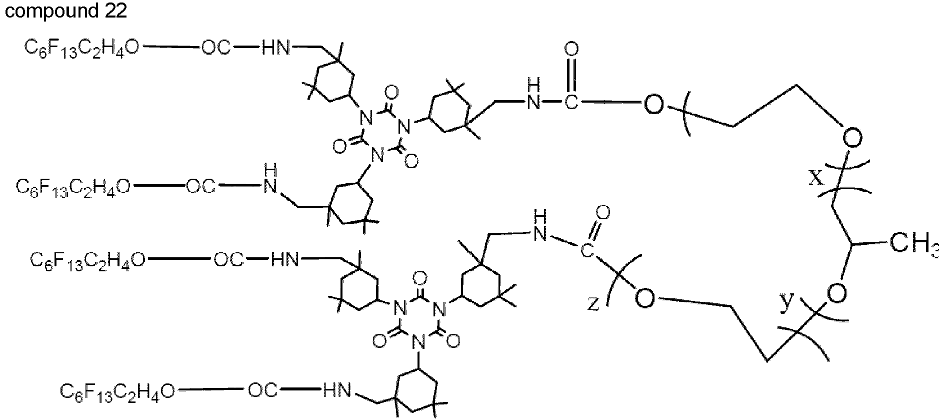 wherein
x, y, and z are integers; or 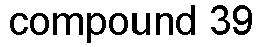 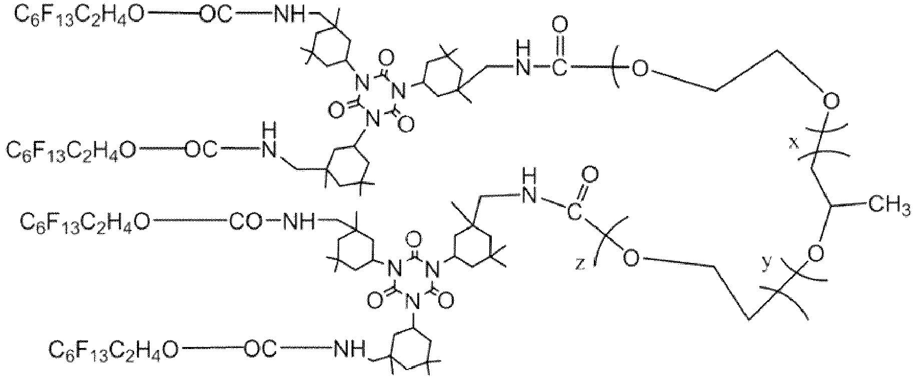 wherein Mw of the diol is 8000 g/mol, PEG = 80% and PPG = 20%.

6. The vascular graft of any one of claims 1-5, wherein the first end and the second end adapted for an attachment to an artery or a vein comprise anchoring barbs or a material suitable for sewing onto a portion of an artery or of a vein.

## Patentansprüche

1. Gefäßtransplantat, umfassend einen röhrenförmigen Körper mit einer Innenfläche und einer Längsachse, wobei die Innenfläche ein oligofluoriertes Additiv umfasst, das mit einem Polyethylenterephthalat umfassenden Basispolymer vermischt ist, wobei der röhrenförmige Körper ein erstes Ende und ein zweites Ende aufweist, die für eine Befestigung an einer Arterie oder einer Vene ausgelegt sind,
wobei die innere Oberfläche 0,05 Gew.-% bis 15 Gew.-% des oligofluorierten Additivs umfasst, das aus der Struktur einer der Formeln (I)-(XVII) ausgewählt ist:
Formel (I):
F_{T}-[B-A]ₙ-B-F_{T} (I)
wobei
(i) A hydriertes Polybutadien, Poly((2,2-dimethyl)-1,3-propylencarbonat), Polybutadien, Poly(diethylenglykol)adipat, Poly(hexamethylencarbonat), Poly(ethylen-co-butylen), (Neopentylglykol-ortho-Phtalsäureanhydrid)-Polyester, (Diethylenglykol-ortho-Phtalsäureanhydrid)-Polyester, (1,6-Hexandiol-ortho-Phtalsäureanhydrid)-Polyester oder Bisphenol-A-ethoxylat einschließt;
(ii) B ein Segment ist, das ein Urethan einschließt, und
(iii) F_{T} eine Polyfluororganogruppe ist und
(iv) n für eine ganze Zahl von 1 bis 10 steht; oder
Formel (II):
F_{T}-[B-A]ₙ-B-F_{T} (II)
wobei
(i) B ein Urethan einschließt;
(ii) A Polypropylenoxid, Polyethylenoxid oder Polytetramethylenoxid einschließt;
(iii) F_{T} eine Polyfluororganogruppe ist und
(iv) n für eine ganze Zahl von 1 bis 10 steht; oder 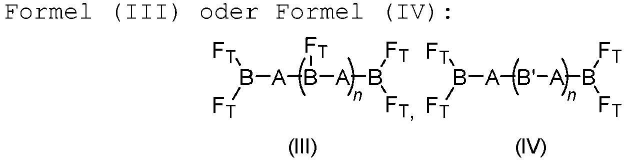
wobei
(i) A ein oligomeres Segment mit einer Etherverknüpfung, einer Esterverknüpfung, einer Carbonatverknüpfung oder einem Polyalkylen ist und ein theoretisches Molekulargewicht von 500 bis 3.500 Da aufweist;
(ii) B ein Segment ist, das ein Isocyanattrimer oder Biurettrimer einschließt; B', wenn vorhanden, ein Segment ist, das ein Urethan einschließt;
(iii) F_{T} jeweils eine Polyfluororganogruppe ist und
(iv) n eine ganze Zahl zwischen 0 bis 10 ist; oder
Formel (V):
F_{T}-[B-A]ₙ-B-F_{T} (V)
wobei
(i) A ein oligomeres Segment ist, das Polypropylenoxid, Polyethylenoxid oder Polytetramethylenoxid einschließt und ein theoretisches Molekulargewicht von 500 bis 3.000 Da aufweist;
(ii) B ein Segment ist, das aus einem Diisocyanat gebildet ist;
(iii) F_{T} eine Polyfluororganogruppe ist und
(iv) n für eine ganze Zahl von 1 bis 10 steht; oder 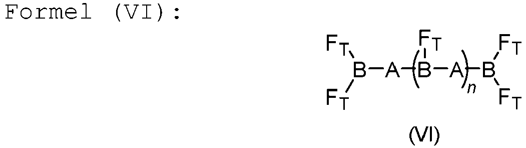
wobei
(i) A ein oligomeres Segment ist, das Polyethylenoxid, Polypropylenoxid, Polytetramethylenoxid oder eine Mischung davon einschließt und ein theoretisches Molekulargewicht von 500 bis 3.000 Da aufweist;
(ii) B ein Segment ist, das aus einem Isocyanattrimer oder Biurettrimer gebildet ist;
(iii) F_{T} eine Polyfluororganogruppe ist und
(iv) n für eine ganze Zahl von 0 bis 10 steht; oder
Formel (VII):
F_{T}-[B-A]ₙ-B-F_{T} (VII)
wobei
(i) A ein Polycarbonatpolyol mit einem theoretischen Molekulargewicht von 500 bis 3.000 Da ist;
(ii) B ein Segment ist, das aus einem Diisocyanat gebildet ist;
(iii) F_{T} eine Polyfluororganogruppe ist und
(iv) n für eine ganze Zahl von 1 bis 10 steht; oder 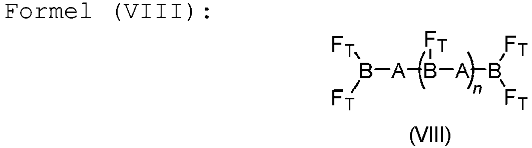
wobei
(i) A ein oligomeres Segment ist, das ein Polycarbonatpolyol mit einem theoretischen Molekulargewicht von 500 bis 3.000 Da einschließt;
(ii) B ein Segment ist, das aus einem Isocyanattrimer oder Biurettrimer gebildet ist;
(iii) F_{T} eine Polyfluororganogruppe ist und
(iv) n für eine ganze Zahl von 0 bis 10 steht; oder 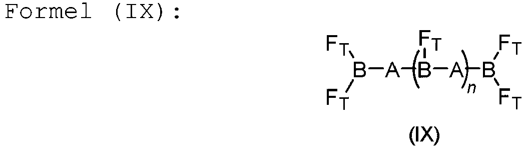
wobei
(i) A ein erstes Blocksegment, das aus Polypropylenoxid, Polyethylenoxid, Polytetramethylenoxid oder einer Mischung davon ausgewählt ist, und ein zweites Blocksegment, das ein Polysiloxan oder Polydimethylsiloxan einschließt, einschließt, wobei A ein theoretisches Molekulargewicht von 1.000 bis 5.000 Da aufweist;
(ii) B ein Segment ist, das aus einem Isocyanattrimer oder Biurettrimer gebildet ist;
(iii) F_{T} eine Polyfluororganogruppe ist und
(iv) n für eine ganze Zahl von 0 bis 10 steht; oder
Formel (X):
F_{T}-[B-A]ₙ-B-F_{T} (X)
wobei
(i) A ein Segment ist, das aus der Gruppe bestehend aus hydriertem Polybutadien, Polybutadien, hydriertem Polyisopren, Polysiloxan-Polyethylenglykol-Blockcopolymer und Polystyrol ausgewählt ist, und ein theoretisches Molekulargewicht von 750 bis 3.500 Da aufweist;
(ii) B ein Segment ist, das aus einem Diisocyanat gebildet ist;
(iii) F_{T} eine Polyfluororganogruppe ist und
(iv) n für eine ganze Zahl von 1 bis 10 steht; oder 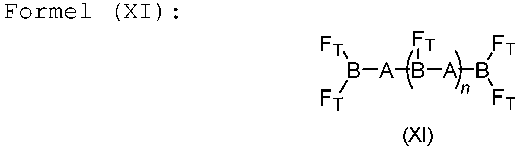
wobei
(i) A hydriertes Polybutadien, Polybutadien, hydriertes Polyisopren oder Polystyrol ist und ein theoretisches Molekulargewicht von 750 bis 3.500 Da aufweist;
(ii) B ein Segment ist, das aus einem Isocyanattrimer oder Biurettrimer gebildet ist;
(iii) F_{T} eine Polyfluororganogruppe ist und
(iv) n für eine ganze Zahl von 0 bis 10 steht; oder 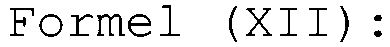 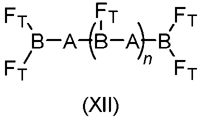
wobei
(i) A ein Polyester mit einem theoretischen Molekulargewicht von 500 bis 3.500 Da ist;
(ii) B ein Segment ist, das aus einem Isocyanattrimer oder Biurettrimer gebildet ist;
(iii) F_{T} eine Polyfluororganogruppe ist und
(iv) n für eine ganze Zahl von 0 bis 10 steht; oder
Formel (XIII):
F_{T}-A-F_{T} **(XIII)**
wobei F_{T} eine Polyfluororganogruppe und A ein oligomeres Segment ist.
(13) 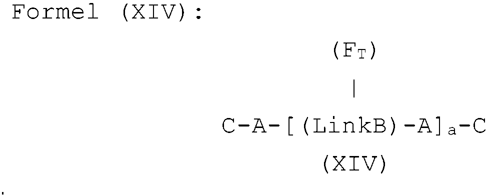 wobei
(i) F_{T} eine kovalent an LinkB gebundene Polyfluororganogruppe ist;
(ii) C eine Kettenabschlussgruppe ist;
(iii) A ein oligomeres Segment ist;
(iv) LinkB ein Kopplungssegment ist und
(v) a eine ganze Zahl größer als 0 ist; oder 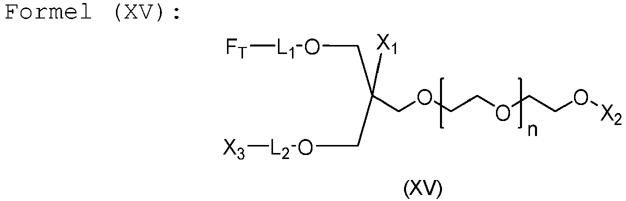
wobei
(i) F_{T} jeweils eine Polyfluororganogruppe ist;
(ii) X₁ H, CH₃ oder CH₂CH₃ ist;
(iii) X₂ und X₃ jeweils unabhängig H, CH₃, CH₂CH₃ oder F_{T} sind;
(iv) L₁ und L₂ jeweils unabhängig eine Bindung, ein oligomerer Linker oder ein Linker mit zwei endständigen Carbonylgruppen sind und
(v) n eine ganze Zahl von 5 bis 50 ist; oder 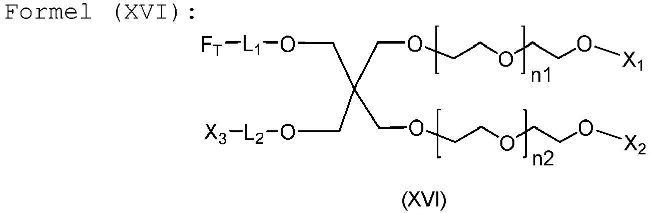
wobei
(i) F_{T} jeweils ein Polyfluororgano ist;
(ii) X₁, X₂, and X₃ jeweils unabhängig H, CH₃, CH₂CH₃ oder F_{T} sind;
(iii) L₁ und L₂ jeweils unabhängig eine Bindung, ein oligomerer Linker oder ein Linker mit zwei endständigen Carbonylgruppen sind oder aus einem Diisocyanat gebildet sind und
(iv) n1 und n2 jeweils unabhängig eine ganze Zahl von 5 bis 50 sind; oder
Formel (XVII):
G - Aₘ - [B - A]ₙ - B - G **(XVII)**
wobei
(i) A jeweils hydriertes Polybutadien, Poly((2,2-dimethyl)-1,3-propylencarbonat), Polybutadien, Poly(diethylenglykol)adipat, Poly(hexamethylencarbonat), Poly(ethylen-co-butylen), (Diethylenglykol-ortho-Phtalsäureanhydrid)-Polyester, (1,6-Hexandiol-ortho-Phtalsäureanhydrid)-Polyester, (Neopentylglykol-ortho-Phtalsäureanhydrid)-Polyester, ein Polysiloxan oder Bisphenol-A-ethoxylat einschließt;
(ii) B jeweils unabhängig eine Bindung, ein oligomerer Linker oder ein Linker mit zwei endständigen Carbonylgruppen ist;
(iii) G jeweils H oder ein Polyfluororgano ist, mit der Maßgabe, dass mindestens ein G ein Polyfluororgano ist;
(iv) n eine ganze Zahl von 1 bis 10 ist und
(v) m 0 oder 1 ist.

2. Gefäßtransplantat, umfassend einen röhrenförmigen Körper mit einer Innenfläche und einer Längsachse, wobei die Innenfläche ein oligofluoriertes Additiv umfasst, das mit einem Polytetrafluorethylen umfassenden Basispolymer vermischt ist, wobei der röhrenförmige Körper ein erstes Ende und ein zweites Ende aufweist, die für eine Befestigung an einer Arterie oder einer Vene ausgelegt sind,
wobei die innere Oberfläche 0,05 Gew.-% bis 15 Gew.-% des oligofluorierten Additivs umfasst, das aus der Struktur einer der Formeln (I)-(XVII) ausgewählt ist:
Formel (I):
F_{T}-[B-A]ₙ-B-F_{T} (I)
wobei
(i) A hydriertes Polybutadien, Poly((2,2-dimethyl)-1,3-propylencarbonat), Polybutadien, Poly(diethylenglykol)adipat, Poly(hexamethylencarbonat), Poly(ethylen-co-butylen), (Neopentylglykol-ortho-Phtalsäureanhydrid)-Polyester, (Diethylenglykol-ortho-Phtalsäureanhydrid)-Polyester, (1,6-Hexandiol-ortho-Phtalsäureanhydrid)-Polyester oder Bisphenol-A-ethoxylat einschließt;
(ii) B ein Segment ist, das ein Urethan einschließt, und
(iii) F_{T} eine Polyfluororganogruppe ist und
(iv) n für eine ganze Zahl von 1 bis 10 steht; oder
Formel (II):
F_{T}-[B-A]ₙ-B-F_{T} (II)
wobei
(v) B ein Urethan einschließt;
(vi) A Polypropylenoxid, Polyethylenoxid oder Polytetramethylenoxid einschließt;
(vii) F_{T} eine Polyfluororganogruppe ist und
(viii) n für eine ganze Zahl von 1 bis 10 steht; oder 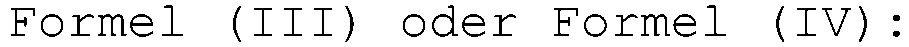 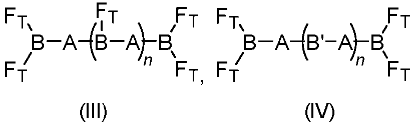
wobei
(v) A ein oligomeres Segment mit einer Etherverknüpfung, einer Esterverknüpfung, einer Carbonatverknüpfung oder einem Polyalkylen ist und ein theoretisches Molekulargewicht von 500 bis 3.500 Da aufweist;
(vi) B ein Segment ist, das ein Isocyanattrimer oder Biurettrimer einschließt; B', wenn vorhanden, ein Segment ist, das ein Urethan einschließt;
(vii) F_{T} jeweils eine Polyfluororganogruppe ist und
(viii) n eine ganze Zahl zwischen 0 bis 10 ist; oder
Formel (V):
F_{T}-[B-A]ₙ-B-F_{T} (V)
wobei
(v) A ein oligomeres Segment ist, das Polypropylenoxid, Polyethylenoxid oder Polytetramethylenoxid einschließt und ein theoretisches Molekulargewicht von 500 bis 3.000 Da aufweist;
(vi) B ein Segment ist, das aus einem Diisocyanat gebildet ist;
(vii) F_{T} eine Polyfluororganogruppe ist und
(viii) n für eine ganze Zahl von 1 bis 10 steht; oder 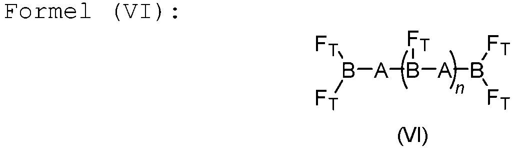
wobei
(v) A ein oligomeres Segment ist, das Polyethylenoxid, Polypropylenoxid, Polytetramethylenoxid oder eine Mischung davon einschließt und ein theoretisches Molekulargewicht von 500 bis 3.000 Da aufweist;
(vi) B ein Segment ist, das aus einem Isocyanattrimer oder Biurettrimer gebildet ist;
(vii) F_{T} eine Polyfluororganogruppe ist und
(viii) n für eine ganze Zahl von 0 bis 10 steht; oder
Formel (VII):
F_{T}-[B-A]ₙ-B-F_{T} (VII)
wobei
(v) A ein Polycarbonatpolyol mit einem theoretischen Molekulargewicht von 500 bis 3.000 Da ist;
(vi) B ein Segment ist, das aus einem Diisocyanat gebildet ist;
(vii) F_{T} eine Polyfluororganogruppe ist und
(viii) n für eine ganze Zahl von 1 bis 10 steht; oder 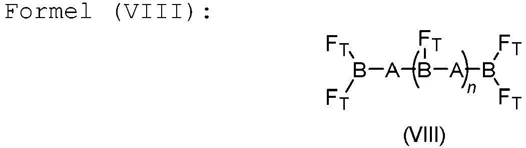
wobei
(v) A ein oligomeres Segment ist, das ein Polycarbonatpolyol mit einem theoretischen Molekulargewicht von 500 bis 3.000 Da einschließt;
(vi) B ein Segment ist, das aus einem Isocyanattrimer oder Biurettrimer gebildet ist;
(vii) F_{T} eine Polyfluororganogruppe ist und
(viii) n für eine ganze Zahl von 0 bis 10 steht; oder 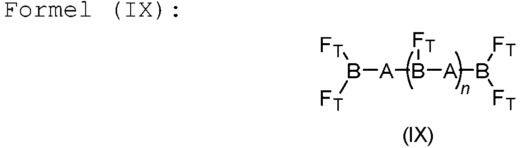
wobei
(v) A ein erstes Blocksegment, das aus Polypropylenoxid, Polyethylenoxid, Polytetramethylenoxid oder einer Mischung davon ausgewählt ist, und ein zweites Blocksegment, das ein Polysiloxan oder Polydimethylsiloxan einschließt, einschließt, wobei A ein theoretisches Molekulargewicht von 1.000 bis 5.000 Da aufweist;
(vi) B ein Segment ist, das aus einem Isocyanattrimer oder Biurettrimer gebildet ist;
(vii) F_{T} eine Polyfluororganogruppe ist und
(viii) n für eine ganze Zahl von 0 bis 10 steht; oder
Formel (X):
F_{T}-[B-A]ₙ-B-F_{T} (X)
wobei
(v) A ein Segment ist, das aus der Gruppe bestehend aus hydriertem Polybutadien, Polybutadien, hydriertem Polyisopren, Polysiloxan-Polyethylenglykol-Blockcopolymer und Polystyrol ausgewählt ist, und ein theoretisches Molekulargewicht von 750 bis 3.500 Da aufweist;
(vi) B ein Segment ist, das aus einem Diisocyanat gebildet ist;
(vii) F_{T} eine Polyfluororganogruppe ist und
(viii) n für eine ganze Zahl von 1 bis 10 steht; oder 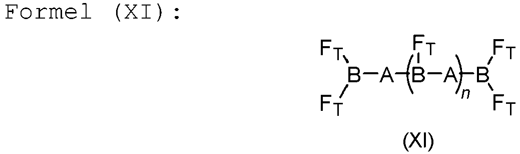
wobei
(v) A hydriertes Polybutadien, Polybutadien, hydriertes Polyisopren oder Polystyrol ist und ein theoretisches Molekulargewicht von 750 bis 3.500 Da aufweist;
(vi) B ein Segment ist, das aus einem Isocyanattrimer oder Biurettrimer gebildet ist;
(vii) F_{T} eine Polyfluororganogruppe ist und
(viii) n für eine ganze Zahl von 0 bis 10 steht; oder 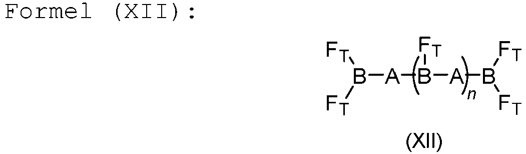
wobei
(v) A ein Polyester mit einem theoretischen Molekulargewicht von 500 bis 3.500 Da ist;
(vi) B ein Segment ist, das aus einem Isocyanattrimer oder Biurettrimer gebildet ist;
(vii) F_{T} eine Polyfluororganogruppe ist und
(viii) n für eine ganze Zahl von 0 bis 10 steht; oder
Formel (XIII):
F_{T}-A-F_{T} (XIII)
wobei F_{T} eine Polyfluororganogruppe und A ein oligomeres Segment ist.
(13) 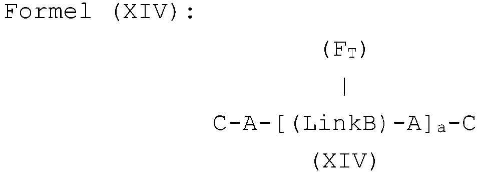 wobei
(i) F_{T} eine kovalent an LinkB gebundene Polyfluororganogruppe ist;
(ii) C eine Kettenabschlussgruppe ist;
(vi) A ein oligomeres Segment ist;
(vii) LinkB ein Kopplungssegment ist und
(viii) a eine ganze Zahl größer als 0 ist; oder 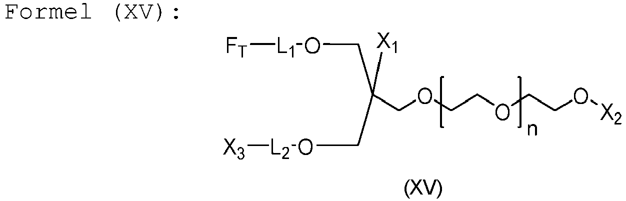
wobei
(i) F_{T} jeweils eine Polyfluororganogruppe ist;
(ii) X₁ H, CH₃ oder CH₂CH₃ ist;
(iii) X₂ und X₃ jeweils unabhängig H, CH₃, CH₂CH₃ oder F_{T} sind;
(iv) L₁ und L₂ jeweils unabhängig eine Bindung, ein oligomerer Linker oder ein Linker mit zwei endständigen Carbonylgruppen sind und
(v) n eine ganze Zahl von 5 bis 50 ist; oder 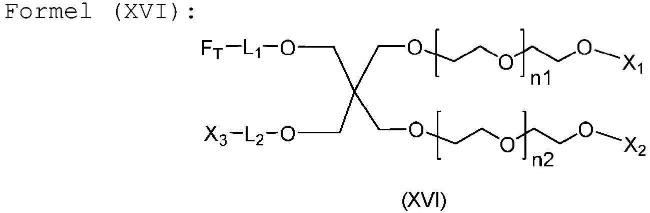
wobei
(i) F_{T} jeweils ein Polyfluororgano ist;
(ii) X₁, X₂, and X₃ jeweils unabhängig H, CH₃, CH₂CH₃ oder F_{T} sind;
(iii) L₁ und L₂ jeweils unabhängig eine Bindung, ein oligomerer Linker oder ein Linker mit zwei endständigen Carbonylgruppen sind oder aus einem Diisocyanat gebildet sind und
(iv) n1 und n2 jeweils unabhängig eine ganze Zahl von 5 bis 50 sind; oder
Formel (XVII):
G - Aₘ - [B - A]ₙ - B - G **(XVII)**
wobei
(i) A jeweils hydriertes Polybutadien, Poly((2,2-dimethyl)-1,3-propylencarbonat), Polybutadien, Poly(diethylenglykol)adipat, Poly(hexamethylencarbonat), Poly(ethylen-co-butylen), (Diethylenglykol-ortho-Phtalsäureanhydrid)-Polyester, (1,6-Hexandiol-ortho-Phtalsäureanhydrid)-Polyester, (Neopentylglykol-ortho-Phtalsäureanhydrid)-Polyester, ein Polysiloxan oder Bisphenol-A-ethoxylat einschließt;
(ii) B jeweils unabhängig eine Bindung, ein oligomerer Linker oder ein Linker mit zwei endständigen Carbonylgruppen ist;
(iii) G jeweils H oder ein Polyfluororgano ist, mit der Maßgabe, dass mindestens ein G ein Polyfluororgano ist;
(iv) n eine ganze Zahl von 1 bis 10 ist und
(v) m 0 oder 1 ist.

3. Gefäßtransplantat, umfassend einen röhrenförmigen Körper mit einer Innenfläche und einer Längsachse, wobei die Innenfläche ein oligofluoriertes Additiv umfasst, das mit einem ein Polyurethan umfassenden Basispolymer vermischt ist, wobei der röhrenförmige Körper ein erstes Ende und ein zweites Ende aufweist, die für eine Befestigung an einer Arterie oder einer Vene ausgelegt sind,
wobei die innere Oberfläche 0,05 Gew.-% bis 15 Gew.-% des oligofluorierten Additivs umfasst, das aus der Struktur einer der Formeln (I)-(XVII) ausgewählt ist:
Formel (I):
F_{T}-[B-A]ₙ-B-F_{T} (I)
wobei
(i) A hydriertes Polybutadien, Poly((2,2-dimethyl)-1,3-propylencarbonat), Polybutadien, Poly(diethylenglykol)adipat, Poly(hexamethylencarbonat), Poly(ethylen-co-butylen), (Neopentylglykol-ortho-Phtalsäureanhydrid)-Polyester, (Diethylenglykol-ortho-Phtalsäureanhydrid)-Polyester, (1,6-Hexandiol-ortho-Phtalsäureanhydrid)-Polyester oder Bisphenol-A-ethoxylat einschließt;
(ii) B ein Segment ist, das ein Urethan einschließt, und
(iii) F_{T} eine Polyfluororganogruppe ist und
(iv) n für eine ganze Zahl von 1 bis 10 steht; oder
Formel (II):
F_{T}-[B-A]ₙ-B-F_{T} (II)
wobei
(ix) B ein Urethan einschließt;
(x) A Polypropylenoxid, Polyethylenoxid oder Polytetramethylenoxid einschließt;
(xi) F_{T} eine Polyfluororganogruppe ist und
(xii) n eine ganze Zahl von 1 bis 10 ist; oder 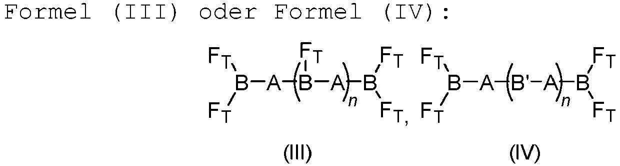
wobei
(ix) A ein oligomeres Segment mit einer Etherverknüpfung, einer Esterverknüpfung, einer Carbonatverknüpfung oder einem Polyalkylen ist und ein theoretisches Molekulargewicht von 500 bis 3.500 Da aufweist;
(x) B ein Segment ist, das ein Isocyanattrimer oder Biurettrimer einschließt; B', wenn vorhanden, ein Segment ist, das ein Urethan einschließt;
(xi) F_{T} jeweils eine Polyfluororganogruppe ist und
(xii) n eine ganze Zahl zwischen 0 bis 10 ist; oder
Formel (V):
F_{T}-[B-A]ₙ-B-F_{T} (V)
wobei
(ix) A ein oligomeres Segment ist, das Polypropylenoxid, Polyethylenoxid oder Polytetramethylenoxid einschließt und ein theoretisches Molekulargewicht von 500 bis 3.000 Da aufweist;
(x) B ein Segment ist, das aus einem Diisocyanat gebildet ist;
(xi) F_{T} eine Polyfluororganogruppe ist und
(xii) n eine ganze Zahl von 1 bis 10 ist; oder 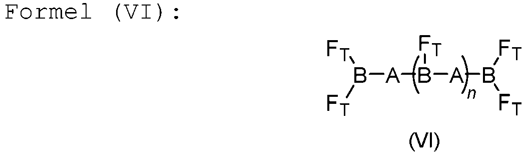
wobei
(ix) A ein oligomeres Segment ist, das Polyethylenoxid, Polypropylenoxid, Polytetramethylenoxid oder eine Mischung davon einschließt und ein theoretisches Molekulargewicht von 500 bis 3.000 Da aufweist;
(x) B ein Segment ist, das aus einem Isocyanattrimer oder Biurettrimer gebildet ist;
(xi) F_{T} eine Polyfluororganogruppe ist und
(xii) n eine ganze Zahl von 0 bis 10 ist; oder
Formel (VII):
F_{T}-[B-A]ₙ-B-F_{T} (VII)
wobei
(ix) A ein Polycarbonatpolyol mit einem theoretischen Molekulargewicht von 500 bis 3.000 Da ist;
(x) B ein Segment ist, das aus einem Diisocyanat gebildet ist;
(xi) F_{T} eine Polyfluororganogruppe ist und
(xii) n eine ganze Zahl von 1 bis 10 ist; oder 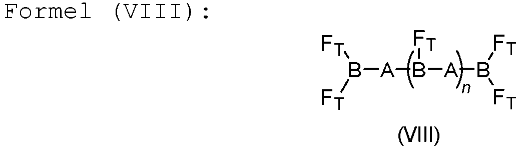
wobei
(ix) A ein oligomeres Segment ist, das ein Polycarbonatpolyol mit einem theoretischen Molekulargewicht von 500 bis 3.000 Da einschließt;
(x) B ein Segment ist, das aus einem Isocyanattrimer oder Biurettrimer gebildet ist;
(xi) F_{T} eine Polyfluororganogruppe ist und
(xii) n eine ganze Zahl von 0 bis 10 ist; oder 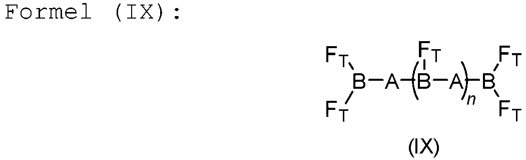
wobei
(ix) A ein erstes Blocksegment, das aus Polypropylenoxid, Polyethylenoxid, Polytetramethylenoxid oder einer Mischung davon ausgewählt ist, und ein zweites Blocksegment, das ein Polysiloxan oder Polydimethylsiloxan einschließt, einschließt, wobei A ein theoretisches Molekulargewicht von 1.000 bis 5.000 Da aufweist;
(x) B ein Segment ist, das aus einem Isocyanattrimer oder Biurettrimer gebildet ist;
(xi) F_{T} eine Polyfluororganogruppe ist und
(xii) n eine ganze Zahl von 0 bis 10 ist; oder
Formel (X):
F_{T}-[B-A]ₙ-B-F_{T} (X)
wobei
(ix) A ein Segment ist, das aus der Gruppe bestehend aus hydriertem Polybutadien, Polybutadien, hydriertem Polyisopren, Polysiloxan-Polyethylenglykol-Blockcopolymer und Polystyrol ausgewählt ist, und ein theoretisches Molekulargewicht von 750 bis 3.500 Da aufweist;
(x) B ein Segment ist, das aus einem Diisocyanat gebildet ist;
(xi) F_{T} eine Polyfluororganogruppe ist und
(xii) n eine ganze Zahl von 1 bis 10 ist; oder 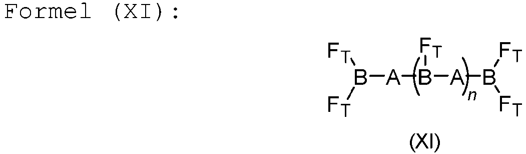
wobei
(ix) A hydriertes Polybutadien, Polybutadien, hydriertes Polyisopren oder Polystyrol ist und ein theoretisches Molekulargewicht von 750 bis 3.500 Da aufweist;
(x) B ein Segment ist, das aus einem Isocyanattrimer oder Biurettrimer gebildet ist;
(xi) F_{T} eine Polyfluororganogruppe ist und
(xii) n eine ganze Zahl von 0 bis 10 ist; oder 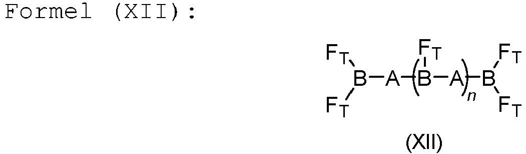
wobei
(ix) A ein Polyester mit einem theoretischen Molekulargewicht von 500 bis 3.500 Da ist;
(x) B ein Segment ist, das aus einem Isocyanattrimer oder Biurettrimer gebildet ist;
(xi) F_{T} eine Polyfluororganogruppe ist und
(xii) n eine ganze Zahl von 0 bis 10 ist; oder
Formel (XIII):
F_{T}-A-F_{T} **(XIII)**
wobei F_{T} eine Polyfluororganogruppe und A ein oligomeres Segment ist.
(13) 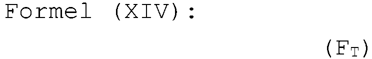 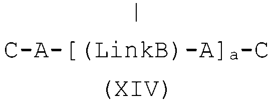 wobei
(i) F_{T} eine kovalent an LinkB gebundene Polyfluororganogruppe ist;
(ii) C eine Kettenabschlussgruppe ist;
(ix) A ein oligomeres Segment ist;
(x) LinkB ein Kopplungssegment ist und
(xi) a eine ganze Zahl größer als 0 ist; oder 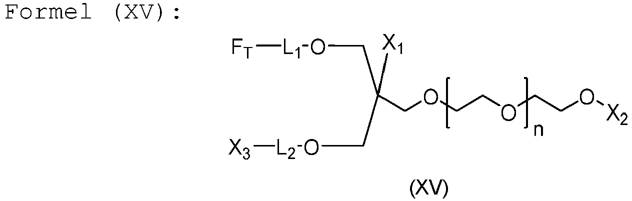
wobei
(i) F_{T} jeweils eine Polyfluororganogruppe ist;
(ii) X₁ H, CH₃ oder CH₂CH₃ ist;
(iii) X₂ und X₃ jeweils unabhängig H, CH₃, CH₂CH₃ oder F_{T} sind;
(iv) L₁ und L₂ jeweils unabhängig eine Bindung, ein oligomerer Linker oder ein Linker mit zwei endständigen Carbonylgruppen sind und
(v) n eine ganze Zahl von 5 bis 50 ist; oder 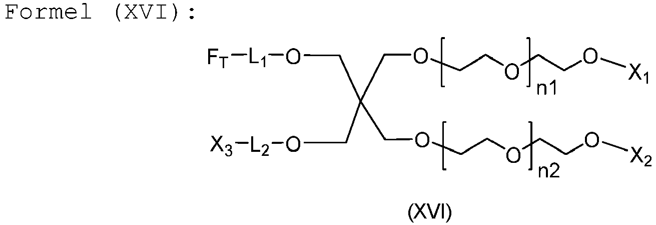
wobei
(i) F_{T} jeweils ein Polyfluororgano ist;
(ii) X₁, X₂, and X₃ jeweils unabhängig H, CH₃, CH₂CH₃ oder F_{T} sind;
(iii) L₁ und L₂ jeweils unabhängig eine Bindung, ein oligomerer Linker oder ein Linker mit zwei endständigen Carbonylgruppen sind oder aus einem Diisocyanat gebildet sind und
(iv) n1 und n2 jeweils unabhängig eine ganze Zahl von 5 bis 50 sind; oder
Formel (XVII):
G - Am - [B - A]ₙ - B - G (XVII)
wobei
(i) A jeweils hydriertes Polybutadien, Poly((2,2-dimethyl)-1,3-propylencarbonat), Polybutadien, Poly(diethylenglykol)adipat, Poly(hexamethylencarbonat), Poly(ethylen-co-butylen), (Diethylenglykol-ortho-Phtalsäureanhydrid)-Polyester, (1,6-Hexandiol-ortho-Phtalsäureanhydrid)-Polyester, (Neopentylglykol-ortho-Phtalsäureanhydrid)-Polyester, ein Polysiloxan oder Bisphenol-A-ethoxylat einschließt;
(ii) B jeweils unabhängig eine Bindung, ein oligomerer Linker oder ein Linker mit zwei endständigen Carbonylgruppen ist;
(iii) G jeweils H oder ein Polyfluororgano ist, mit der Maßgabe, dass mindestens ein G ein Polyfluororgano ist;
(iv) n eine ganze Zahl von 1 bis 10 ist und
(v) m 0 oder 1 ist.

4. Gefäßtransplantat nach Anspruch 3, wobei das Polyurethan aus Polycarbonaturethanen, Polyurethan mit einem weichen Poly(dimethylsiloxan)-Segment, einem Polyurethanelastomer auf Polytetramethylenglykol-Basis, segmentierten Polyurethanen und Polyetherurethanen ausgewählt ist.

5. Gefäßtransplantat nach einem der Ansprüche 1 bis 4, wobei das oligofluorierte Additiv ausgewählt ist aus 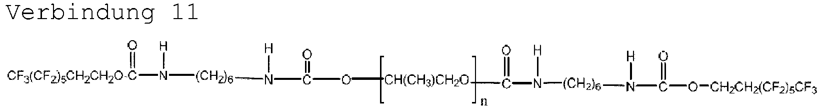 wobei das Molekulargewicht 800 bis 1200 g/mol beträgt; 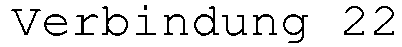 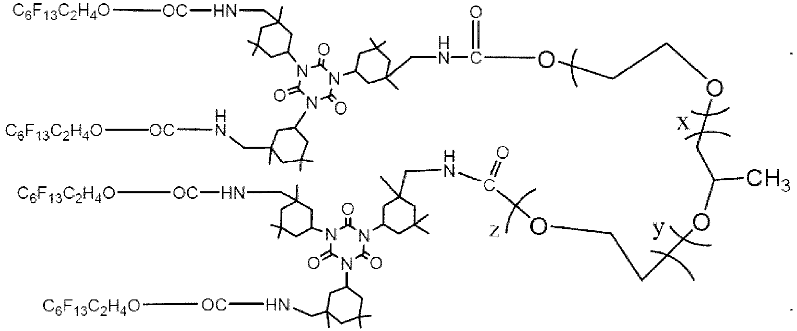 wobei
x, y und z ganze Zahlen sind; oder 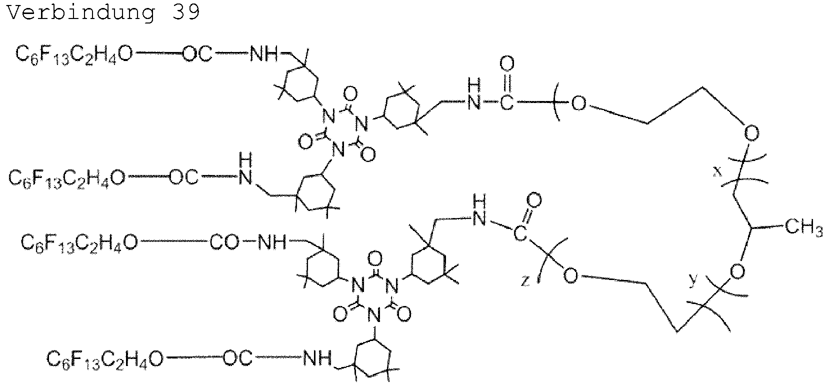 wobei das Mw des Diols 8000 g/mol beträgt, PEG = 80 % und PPG = 20 %.

6. Gefäßtransplantat nach einem der Ansprüche 1-5, wobei das erste Ende und das zweite Ende, die für eine Befestigung an einer Arterie oder einer Vene ausgelegt sind, Verankerungswiderhaken oder ein zum Annähen an einen Abschnitt einer Arterie oder einer Vene geeignetes Material umfassen.

## Revendications

1. Greffe vasculaire comprenant un corps tubulaire ayant une surface interne et un axe long, la surface interne comprenant un additif oligofluoré mélangé avec un polymère de base comprenant un poly(téréphtalate d'éthylène), le corps tubulaire ayant une première extrémité et une seconde extrémité adaptées pour une fixation à une artère ou une veine,
dans laquelle la surface intérieure comprend de 0,05 % (p/p) à 15 % (p/p) de l'additif oligofluoré choisi parmi la structure de l'une quelconque des formules (I)-(XVII) :
Formule (I) :
F_{T}-[B-A]ₙ-B-F_{T} (I)
dans laquelle
(i) A comprend un polybutadiène hydrogéné, poly(carbonate de (2,2-diméthyl)-1,3-propylène), polybutadiène, poly(adipate de diéthylèneglycol), poly(carbonate d'hexaméthylène), poly(éthylène-co-butylène), polyester (néopentylglycol-anhydride orthophtalique), polyester (diéthylèneglycol-anhydride orthophtalique), polyester (1,6-hexanediol-anhydride orthophtalique), ou éthoxylate de bisphénol A ;
(ii) B est un segment comprenant un uréthane ; et
(iii) F_{T} est un groupe polyfluoroorgano, et
(iv) n est un entier de 1 à 10 ; ou
Formule (II) :
F_{T}-[B-A]ₙ-B-F_{T} (II)
dans laquelle
(i) B comprend un uréthane ;
(ii) A comprend poly(oxyde de propylène), poly(oxyde d'éthylène), ou poly(oxyde de tétraméthylène) ;
(iii) F_{T} est un groupe polyfluoroorgano ; et
(iv) n est un entier de 1 à 10 ; ou 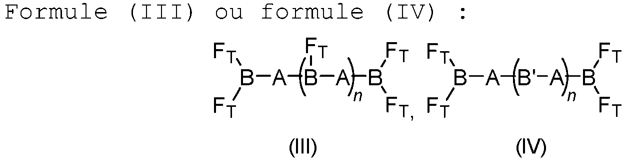
dans laquelle
(i) A est un segment oligomérique contenant une liaison éther, une liaison ester, une liaison carbonate, ou un polyalkylène et ayant un poids moléculaire théorique allant de 500 à 3 500 Da ;
(ii) B est un segment comprenant un trimère d'isocyanurate ou un trimère de biuret ; B', s'il est présent, est un segment comprenant un uréthane ;
(iii) chaque F_{T} est un groupe polyfluoroorgano ; et
(iv) n est un entier compris entre 0 et 10 ; ou
Formule (V) :
F_{T}-[B-A]ₙ-B-F_{T} (V)
dans laquelle
(i) A est un segment oligomérique comprenant un poly(oxyde de propylène), poly(oxyde d'éthylène), ou poly(oxyde de tétraméthylène) et ayant un poids moléculaire théorique allant de 500 à 3 000 Da ;
(ii) B est un segment formé à partir d'un diisocyanate ;
(iii) F_{T} est un groupe polyfluoroorgano ; et
(iv) n est un entier de 1 à 10 ; ou 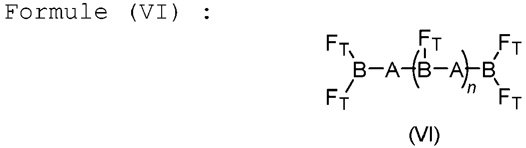
dans laquelle
(i) A est un segment oligomérique comprenant un poly(oxyde d'éthylène), poly(oxyde de propylène), poly(oxyde de tétraméthylène), ou un mélange correspondant, et ayant un poids moléculaire théorique allant de 500 à 3 000 Da ;
(ii) B est un segment comprenant un trimère d'isocyanurate ou un trimère de biuret ;
(iii) F_{T} est un groupe polyfluoroorgano ; et
(iv) n est un entier de 0 à 10 ; ou
Formule (VII) :
F_{T}-[B-A]ₙ-B-F_{T} (VII)
dans laquelle
(i) A est un polycarbonate polyol ayant un poids moléculaire théorique allant de 500 à 3 000 Da ;
(ii) B est un segment formé à partir d'un diisocyanate ;
(iii) F_{T} est un groupe polyfluoroorgano ; et
(iv) n est un entier de 1 à 10 ; ou 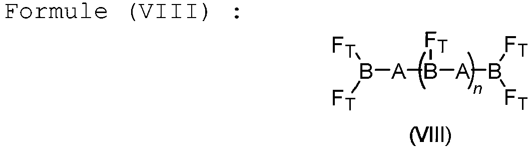
dans laquelle
(i) A est un segment oligomérique comprenant un polycarbonate polyol ayant un poids moléculaire théorique allant de 500 à 3 000 Da ;
(ii) B est un segment comprenant un trimère d'isocyanurate ou un trimère de biuret ;
(iii) F_{T} est un groupe polyfluoroorgano ; et
(iv) n est un entier de 0 à 10 ; ou 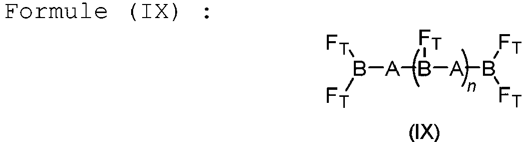
dans laquelle
(i) A comprend un premier segment de bloc choisi parmi poly(oxyde de propylène), poly(oxyde d'éthylène), poly(oxyde de tétraméthylène), ou un mélange correspondant, et un deuxième segment de bloc comprenant un polysiloxane ou polydiméthylsiloxane, où A possède un poids moléculaire théorique allant de 1 000 à 5 000 Da ;
(ii) B est un segment comprenant un trimère d'isocyanurate ou un trimère de biuret ;
(iii) F_{T} est un groupe polyfluoroorgano ; et
(iv) n est un entier de 0 à 10 ; ou
Formule (X) :
F_{T}-[B-A]ₙ-B-F_{T} **(X)**
dans laquelle
(i) A est un segment choisi dans le groupe constitué par polybutadiène hydrogéné, polybutadiène, polyisoprène hydrogéné, copolymère à blocs de polysiloxane-polyéthylène glycol, et polystyrène et possède un poids moléculaire théorique allant de 750 à 3 500 Da ;
(ii) B est un segment formé à partir d'un diisocyanate ;
(iii) F_{T} est un groupe polyfluoroorgano ; et
(iv) n est un entier de 1 à 10 ; ou 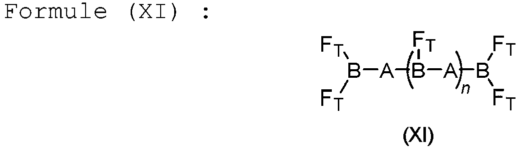
dans laquelle
(i) A est un polybutadiène hydrogéné, polybutadiène, polyisoprène hydrogéné, ou polystyrène et possède un poids moléculaire théorique allant de 750 à 3 500 Da ;
(ii) B est un segment comprenant un trimère d'isocyanurate ou un trimère de biuret ;
(iii) F_{T} est un groupe polyfluoroorgano ; et
(iv) n est un entier de 0 à 10 ; ou 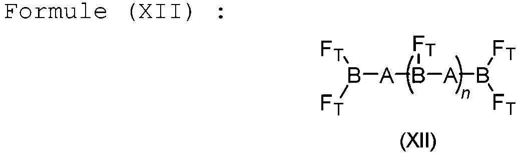
dans laquelle
(i) A est un polyester ayant un poids moléculaire théorique allant de 500 à 3 500 Da ;
(ii) B est un segment comprenant un trimère d'isocyanurate ou un trimère de biuret ;
(iii) F_{T} est un groupe polyfluoroorgano ; et
(iv) n est un entier de 0 à 10 ; ou
Formule (XIII) :
F_{T}-A-F_{T} (XIII)
où F_{T} est un groupe polyfluoroorgano et A est un segment oligomérique.
(13) Formule (XIV) : dans laquelle
(i) F_{T} est un groupe polyfluoroorgano fixé de manière covalente à LinkB ;
(ii) C est un groupe de terminaison de chaîne ;
(iii) A est un segment oligomérique ;
(iv) LinkB est un segment de couplage ; et
(v) a est un entier supérieur à 0 ; ou 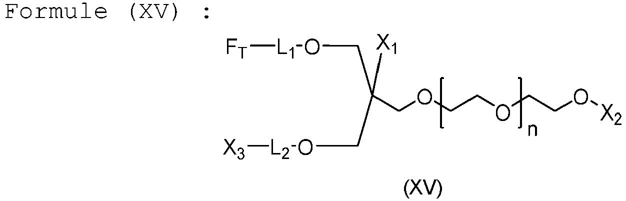
dans laquelle
(i) chaque F_{T} est un groupe polyfluoroorgano ;
(ii) X₁ est H, CH₃, ou CH₂CH₃ ;
(iii) chacun parmi X₂ et X₃ est indépendamment H, CH₃, CH₂CH₃, ou F_{T} ;
(iv) chacun parmi L₁ et L₂ est indépendamment une liaison, un lieur oligomérique, ou un lieur comportant deux carbonyles terminaux ; et
(v) n est un entier de 5 à 50 ; ou 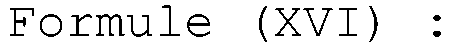 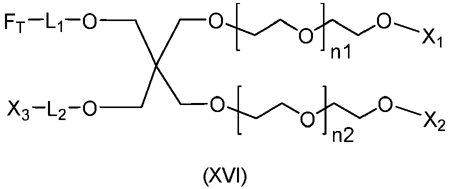
dans laquelle
(i) chaque F_{T} est un polyfluoroorgano ;
(ii) chacun parmi X₁, X₂, et X₃ est indépendamment H, CH₃, CH₂CH₃, ou F_{T} ;
(iii) chacun parmi L₁ et L₂ est indépendamment une liaison, un lieur oligomérique, un lieur comportant deux carbonyles terminaux, ou est formé à partir d'un diisocyanate ; et
(iv) chacun parmi n1 et n2 est indépendamment un entier de 5 à 50 ; ou
Formule (XVII) :
G - Am - [B - A]ₙ - B - G (XVII)
dans laquelle
(i) chaque A comprend un polybutadiène hydrogéné, poly(carbonate de (2,2-diméthyl)-1,3-propylène), polybutadiène, poly(adipate de diéthylèneglycol), poly(carbonate d'hexaméthylène), poly(éthylène-co-butylène), polyester (diéthylèneglycol-anhydride orthophtalique), polyester (1,6-hexanediol-anhydride orthophtalique), polyester (néopentylglycol-anhydride orthophtalique), un polysiloxane, ou éthoxylate de bisphénol A ;
(ii) chaque B est indépendamment une liaison, un lieur oligomérique, ou un lieur comportant deux carbonyles terminaux ;
(iii) chaque G est H ou un polyfluoroorgano, à condition qu'au moins un G soit un polyfluoroorgano ;
(iv) n est un entier de 1 à 10 ; et
(v) m est 0 ou 1.

2. Greffe vasculaire comprenant un corps tubulaire ayant une surface interne et un axe long, la surface interne comprenant un additif oligofluoré mélangé avec un polymère de base comprenant un polytétrafluoroéthylène, le corps tubulaire ayant une première extrémité et une seconde extrémité adaptées pour une fixation à une artère ou une veine,
dans laquelle la surface intérieure comprend de 0,05 % (p/p) à 15 % (p/p) de l'additif oligofluoré choisi parmi la structure de l'une quelconque des formules (I)-(XVII) :
Formule (I) :
F_{T}-[B-A]ₙ-B-F_{T} (I)
dans laquelle
(i) A comprend un polybutadiène hydrogéné, poly(carbonate de (2,2-diméthyl)-1,3-propylène), polybutadiène, poly(adipate de diéthylèneglycol), poly(carbonate d'hexaméthylène), poly(éthylène-co-butylène), polyester (néopentylglycol-anhydride orthophtalique), polyester (diéthylèneglycol-anhydride orthophtalique), polyester (1,6-hexanediol-anhydride orthophtalique), ou éthoxylate de bisphénol A ;
(ii) B est un segment comprenant un uréthane ; et
(iii) F_{T} est un groupe polyfluoroorgano, et
(iv) n est un entier de 1 à 10 ; ou
Formule (II) :
F_{T}-[B-A]ₙ-B-F_{T} (II)
dans laquelle
(v) B comprend un uréthane ;
(vi) A comprend un poly(oxyde de propylène), poly(oxyde d'éthylène), ou poly(oxyde de tétraméthylène) ;
(vii) F_{T} est un groupe polyfluoroorgano ; et
(viii) n est un entier de 1 à 10 ; ou 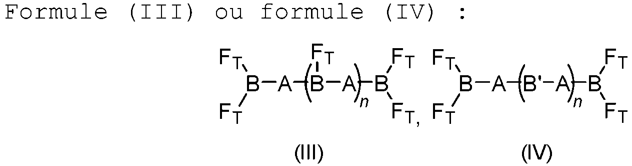
dans laquelle
(v) A est un segment oligomérique contenant une liaison éther, une liaison ester, une liaison carbonate, ou un polyalkylène et ayant un poids moléculaire théorique allant de 500 à 3 500 Da ;
(vi) B est un segment comprenant un trimère d'isocyanurate ou un trimère de biuret ; B', s'il est présent, est un segment comprenant un uréthane ;
(vii) chaque F_{T} est un groupe polyfluoroorgano ; et
(viii) n est un entier compris entre 0 et 10 ; ou
Formule (V) :
F_{T}-[B-A]ₙ-B-F_{T} (V)
dans laquelle
(v) A est un segment oligomérique comprenant un poly(oxyde de propylène), poly(oxyde d'éthylène), ou poly(oxyde de tétraméthylène) et ayant un poids moléculaire théorique allant de 500 à 3 000 Da ;
(vi) B est un segment formé à partir d'un diisocyanate ;
(vii) F_{T} est un groupe polyfluoroorgano ; et
(viii) n est un entier de 1 à 10 ; ou 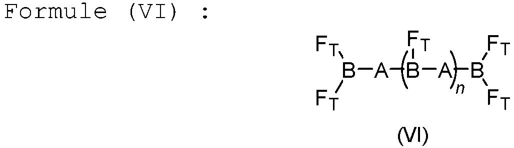
dans laquelle
(v) A est un segment oligomérique comprenant un poly(oxyde d'éthylène), poly(oxyde de propylène), poly(oxyde de tétraméthylène), ou un mélange correspondant, et ayant un poids moléculaire théorique allant de 500 à 3 000 Da ;
(vi) B est un segment comprenant un trimère d'isocyanurate ou un trimère de biuret ;
(vii) F_{T} est un groupe polyfluoroorgano ; et
(viii) n est un entier de 0 à 10 ; ou
Formule (VII) :
F_{T}-[B-A]ₙ-B-F_{T} (VII)
dans laquelle
(v) A est un polycarbonate polyol ayant un poids moléculaire théorique allant de 500 à 3 000 Da ;
(vi) B est un segment formé à partir d'un diisocyanate ;
(vii) F_{T} est un groupe polyfluoroorgano ; et
(viii) n est un entier de 1 à 10 ; ou 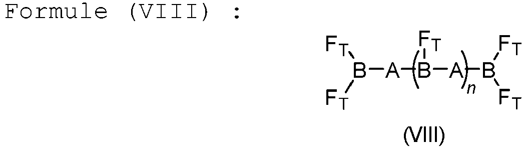
dans laquelle
(v) A est un segment oligomérique comprenant un polycarbonate polyol ayant un poids moléculaire théorique allant de 500 à 3 000 Da ;
(vi) B est un segment comprenant un trimère d'isocyanurate ou un trimère de biuret ;
(vii) F_{T} est un groupe polyfluoroorgano ; et
(viii) n est un entier de 0 à 10 ; ou 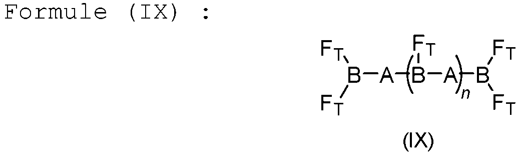
dans laquelle
(v) A comprend un premier segment de bloc choisi parmi un poly(oxyde de propylène), poly(oxyde d'éthylène), poly(oxyde de tétraméthylène), ou un mélange correspondant, et un deuxième segment de bloc comprenant un polysiloxane ou polydiméthylsiloxane, où A possède un poids moléculaire théorique allant de 1 000 à 5 000 Da ;
(vi) B est un segment comprenant un trimère d'isocyanurate ou un trimère de biuret ;
(vii) F_{T} est un groupe polyfluoroorgano ; et
(viii) n est un entier de 0 à 10 ; ou
Formule (X) :
F_{T}-[B-A]ₙ-B-F_{T} (X)
dans laquelle
(v) A est un segment choisi dans le groupe constitué par un polybutadiène hydrogéné, polybutadiène, polyisoprène hydrogéné, copolymère à blocs de polysiloxane-polyéthylène glycol, et polystyrène et possède un poids moléculaire théorique allant de 750 à 3 500 Da ;
(vi) B est un segment formé à partir d'un diisocyanate ;
(vii) F_{T} est un groupe polyfluoroorgano ; et
(viii) n est un entier de 1 à 10 ; ou 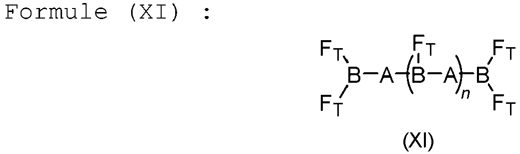
dans laquelle
(v) A est un polybutadiène hydrogéné, polybutadiène, polyisoprène hydrogéné, ou polystyrène et possède un poids moléculaire théorique allant de 750 à 3 500 Da ;
(vi) B est un segment comprenant un trimère d'isocyanurate ou un trimère de biuret ;
(vii) F_{T} est un groupe polyfluoroorgano ; et
(viii) n est un entier de 0 à 10 ; ou 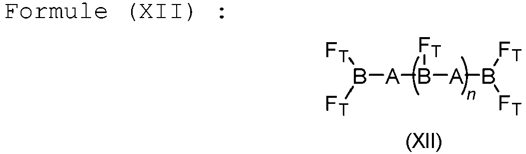
dans laquelle
(v) A est un polyester ayant un poids moléculaire théorique allant de 500 à 3 500 Da ;
(vi) B est un segment comprenant un trimère d'isocyanurate ou un trimère de biuret ;
(vii) F_{T} est un groupe polyfluoroorgano ; et
(viii) n est un entier de 0 à 10 ; ou
Formule (XIII) :
F_{T}-A-F_{T} (XIII)
où F_{T} est un groupe polyfluoroorgano et A est un segment oligomérique.
(13) 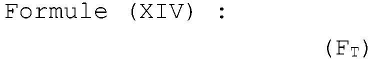 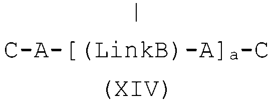 dans laquelle
(i) F_{T} est un groupe polyfluoroorgano fixé de manière covalente à LinkB ;
(ii) C est un groupe de terminaison de chaîne ;
(vi) A est un segment oligomérique ;
(vii) LinkB est un segment de couplage ; et
(viii) a est un entier supérieur à 0 ; ou 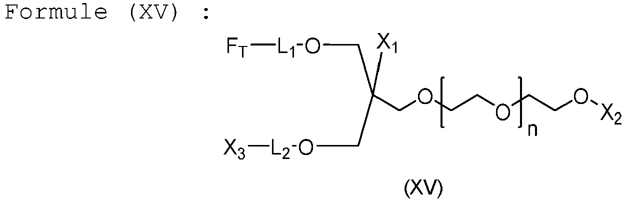
dans laquelle
(i) chaque F_{T} est un groupe polyfluoroorgano ;
(ii) X₁ est H, CH₃, ou CH₂CH₃ ;
(iii) chacun parmi X₂ et X₃ est indépendamment H, CH₃, CH₂CH₃, ou F_{T} ;
(iv) chacun parmi L₁ et L₂ est indépendamment une liaison, un lieur oligomérique, ou un lieur comportant deux carbonyles terminaux ; et
(v) n est un entier de 5 à 50 ; ou 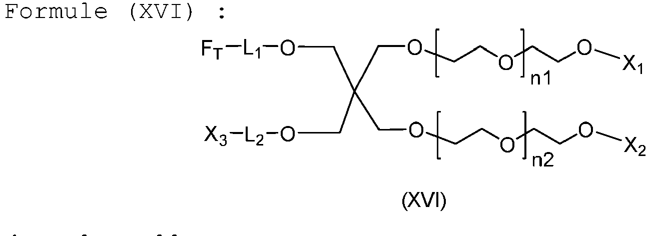
dans laquelle
(i) chaque F_{T} est un polyfluoroorgano ;
(ii) chacun parmi X₁, X₂, et X₃ est indépendamment H, CH₃, CH₂CH₃, ou F_{T} ;
(iii) chacun parmi L₁ et L₂ est indépendamment une liaison, un lieur oligomérique, un lieur comportant deux carbonyles terminaux, ou est formé à partir d'un diisocyanate ; et
(iv) chacun parmi n1 et n2 est indépendamment un entier de 5 à 50 ; ou
Formule (XVII) :
G - Am - [B - A]ₙ - B - G (XVII)
dans laquelle
(i) chaque A comprend un polybutadiène hydrogéné, poly(carbonate de (2,2-diméthyl)-1,3-propylène), polybutadiène, poly(adipate de diéthylèneglycol), poly(carbonate d'hexaméthylène), poly(éthylène-co-butylène), polyester (diéthylèneglycol-anhydride orthophtalique), polyester (1,6-hexanediol-anhydride orthophtalique), polyester (néopentylglycol-anhydride orthophtalique), un polysiloxane, ou éthoxylate de bisphénol A ;
(ii) chaque B est indépendamment une liaison, un lieur oligomérique, ou un lieur comportant deux carbonyles terminaux ;
(iii) chaque G est H ou un polyfluoroorgano, à condition qu'au moins un G soit un polyfluoroorgano ;
(iv) n est un entier de 1 à 10 ; et
(v) m est 0 ou 1.

3. Greffe vasculaire comprenant un corps tubulaire ayant une surface interne et un axe long, la surface interne comprenant un additif oligofluoré mélangé avec un polymère de base comprenant un polyuréthane, le corps tubulaire ayant une première extrémité et une seconde extrémité adaptées pour une fixation à une artère ou une veine,
dans laquelle la surface intérieure comprend de 0,05 % (p/p) à 15 % (p/p) de l'additif oligofluoré choisi parmi la structure de l'une quelconque des formules (I)-(XVII) :
Formule (I) :
F_{T}-[B-A]ₙ-B-F_{T} (I)
dans laquelle
(i) A comprend un polybutadiène hydrogéné, poly(carbonate de (2,2-diméthyl)-1,3-propylène), polybutadiène, poly(adipate de diéthylèneglycol), poly(carbonate d'hexaméthylène), poly(éthylène-co-butylène), polyester (néopentylglycol-anhydride orthophtalique), polyester (diéthylèneglycol-anhydride orthophtalique), polyester (1,6-hexanediol-anhydride orthophtalique), ou éthoxylate de bisphénol A ;
(ii) B est un segment comprenant un uréthane ; et
(iii) F_{T} est un groupe polyfluoroorgano, et
(iv) n est un entier de 1 à 10 ; ou
Formule (II) :
F_{T}-[B-A]ₙ-B-F_{T} (II)
dans laquelle
(ix) B comprend un uréthane ;
(x) A comprend un poly(oxyde de propylène), poly(oxyde d'éthylène), ou poly(oxyde de tétraméthylène) ;
(xi) F_{T} est un groupe polyfluoroorgano ; et
(xii) n est un entier de 1 à 10 ; ou 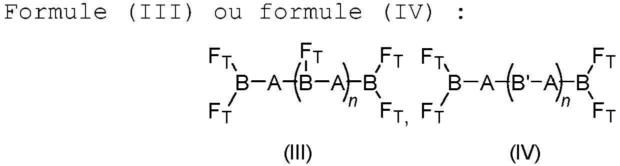
dans laquelle
(ix) A est un segment oligomérique contenant une liaison éther, une liaison ester, une liaison carbonate, ou un polyalkylène et ayant un poids moléculaire théorique allant de 500 à 3 500 Da ;
(x) B est un segment comprenant un trimère d'isocyanurate ou un trimère de biuret ; B', s'il est présent, est un segment comprenant un uréthane ;
(xi) chaque F_{T} est un groupe polyfluoroorgano ; et
(xii) n est un entier compris entre 0 et 10 ; ou
Formule (V) :
F_{T}-[B-A]ₙ-B-F_{T} (V)
dans laquelle
(ix) A est un segment oligomérique comprenant un poly(oxyde de propylène), poly(oxyde d'éthylène), ou poly(oxyde de tétraméthylène) et ayant un poids moléculaire théorique allant de 500 à 3 000 Da ;
(x) B est un segment formé à partir d'un diisocyanate ;
(xi) F_{T} est un groupe polyfluoroorgano ; et
(xii) n est un entier de 1 à 10 ; ou 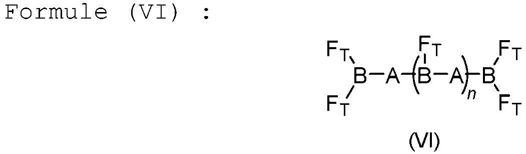
dans laquelle
(ix) A est un segment oligomérique comprenant un poly(oxyde d'éthylène), poly(oxyde de propylène), poly(oxyde de tétraméthylène), ou un mélange correspondant, et ayant un poids moléculaire théorique allant de 500 à 3 000 Da ;
(x) B est un segment comprenant un trimère d'isocyanurate ou un trimère de biuret ;
(xi) F_{T} est un groupe polyfluoroorgano ; et
(xii) n est un entier de 0 à 10 ; ou
Formule (VII) :
F_{T}-[B-A]ₙ-B-F_{T} (VII)
dans laquelle
(ix) A est un polycarbonate polyol ayant un poids moléculaire théorique allant de 500 à 3 000 Da ;
(x) B est un segment formé à partir d'un diisocyanate ;
(xi) F_{T} est un groupe polyfluoroorgano ; et
(xii) n est un entier de 1 à 10 ; ou 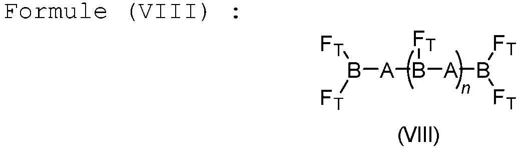
dans laquelle
(ix) A est un segment oligomérique comprenant un polycarbonate polyol ayant un poids moléculaire théorique allant de 500 à 3 000 Da ;
(x) B est un segment comprenant un trimère d'isocyanurate ou un trimère de biuret ;
(xi) F_{T} est un groupe polyfluoroorgano ; et
(xii) n est un entier de 0 à 10 ; ou 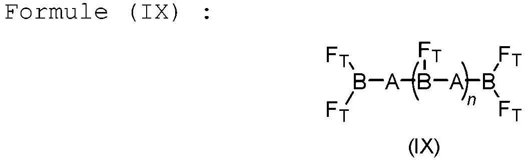
dans laquelle
(ix) A comprend un premier segment de bloc choisi parmi poly(oxyde de propylène), poly(oxyde d'éthylène), poly(oxyde de tétraméthylène), ou un mélange correspondant, et un deuxième segment de bloc comprenant un polysiloxane ou polydiméthylsiloxane, où A possède un poids moléculaire théorique allant de 1 000 à 5 000 Da ;
(x) B est un segment comprenant un trimère d'isocyanurate ou un trimère de biuret ;
(xi) F_{T} est un groupe polyfluoroorgano ; et
(xii) n est un entier de 0 à 10 ; ou
Formule (X) :
F_{T}-[B-A]ₙ-B-F_{T} **(X)**
dans laquelle
(ix) A est un segment choisi dans le groupe constitué par polybutadiène hydrogéné, polybutadiène, polyisoprène hydrogéné, copolymère à blocs de polysiloxane-polyéthylène glycol, et polystyrène et a un poids moléculaire théorique allant de 750 à 3 500 Da ;
(x) B est un segment formé à partir d'un diisocyanate ;
(xi) F_{T} est un groupe polyfluoroorgano ; et
(xii) n est un entier de 1 à 10 ; ou 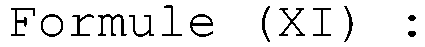 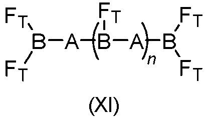
dans laquelle
(ix) A est un polybutadiène hydrogéné, polybutadiène, polyisoprène hydrogéné, ou polystyrène et possède un poids moléculaire théorique allant de 750 à 3 500 Da ;
(x) B est un segment comprenant un trimère d'isocyanurate ou un trimère de biuret ;
(xi) F_{T} est un groupe polyfluoroorgano ; et
(xii) n est un entier de 0 à 10 ; ou 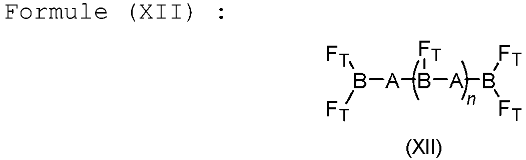
dans laquelle
(ix) A est un polyester ayant un poids moléculaire théorique allant de 500 à 3 500 Da ;
(x) B est un segment comprenant un trimère d'isocyanurate ou un trimère de biuret ;
(xi) F_{T} est un groupe polyfluoroorgano ; et
(xii) n est un entier de 0 à 10 ; ou
Formule (XIII) :
F_{T}-A-F_{T} **(XIII)**
où F_{T} est un groupe polyfluoroorgano et A est un segment oligomérique.
(13) 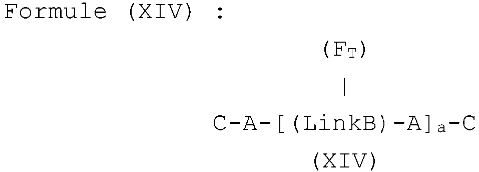 dans laquelle
(i) F_{T} est un groupe polyfluoroorgano fixé de manière covalente à LinkB ;
(ii) C est un groupe de terminaison de chaîne ;
(ix) A est un segment oligomérique ;
(x) LinkB est un segment de couplage ; et
(xi) a est un entier supérieur à 0 ; ou 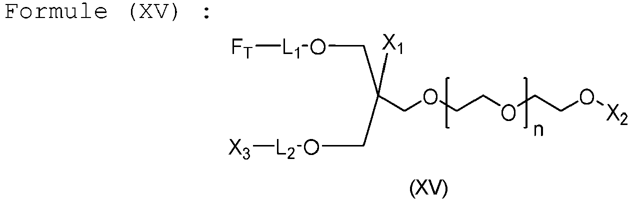
dans laquelle
(i) chaque F_{T} est un groupe polyfluoroorgano ;
(ii) X₁ est H, CH₃, ou CH₂CH₃ ;
(iii) chacun parmi X₂ et X₃ est indépendamment H, CH₃, CH₂CH₃, ou F_{T} ;
(iv) chacun parmi L₁ et L₂ est indépendamment une liaison, un lieur oligomérique, ou un lieur comportant deux carbonyles terminaux ; et
(v) n est un entier de 5 à 50 ; ou 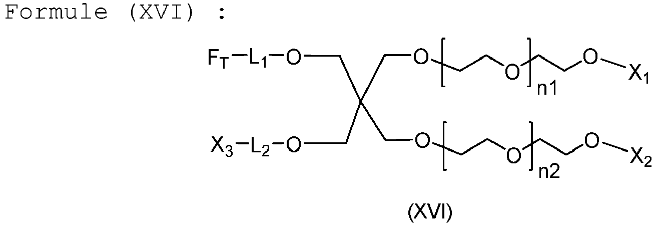
dans laquelle
(i) chaque F_{T} est un polyfluoroorgano ;
(ii) chacun parmi X₁, X₂, et X₃ est indépendamment H, CH₃, CH₂CH₃, ou F_{T} ;
(iii) chacun parmi L₁ et L₂ est indépendamment une liaison, un lieur oligomérique, un lieur comportant deux carbonyles terminaux, ou est formé à partir d'un diisocyanate ; et
(iv) chacun parmi n1 et n2 est indépendamment un entier de 5 à 50 ; ou
Formule (XVII) :
G - Am - [B - A]ₙ - B - G (XVII)
dans laquelle
(i) chaque A comprend un polybutadiène hydrogéné, poly(carbonate de (2,2-diméthyl)-1,3-propylène), polybutadiène, poly(adipate de diéthylèneglycol), poly(carbonate d'hexaméthylène), poly(éthylène-co-butylène), polyester (diéthylèneglycol-anhydride orthophtalique), polyester (1,6-hexanediol-anhydride orthophtalique), polyester (néopentylglycol-anhydride orthophtalique), un polysiloxane, ou éthoxylate de bisphénol A ;
(ii) chaque B est indépendamment une liaison, un lieur oligomérique, ou un lieur comportant deux carbonyles terminaux ;
(iii) chaque G est H ou un polyfluoroorgano, à condition qu'au moins un G soit un polyfluoroorgano ;
(iv) n est un entier de 1 à 10 ; et
(v) m est 0 ou 1.

4. Greffe vasculaire selon la revendication 3, dans laquelle le polyuréthane est choisi parmi les polycarbonates uréthanes, un polyuréthane comportant un segment mou de poly(diméthylsiloxane), un élastomère de polyuréthane à base de poly(tétraméthylène glycol), les polyuréthanes segmentés et les polyétheruréthanes.

5. Greffe vasculaire selon les revendications 1 à 4, dans laquelle l'additif oligofluoré est choisi parmi le 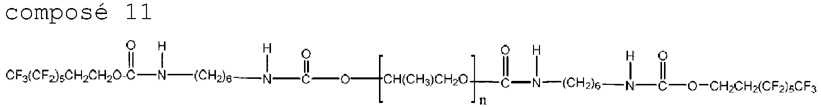 dans lequel le poids moléculaire est de 800 à 1 200 g/mole ; 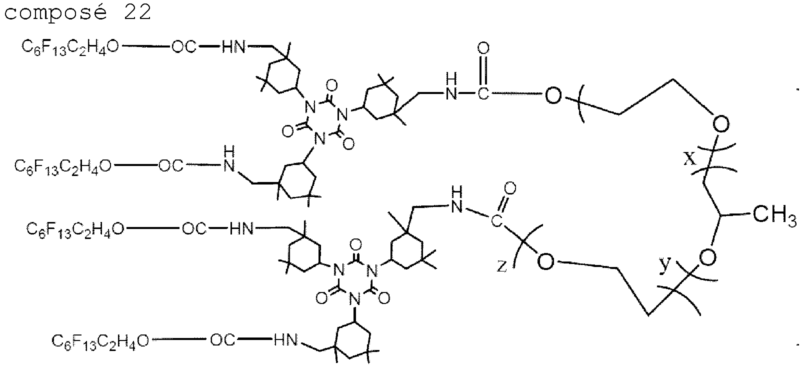 dans lequel
x, y et z sont des entiers ; ou 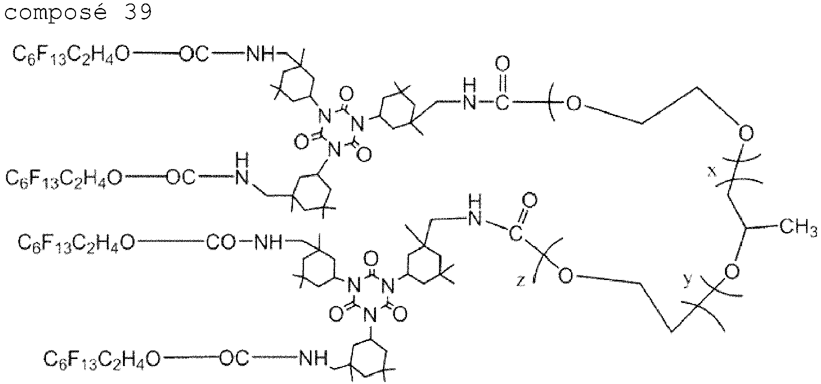 dans lequel Mw du diol est 8 000 g/mole, PEG = 80 % et PPG = 20 %.

6. Greffe - vasculaire selon l'une quelconque des revendications 1 à 5, dans laquelle la première extrémité et la seconde extrémité adaptées pour une fixation à une artère ou une veine comprennent des barbes d'ancrage ou un matériau approprié pour une couture sur une partie d'une artère ou d'une veine.
